(19) Europäisches Patentamt
European Patent Office
Office européen des brevets

(11) **EP 4 592 257 A1**

(12) **EUROPEAN PATENT APPLICATION**

(43) Date of publication:
**30.07.2025 Bulletin 2025/31**

(21) Application number: 24382074.3

(22) Date of filing: **26.01.2024**

(51) International Patent Classification (IPC):
*C02F 9/00* (2023.01)      *C05F 11/00* (2006.01)
*C12M 1/00* (2006.01)      *C12N 1/12* (2006.01)
*C02F 1/34* (2023.01)      *C02F 1/36* (2023.01)
*C02F 1/38* (2023.01)      *C02F 1/44* (2023.01)
*C02F 3/32* (2023.01)      *C02F 11/127* (2019.01)
*C02F 103/20* (2006.01)

(52) Cooperative Patent Classification (CPC):
(C-Sets available)
**C02F 9/00; C05F 3/00; C05F 3/06; C12M 21/02;
C12N 1/12;** C02F 1/34; C02F 1/36; C02F 1/385;
C02F 1/444; C02F 3/322; C02F 11/127; C02F 11/15;
C02F 2103/20            (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Worldpathol Global United, S.A.**
**50720 Zaragoza (ES)**
• **Galindo Cardiel, Iván José**
**50720 Zaragoza (ES)**

(72) Inventors:
• **CARTAGENA LIROLA, Hugo Fernando**
**Zaragoza (ES)**

• **MARTINEZ MATAMOROS, Diana**
**Zaragoza (ES)**
• **OSANTE GARCIA, Iñaki**
**Zaragoza (ES)**
• **GALINDO CARDIEL, Ignacio Miguel**
**Zaragoza (ES)**
• **GALINDO CARDIEL, Raul Luis**
**Zaragoza (ES)**
• **MORATA GALVE, Juan José**
**Zargoza (ES)**
• **BERNED NAVALES, Raquel**
**Zaragoza (ES)**
• **GALINDO CARDIEL, Iván José**
**Zaragoza (ES)**

(74) Representative: **Ponti & Partners, S.L.P**
**Edifici PRISMA**
**Av. Diagonal núm. 611-613, Planta 2**
**08028 Barcelona (ES)**

(54) **AN ON-SITE MODULAR SYSTEM FOR PROCESSING AND TRANSFORMING ANIMAL WASTE AND A PROCESS AND USES THEREOF**

(57)      The present invention relates to an on-site modular system for processing and transforming animal waste. The present invention also relates to a process for transforming animal waste *in situ* using said on-site modular system. The present invention further relates to the uses and derived advantages in terms of farm greenhouse gas emissions management and agricultural or natural soil amendments of a processed rich-nutrients biologically absorbable nitrogen-rich phosphorus-rich potassium-rich liquid microalgae culture fraction obtained with the process of the invention. Besides, the invention further relates to the uses as recycler of toxic or contaminants in animal waste farm and/or for animal feed of a processed rich-carbon solid inactivated microalgae culture fraction obtained with the process of the invention.

(52) Cooperative Patent Classification (CPC): (Cont.)

C-Sets
**C05F 3/00, C05F 7/00, C05F 11/08**

## Description

### FIELD OF THE INVENTION

The present invention relates to a system for processing and transforming animal waste. In particular, the present invention relates to a modular biotechnological platform for *in situ* recycling and transforming animal waste, specifically slurry, through the production of microalgae and its derivatives cultured on sequentially physical-processed liquid slurry-derived fractions. This innovation makes it possible to obtain high value-added by-products, such as nutrients for animal feed and a new phytoremediator for agricultural and natural soils amendments. In addition, it offers significant advantages over existing systems, including 10 to 95% of nitrogen, phosphorus, and potassium biologic fixation of the slurry, 50 to 85% prevention of greenhouse gas emissions of the farm, and the capability of pollutants removal from the farm ponds. In short, it provides an efficient and sustainable solution for animal waste management derived of intensive production farm.

### BACKGROUND OF THE INVENTION

### Animal slurry as an environmental problem

Slurry is a type of manure, a by-product composed of the mixture of feces, urine, straw, cleaning water, chemicals of farm disinfection, degraded plastic residues, and so on. Slurry is mainly generated on livestock farms. Its nutrient concentration is very low, because most of it is water, and it is mainly composed of water, organic matter, nitrogen, phosphorus, potassium, macronutrients (calcium, sulfur and magnesium) and micronutrients (boron, iron, manganese, nickel, copper and zinc).(1, 2) Its composition is directly related to the diet eaten by the animals, since the contribution derived from the organism of the generating animal is practically negligible.

The steady increase in swine wastewater (slurry) has become a serious environmental concern. High levels of nutrients and toxic pollutants in slurry have a significant impact on the ecosystem and public health.(3) Regulators set up a complex and complete administrative machinery to try to control or maintain at acceptable levels these problems associated with industrial activity.(4)

As illustrative example, environmental impact associated with the swine sector affects both intensive and extensive farms, although the higher concentration of animals in the intensive farms means that the generation of slurry and manure requires a specific management system for the leachate and waste produced (ammonia, nitrates, greenhouse gases, diverse biosecurity concerns such as bacteria antibiotic resistance, among others).(5) The basis of the concept of integrated slurry management leads us to act from the source (animal feed) and make decisions about the storage and eventual treatment. In addition, we must consider the design and management of the farms, the automatic and frequent removal of slurry and make an agricultural use planning.(6, 7, 8, 9)

A comprehensive slurry management plan is an actions program, individual or collective, that leads to adapting the continuous generation of this residue to the needs of the crops, in space and time (10). It envisages measures in three distinct areas:

1. MANAGEMENT: Management measures for the reduction at source, of diets, flow rates and components that limit their subsequent use, such as excessive content of water, nitrogen, phosphorus, or heavy metals.

The calculation of the storage ponds, the volume of which must allow the adequacy between the continuous production of slurry and the discontinuous consumption as quality by-products, as well as adequate strategic planning and analysis of critical points and control, are the pillars of a slurry management plan. Changes in feeding and correction of diets not only help the health of the animals and their adequate productive performance but must also serve to contain the nitrogen content in the waste.

2. FERTILIZATION (USE): Fertilization plan and the use of slurry based on knowledge of the soils, crop requirements, both dosage and seasonality, and climatological and hydrological characteristics.

Water consumption optimization on the farm, especially in better and more efficient cleaning, allows a lower flow of slurry, lower storage volume requirements and a reduction in transport costs. Cooperation between livestock breeders and farmers, for the collective management of the fertilizer value of manure, must make it possible to optimize transport and application logistics.

3. TREATMENT (PROCESS): Strategic combination of processes that modify the characteristics of slurry to adapt it to demand as a quality product.

The suitability of a treatment process will depend on each geographical area, the needs highlighted by the management plan, the desired quality of the final product obtained and the associated economic costs.(10)

The livestock sector is responsible for 0.8% of nitrate emissions, 9.1% of greenhouse gases and the systematic increase in ammonia emissions (1% per year for the period 1999-2019) (2019 data).(9) According to European Commission, (a) nitrogen emissions cost at least EUR 70 billion annual of losses in EU; (b) 81% of agricultural nitrogen input to aquatic systems are caused by livestock production, and (c) 87% of ammonia emissions from agriculture to the atmosphere are caused by livestock production.(11) The nominal composition parameters of the main pollutants of the slurry have been established in our operating conditions.(12, 13)

**Relevant Facts to Consider: Problem, Technology and Limitations (Quality by Design)**

So far, various strategies for the containment of this animal waste have been proposed; all of them involve moving the waste from point A to point B at X distance from the generator (farm).(3, 4, 10, 14, 15, 16, 17, 18) A large part of these transports are carried out by road in highly polluting specialized trucks, caused by the energy needs of the processing plant (14, 18, 19); others are carried out on rural roads using agricultural tractors and are due to issues of traditional management and organization of livestock production regions (1, 20); The least of them are carried out due to regulatory issues that have found no other solution than the official transport of waste to avoid concentration, but obliging its use and reprocessing at full cost for the community that processes it.(2, 4, 10) This cost varies depending on the distance and volume transported, and its incidence changes considerably depending on the economic value of the nutrients transported. Thus, the maximum permissible transport distance depends on the economic value of what is being transported and must be assessed on a case-by-case or phase-by-phase basis. On gestation and lactation phase the cost of transport for diluted slurry can be equated with its fertilizer value for a distance of around 5-7 km, while for fattening slurry this distance can exceed 15 km.(13) These distances can increase considerably if a centralized or collective transport service is used, as it allows logistics, equipment usage time and depreciation to be optimized, always complying with the necessary health restrictions.(10)

As illustrative example, in Spain there are 26 slurry treatment plants, owned by Ignis (6), Iberdrola (4), Naturgy (2), Cs Energy (2) and other companies, including some livestock cooperatives. These facilities are devoted to drying the slurry, turning it into fertilizer to prevent its environmental impact on soils and aquifers. They can process about 2.5 MM/m$^3$ of waste annually in regions with a high concentration of farms - in some areas the water was not drinkable due to the excess of nitrates - avoiding the emission of 700,000 TCO$_2$eq. All the slurry treated in these plants is transported in tanker trucks with a capacity of 12,000-25,000 L, so moving the waste to the treatment facilities is the main operative (€) and environmental (fuel consumption) cost of these systems, significantly diminishing its efficacy. Despite these data, there is a need to optimize processes so that they are viable, safe, and effective in processing waste, in recovering byproducts, and improving swine and human population wellbeing.

Cost saving is a pillar for the stability of the animal intensive farms, which avoids as much as possible external fluctuations (epidemics, state, regional or community interventions, investment funds, fluctuations in the prices of raw materials, etc.).(21) So far, economic value of fattening swine slurry has been quantified between 5 and 7,3 euros/m$^3$ in its equivalent in prices of the NPK mineral fertilizers.(6, 10, 11) Swine slurry transportation costs vary. A 2014 study set costs at approximately €5.40 per tonne plus €1.98 per kilometer hauled. Liquid manure haulers charged around €0.018 per litre, plus €0.0031 per litre per kilometer. A 2016 survey showed an average price of €0.011 per litre of manure applied at one kilometer, with an additional cost of about €0.0031 per litre per kilometer hauled. A 2017 report estimated the annual manure application cost for a 4800-head swine farm at about €33,750.(22, 23, 24, 25) While the reference of the cost of management and transport of these same slurry is estimated at 2.92 euros/m$^3$ on average in the experimental farm where the proof-of-concept is installed (Granja de Experimentación Tecnológica S.L. -B02859924-, ES50164.1, Central Region of Zaragoza, Mediana de Aragon, Spain) (PoC farm). Annual costs associated with raw pig manure transport and spreading by tractor and vacuum tanker and by truck for a 500-sow integrated pig farm ranged from 3,4 € m$^{-3}$ 1 Km to 11,8 € m$^{-3}$ 100 Km of displacement.(26) Mineral fertilizer use is replaced by swine slurry under conditions of minimum tillage, totally in barley and partially in corn, being also economically profitable in rainfed areas with a continental Mediterranean climate.(2)

In Spain, 58,703,600 pigs were slaughtered in 2021, with a meat production of 5,220,773 T, ranking third in the world, after China and the United States. The most important agricultural subsector in Aragon is the swine sector. It represents 66% of the Final Livestock Production and 39% of the Final Agricultural Production, these values being much higher than the national average (42.5% and 15.9% respectively). To this fact, we must add that most of the cereal produced in Aragon is used for pig feed.(27)

The Mediterranean climate, as it does not have constant rainfall, varies considerably as it moves away from the coast, since the air has a lower degree of humidity, so that it acquires elements of the continental climate, with pronounced thermal amplitudes both daily and annual (continental Mediterranean subtype).(28) They can range from mild winters and very hot summers to cold winters and mild summers, in the latter case with frost and precipitation in the form of snow. This is the climate of the study area in which the proposed solution was tested, Mediana de Aragón. In these climatic conditions, cultivating microalgae requires constant and guaranteed energy, for the maintenance of the operators and support structures of the vital system and processing of by-products.(28)

**Culture biotechnology system: scale-up criteria**

The algae cycle has been intensively studied in production demonstrations for slurry recycling in pig farms,(3, 14, 16, 20) and mixed pig leachate,(15, 16) but there are important gaps and study trends that need to be evaluated on medium-large scales and real study conditions, in order to complete the practical knowledge that allows us to evolve in their applicability on a large scale,(15, 17, 29) and local circular economy related to animal health.(30) Defining the parameters of cultivation under modified, partially modified environmental conditions has been one of the research objectives that we have been studying throughout the invention process.

Scale-up criterion depends on experience in the specific fermentation process. There is no single rigid and safe criterion for scaling up aerobic culture processes. For this reason, the validation design of a scaling process is a verification of the applicability of the data obtained in the R&D phase, generating more or less fixed experimentation schemes that study a maximum of two variables at the same time.(31, 32, 33) When working with microalgae, which are very sensitive to several factors, more than one scaling criterion is used. Factors such as pH, temperature or cultivation time are usually set from the technology transferred by the R&D and, generally, it is established as a starting hypothesis that varies in the same way at both scales, from pilot to industrial.(34, 35, 36) The principle of similarity is linked to the concept of similarity between the small scale and the larger scale with respect to geometric, kinematic and dynamic parameters. In essence, the greater the similarity between technologies applied to bioreaction, the greater the probability that the process will be close to 100% scalable. The geometric similarity corresponds to the ratios between lengths must be equal in both systems.(29, 32, 37) As a general rule for the scaling of the bioprocess with microalgae, we have established the criterion of geometric similarity and scaling part of the criterion of similarity in the invention process.(31, 32)

**Brief overview of the separation of solid and liquid fractions of swine slurry**

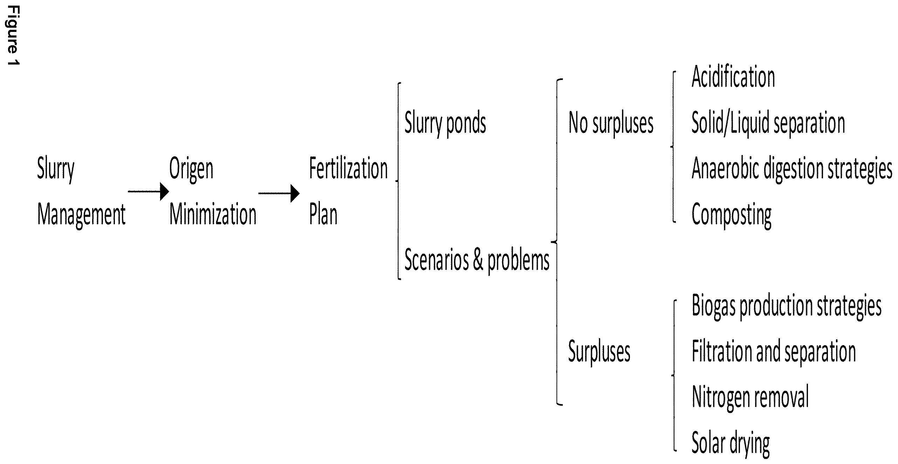

Solid/liquid separation systems for slurry make it possible to improve its subsequent management, such as its transport or subsequent treatment of each phase of the slurry separately (Figure 1.) Solid/liquid separation equipment allows the separation of slurry into two phases, a solid and a liquid fraction. The objective set for this separation is to improve management, facilitate the transport of the solid fraction, enable treatment processes for each phase separately or reduce emissions from the liquid phase, which is the largest volumetric. This liquid fraction can enable precision irrigation systems as it contains a lower concentration of organic matter.(6, 8, 38)

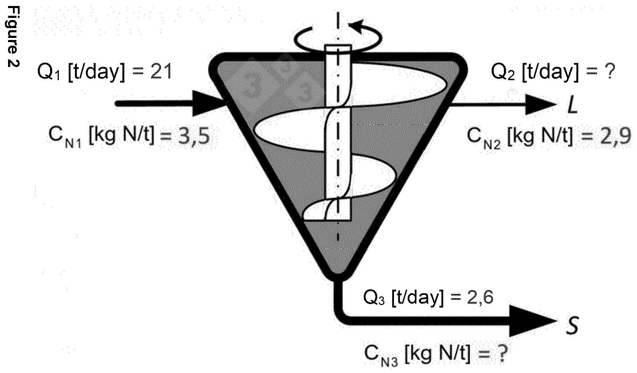

The efficiency of a separator is quantified with two main values: 1) the efficiency in the separation of flows ($\eta Q$); and 2) the separation efficiency of charges, such as nitrogen ($\eta N$) or phosphorus ($\eta P$). Each of these efficiencies is defined as the fraction of the mass flow from the inlet that is transferred to the solid fraction; The remainder goes to the liquid fraction, since the principle of conservation of mass must be adhered to. As reviewed in Flotats Ripoll (2019), these efficiencies and unknown data can be calculated from actual measurements in a given slurry processing system (nomenclature indicated in Figure 2.)(13)

$$Q_2 = Q_1 - Q_3 = 18,4 \ t/d \Rightarrow \eta_Q = 2,6/21 = 0,124 \rightarrow 12,4\%$$

$$Q_1 C_{N1} = Q_2 C_{N2} + Q_3 C_{N3} \Rightarrow C_{N3} = (Q_1 C_{N1} - Q_2 C_{N2})/Q_3 = 7,75 \ kg \ N/t$$

$$\eta_N = Q_3 C_{N3}/Q_1 C_{N1} = 0,274 \rightarrow 27,4\%$$

In the example developed by Flotats Ripoll (2019),(13) the 27.4% of the nitrogen is exported from the slurry with a concentration degree of 2.2 by separating the solid fraction; Note that what is exported has a nitrogen concentration 2.2 times higher than the original concentration. Obtaining high concentrations (combining several separators in series, for example) implies reducing the overall separation efficiency, i.e., less percentage of nitrogen is exported, but more concentrated, which may be of interest depending on the subsequent use of the solid fraction.

Table 1 shows efficiency ranges are given for the three main types into which S/L separation systems are classified.(13) By using coagulants (iron or aluminum salts) or flocculants (organic polymers) the efficiency of a piece of equipment can be improved, although this effect is not generalizable and depends on the composition of the slurry. Flocculant separation is improved by flotation by adding air bubbles when the slurry is very diluted. Coagulants can be replaced by an electrocoagulation system, in which an electric current solubilizes an electrode, usually made of iron, to achieve the same particle aggregation effect.

In general, in terms of efficiency: centrifugation >> pressure separation >> size separation. However, wide ranges of efficiencies have been found (Table 1). Therefore, efficiency depends on the composition of the slurry and, above all, on its age at the time of separation; The younger the slurry, the higher the efficiency, being higher when the slurry is separated within a few hours of excretion. This is because the saprophytic slurry infundibulum's involved in the decomposition processes solubilize many solid compounds from the beginning of their formation, and once solubilized they can no longer be separated by mechanical methods. Efficiency depends more on the previous handling of the slurry than on the quality of the separators.(9, 13, 39) The literature consulted estimates that the separation performance of phosphorus (solid fraction) is usually higher than that of nitrogen (liquid fraction).(7, 9, 39, 40) As reported by Cárdenas Calvachi and Sánchez-Ortíz (2013), nitrogenous carbon biological degradation is slower than the degradation of carbon compounds, since only part of the organic nitrogen can be mineralized or converted to inorganic nitrogen, and the rest is used to produce bacterial proteins.(41) This implies that, in the liquid fraction, the N/P ratio will be higher than that present in fresh slurry, due to the addition of the concentration of inorganic, organic and protein-derived nitrogen. This ratio will be more suited to the demand of many crops.(6, 8, 38)

**Table 1. Efficiency ranges expressed as a percentage of the indicated parameter that is transferred to the solid fraction, of mass flow rates (Q), nitrogen (N) and phosphorus (P), for different types of solid/liquid separators. Adapted from 3tres3, Flotats Ripoll, 2019.(13)**

| Separation system | Separation technology | Separation Efficiency (%) | | |
|---|---|---|---|---|
| | | $\eta_Q$ | $\eta_N$ | $\eta_P$ |
| By Particle Size and Gravity | Static or rotary sieve, grid, drum, settling | 10-20 | 15-25 | 15-40 |
| By pressure | Screw Press, Filter Press | 10-25 | 15-30 | 20-80 |
| By centrifugation | Centrifuge | 10-20 | 25-50 | 40-80 |

To improve separation efficiency, the Cooperl cooperative (France) developed the TRAC[©] (*Traitement par RAclage Cooperl*) system, which consists of separation within the pits under *slat* every few hours.(42) A system of scrubbers runs upwards on the floor of the pit, which has a slight slope; the solid fraction is compressed upwards while the liquid drains downwards, so that on one side of the house the solid fraction is obtained and on the other the liquid fraction. Efficiencies in nitrogen separation in the order of 55% have been measured with this system, efficiencies not achievable with conventional separation systems, with the additional advantages of fewer respiratory problems, lower mortality rate and more productivity, due to the avoidance of decomposition processes and gas emissions in the house itself. One of the most interesting conclusions of the work of this French cooperative is the demonstration of the feasibility of the treatment of this liquid phase by biological processes in situations of "old" slurry (low Chemical Oxygen Demand (COD)/nitrogen (N) ratio). The salinity observed in this liquid fraction, composed of a cocktail of ions and concentrations of organic matter, together with a high ratio of "monovalent cations"/"divalent cations", have been identified as the main parameters affecting the activity of Anammox bacteria. In their trials, it was not possible to identify further parameters specifically and quantitatively.(42)

**Ultrasonic homogenization system**

The phenomenon of cavitation generated in sonication creates an unusual environment for chemical reactions. The rapid compression of gases and vapors within the bubble generates enormous temperatures and pressures, up to 5000 °C and 1000 atm, respectively. Since the bubbles are very small compared to the volume of the surrounding liquid, the heat generated is "dissipated" very quickly (> 1010 °C × s-1), so that the ambient conditions remain essentially unchanged. This combination of high temperatures, high pressures, and rapid cooling creates conditions that can affect the stability of proteins.(43, 44)

In addition, rapid collapse also generates shock waves that can induce mechanical effects. At the interface between two-phase systems, collapse takes place in a non-symmetrical manner, with a jet of liquid crossing the cavity at a velocity of hundreds of m s$^{-1}$. Due to the impact, small solid or liquid particles are released. In liquid-liquid systems, emulsions are formed, which are generally much more stable than those formed conventionally. Solids experience fragmentation and erosion, which increase the activated areas. Cavitation also accelerates mass transport and decreases repassivation by reaction products. The overall result is much easier contact between immiscible or poorly soluble reagents. Because of the highly energetic conditions generated, many chemical reactions can be activated by sonication. These mechanical phenomena can lead to the mechanical denaturation of proteins in the environment.(43, 44)

Ultrasonication is a very efficient means of dispersing solids or emulsifying liquids.(45) As the measurement of the solid particle or liquid droplet decreases, the total contact surface area increases and consequently the reaction rate increases. Another effect of sonication is mass transfer. In heterogeneous systems, reaction products accumulate on the contact surface, preventing the interaction of new reactant molecules with this surface. The mechanical shear forces caused by cavitational rays produce a turbulent flow and transport of material to and from the surfaces of solid particles or droplets, so that the exposure of one reagent to the other is maximized. On the other hand, the combination of high-speed liquid jets, high pressures and temperatures, and very high heating and cooling rates that take place during the implosion of cavitational bubbles make ultrasonic cavitation a unique means of transferring energy to chemical reactions.(45)

The key piece in any ultrasonic device is the transducer, an electromechanical component responsible for generating ultrasound, by converting the electrical oscillations produced by a generator into mechanical vibrations.(46) Typically, transducers are constructed of piezoelectric ceramics. The application of an electric potential difference across the opposite faces of this material produces an expansion or contraction of the material depending on the polarity of the applied charges. These fluctuations in the dimensions of the material are responsible for the generation of ultrasonic vibrations. The main objective of applying ultrasound to chemical reactions is to improve their speed and performance and/or induce specific chemical reactivity.(46)

Acoustic waves, which are purely mechanical in nature, cannot be absorbed by molecules and must be transformed into a chemically useful form through an indirect and complex process called cavitation.(47) Like all sounds, ultrasound propagates through a series of waves of compression and expansion that travel through a medium. Compression cycles bring the molecules in the medium together while expansion cycles separate them. In a liquid medium, the ultrasonic expansion cycle can generate enough negative pressure to break the cohesive forces of the liquid molecules, separating them locally, creating a true microcavity or bubble. Normally, this takes place in previously contaminated areas of the solution, where there are small particles, dissolved gases or microbubbles due to a previous cavitation process. These bubbles grow in a few cycles, from less than a micrometer to a few tens of micrometers, trapping vapors or gases from the medium. The growth of the cavity during each expansion is slightly larger than the shrinkage during compression. Thus, over many acoustic cycles, the cavity grows until it finally achieves a critical measurement with which it can efficiently absorb energy from ultrasonic irradiation. At this point, the cavity can grow rapidly during an acoustic cycle acquiring an unstable size with which it can no longer absorb energy efficiently. Without this energy, the cavity cannot be maintained and the fluid that surrounds it violently enters the cavity, causing it to implode.

## Culture conditions for *A. platensis* production

This microorganism is a Gram-negative microalgae or cyanobacterium, which reproduces about 25% per day.(48) As for its morphology, it is characterized by having a row of cylindrical trichomes with widths between 2.5 and 16 $\mu$m that in turn are separated by septa visible to the light microscope, a normally open helix with a distance between turns from 0 to 80 $\mu$m and relatively large diameters from 15 to 60 $\mu$m.(49) It grows preferentially in an alkaline medium in a pH range of 8.5 to 10, with an optimal growth pH of around 9 that inhibits, at the same time, the growth of other planktonic microorganisms. Its growth temperature ranges from 24°C to 35°C, with an optimal temperature of 33°C.

Regarding light, they need to be subjected to some exposure due to their photosynthetic nature, however, it has been described in the literature that prolonged exposures of light inhibit the growth of the microorganism.(49)

## Filtration system for solid/liquid culture fractions separation

Separation slurry processes are a well-known technology used for treatment and recycling manure and slurry.(10, 50) Some of the best technologies used include filtration or high-pressure systems are:

- Batch Filtration: Batch filtration involves the separation of suspended solids from a slurry containing a quantity of associated liquid. The slurry is directed towards the filter using various methods, such as pumping, gas pressure, gravitational head, or centrifugal force. Solids are retained by a filter medium, which allows the passage of the filtrate. The flow of the filtrate is created by the pressure differential generated over the system.(10)
- High-Pressure Slurry Ablation: High-Pressure Slurry Ablation (HPSA) is a patented particle attrition technology for the comminution and selective liberation of minerals. Two slurry streams are pumped at high pressure through opposing nozzles, and collisions between solid particles suspended in the slurry streams cause breakage of the feed material.(51)
- Combination Mechanical Slurry Conditioning and Filtration: This approach relies on slurry analysis and testing to develop a process solution called combination mechanical slurry conditioning and filtration. The process involves primary filtration using candle filters on a continuous basis, which retain particles down to 0.5 microns and discharge the solids as a concentrated sludge/slurry. The concentrated slurry is then filtered on a secondary pressure plate filter to recover the remaining solvent and discharge the dry solids.(52)

These processes offer different advantages and disadvantages depending on the specific application and slurry characteristics. Batch filtration is suitable for various particle sizes and concentrations, while HPSA focuses on selective liberation of minerals. Combination mechanical slurry conditioning and filtration provides a dependable process with high-quality filtrate and minimal utility usage.

**Theoretical approach and fundamentals for the proposed designed solution**

The economic, social and environmental costs of slurry contamination is a major problem in the swine industry, which is, in every sense, a sector of margin.(53) For this reason, the objective of the solution is to undertake the research of a new cheap scalable comprehensive technological treatment, transformation and byproduct's production for *in situ* slurry reuse, through the development of an originally-managed microalgae production system, which will allow the obtaining of high value-added byproducts: (a) new ingredient to feed animals, and (b) phytoremediators for agricultural and natural soil amendments without transportation or reprocessing out of the farm. Platform will allow to avoid avoiding greenhouse gas emissions (GGE), composed but not restricted to ammonia ($NH_3$), nitrous oxide ($N_2O$), methane ($CH_4$), carbon dioxide ($CO_2$) and hydrogen sulfide ($H_2S$), by significantly decreasing open-air fermentation days of the slurry.

Microalgae with a short shelf life, high growth rate, and high $CO_2$ utilization efficiency is one of the viable renewable resource techniques to produce biomass using nutrients in wastewater.(54, 55) The potential to convert slurry nutrients into biomass for industrial use (food, biofertilizers, biolipids, bioenergy) has attracted increasing attention, generating a wide range of industrial use in the circular economy of farms.(14, 56, 57) In particular, swine wastewater is considered a valuable source of water and nutrients for microalgae culture, having been treated by anaerobic digestion, various A/O processes, microbial fuel cells, and the cultivation of various microalgae.(3, 14, 16) Under this context, it is critical to improve microalgae production system to save economical (transport, management), environmental (GGE, farmland microbial contamination) or social (time consuming actions) cost in animal slurry planning.

Any solution applicable to slurry management must involve minimizing the barriers to the disposal of this natural waste derived from intensive animal production, namely:

1. High consumption of oil fuels, time, and energy in the slurry transportation (coarse raw material) to the waste (farmland), processing (slurry digesters) and desiccation centers (energy transformation facilities).
2. Need to contract and manage multiple arable lands for the proper disposal of waste according to a fertilization plan that is often left in the hands of third parties and outside the perimeter of control of the farmers.
3. High concentration of nitrates/ nitrites/ toxins and / or potentially zoonotic microbial populations in unprocessed slurry may contaminate farmlands.

## SUMMARY OF THE INVENTION

According to the first aspect, the present invention relates to an on-site modular system for processing and transforming animal waste.

According to a second aspect, the present invention relates to a process for transforming animal waste *in situ* using the on-site modular system according to the first aspect of the invention.

According to a third aspect, the present invention relates to the uses of a processed rich-nutrients biologically absorbable nitrogen-rich phosphorus-rich potassium-rich liquid microalgae culture fraction obtained with the process according to the second aspect of the invention.

## BRIEF DESCRIPTION OF THE DRAWINGS

Embodiments of the present invention will now be further described in the following paragraphs of this specification, by means of illustrative example only, without intending to constitute any limitation of the scope of the present invention. The following description may be better understood when read in conjunction with the attached drawings, in which:

Figure 1 Flowchart for determining the slurry management strategy that best suits the field situations. Adapted from 3tres3, Flotats Ripoll, 2019.(13)

Figure 2 Press screw diagram, with indication of nomenclature and data used to calculate efficiency. Printed by 3tres3, Flotats Ripoll, 2019. The data in red are theoretical actual measurements of the slurry processing system analyzed. Interrogations are the magnitudes that we can estimate.

Figure 3. Design of on-site modular biotechnology platform for the processing and transformation of the animal waste. Classification of biological and contaminant risk by intensity of grey color: darkness (high), middle (medium) and softness (low). Legend: circles (homogenization processes); triangle (solid-liquid separation processes), squares (storage of raw materials and products); oval (cultivation operations), fine lines (flow of liquid fractions), coarse lines (flow of solid fractions). The numbers correspond to the actions described in Table 3.

Figure 4. MS 100 SEPARATOR. Image from Mec6niques Segalés S.L., Gurb, Spain.

Figure 5. Bioreactor 3L (BioBundle) (left) and its Ez-Control controller (right). Note that the computer is on standby and unplugged.

Figure 6. Bioreactor 100L (left) and cooling/heating recirculator (right). Note that the computer is on standby and unplugged.

Figure 7. Open rectangular pool-type bioreactors. (A) Example of the system assembled in a 1200 L-culture capacity outside facility. (B) Example of the system assembled in a 50 L-culture capacity inside facility. Both examples are producing *Arthrospira platensis paracas.*

Figure 8. Open tubular FBR station (up to 1,500 L) at the Mediana de Aragón experimental station. The system is presented at rest, without operation. Each tubular system has a filtration pump and UV lamps. It can be closed or not depending on the light requirements of the strain under study.

Figure 9. Basic design scheme of the on-site modular biotechnology platform for the processing and transformation of the animal waste at Mediana de Aragón swine farm. The slurry flow is marked in red, and the homogenization and microalgae culture flow are green. There are two homogenization processes, at the entrance of the demonstrator and at the exit.

Figure 10. Model laboratory station based on <50L-culture capacity inside facility with medium-scale inoculum preparation system. A. Injection of *Artrosphira platensis paracas* into flasks and beginning of formation of floating structures in microalgae dispersion. B. Laboratory demonstration with controlled light (12 hours light / 12 hours darkness). The two flasks on the left are injected with water and culture medium (50/50). The two central mills with EMRA-derived slurry-based colloid media. 28-day trial.

Figure 11. Comparative culture of *A. platensis* in EMRA-processed slurry and Zarrouk medium in the laboratory. Conditions: 3000 lux intensity, 12:12 hour photoperiod (light/dark), temperature 30 °C and manual agitation.

Figure 12. *A. platensis paracas* growth in FBR cultures inside a clean room with two luminaires inside the reactor.

Figure 13. Relative growth rate of microorganisms classified by temperature ranges. The red box indicates the vegetative growth temperature range reported in the literature. Modified from Blanco et al. 2015.(58)

Figure 14. Growth control of *A. platensis paracas* seeded in an open raft-type reactor outside.

Figure 15. (A) Control photobioreactor (FBR) (open pond), culture of *A. platensis* in 650 L in synthetic sea salt medium day 0 (D0). (B) EMRA-processed slurry FBR (open pond), culture of *A. platensis* (from original dilution 1:1 v/v). Conditions: light intensity 3000-4000 lux, 27 °C, photoperiod 12 on/12 off and constant oxygenation.

Figure 16. Comparative *A. platensis* growth between Spirulina-specific media culture (control) and EMRA-derived slurry-based colloid media culture (from original dilution 1:1 v/v) in 1.000L photobioreactors (FBR) (open ponds).

Figure 17. Diagram of the proposed experiment (A) and the three solutions used for the experiment (B): network water from Mediana de Aragón (left), culture water with *A. platensis* (center) and EMRA-derived slurry-based colloid media (right).

Figure 18. Seed germination, day 2 (D2). Condition 1 (A): Mediana de Aragón drinking water; Condition 2 (B): culture water with *A. platensis;* Condition 3 (C): EMRA-derived slurry-based amendment. It was observed that germination began in all conditions.

Figure 19. Seed germination, day 4 (D4). Condition 1 (A): Mediana de Aragón drinking water; Condition 2 (B): *A. platensis*-specific media culture; Condition 3 (C): EMRA-derived slurry-based amendment. Not only did the seed germinate, but it was observed that it was slowly growing and turning to take root. In the case of condition 2 (green) it was confirmed that the seeds were increasing in size but were taking longer to germinate.

Figure 20. Day 4 (D4). Seedbeds with transparent paper to conserve moisture inside.

Figure 21. Vegetative growth, day 15 (D15). Condition 1 (A): Drinking water from Mediana de Aragón. Replicas 1 and 2. The seedlings continued to grow.

Figure 22. Fungal growth, day 15 (D15). Condition 2 (B): culture water with *A. platensis*. Replicas 1 and 2. Fungi were observed in all wells and no germinated seeds were observed.

Figure 23. Vegetative growth, day 15 (D15). Condition 3 (C): EMRA-derived slurry-based amendment. Replicas 1 and 2. The seedlings continued to grow.

Figure 24. Number of replicates sent for analysis of *Cicer arietinum* crops.

Figure 25. Seed samples of the "white" condition ready for nutritional analysis. Remember that these samples were not subjected to any treatment, and their use is to determine the starting point of the used seeds.

Figure 26. Physicochemical analysis of the tested culture conditions of *Cicer arietinum* for 18 days.

Figure 27. Foliar biomass reserved for chlorophyll analysis.

## DETAILED DESCRIPTION OF THE INVENTION

The present invention relates, in a first aspect, to an on-site modular system for processing and transforming animal waste, comprising:

(a) a treatment unit for treating animal waste comprising a solid/liquid separation unit in order to obtain a slurry-derived liquid animal waste fraction;

**(b)** a treatment unit for homogenizing liquid animal waste fraction of unit (a) comprising a high-pressure homogenization and/or sonication unit in order to obtain a homogenized nutrient-rich liquid fraction;

**(c)** a culture unit for culturing microalgae by using the homogenized nutrient-rich liquid fraction obtained in the unit (b) as a culture medium;

**(d)** a unit for collecting and processing the microalgae culture produced in unit (c) comprising a high-pressure homogenization and/or sonication unit in order to obtain a homogenized nutrient-rich microalgae-derived colloid suspension;

**(e)** a unit for separating solid/liquid homogenized nutrient-rich microalgae-derived colloid suspension produced in unit (d) comprising a solid/liquid separation unit and/or a microfiltration unit in order to obtain two byproducts:

(1) processed rich-nutrients biologically absorbable nitrogen-rich phosphorus-rich potassium-rich liquid microalgae culture fraction, and (2) processed rich-carbon solid inactivated microalgae culture fraction;

**(f)** a control and monitoring unit for monitoring cultivation conditions and processing efficiency.

In the context of the present invention, the term "on-site modular system" means the set of mechanical, physical and biotechnological systems and units used in the processing and transformation of animal waste located within the intensive production farm, closely related to the ponds and storage sites for organic waste.

In the context of the present invention, the term "slurry-derived liquid animal waste fraction" means the homogenized (i.e. sonicated) nutrient-rich biological carbon molecules-fragmented liquid fraction and separated from the solid particles in suspension of animal waste from an intensive farm, that is used as a microalgae culture medium in the form of a colloidal suspension with low microbiological burden.

In a preferred embodiment, the microalgae are selected from *Arthrospira platensis, Chlorella vulgaris* or a combination thereof.

The on-site modular system for processing and transforming animal waste (also defined herein as EMRA) has been proven to be effective producing microalgae in mediterranean continental climatic area, where cultured microalgae may include but not being limited to *Arthrospira platensis, Chlorella vulgaris* and other microalgae with the ability to grow in animal waste-derived nutrient-rich media, exemplified by a 10-98%-rich *Arthrospira platensis* culture production, in a range of temperature from 10 to 30 °C, a range of light intensity from 1500 to 3000 lux and a range of photoperiod from 6 to 12:6 to 12 hours (light/dark).

The on-site modular system for processing and transforming animal waste according to the present invention is a promising candidate to resolve the need of (a) saving animal alimentary cost (microalgae as new food ingredient) (conversions of at least 0.5 to 3% of slurry into feed), (b) producing new incomes for farmers (high-tech rich-nutrients biologically absorbable nitrogen-rich phosphorus-rich potassium-rich liquid phytoremediator, up to 75% of total produced slurry), and (c) avoiding significant greenhouse gas emissions (composed but not restricted to ammonia ($NH_3$), nitrous oxide ($N_2O$), methane ($CH_4$), carbon dioxide ($CO_2$) and hydrogen sulfide ($H_2S$), from 10 to 80% of total greenhouse gas emissions).

In a particular embodiment, the on-site modular system for processing and transforming animal waste consists of entirely constituted modular interconnected tubular closed transforming platform composed but not restricted to semi-automatic pumps for liquid management and secondary deposits for mechanical and physical slurry and culture modifications.

In another preferred embodiment of the system of the invention, the solid/liquid separation unit in unit (a) and unit (e) comprises at least one of the followings: a particle size and gravity separation unit (static or rotary sieve, grid, drum, settling), a pressure separation unit (screw press, filter press), and centrifugal separation unit. The efficiency of these units is measured in terms of mass flow rates (from 1 kilogram onwards) and the separation of nutrients loads such as nitrogen and phosphorus (from 1% onwards).

The units (b) and (d) of the system of the invention comprise a high-pressure homogenization and/or sonication unit for the liquid and culture fractions. Both homogenization units are used (1) to improve the efficiency of animal waste processing and transforming, and (2) to optimize the culture media of microorganisms, including microalgae. This homogenization unit may be based on but not restricted to any device using stirring blades and/or sound waves to break down cellular structures and release intracellular materials, which is useful for fermentation, digestion, and other organic matter conversion processes. Key sonication platform parameters include sonication amplitude (at least 80), sonication time (starting on cycles of at least 45 seconds or continuous flow), number of sonication cycles (at least 5 cycles or continuous), and sonication mode (on/off). Sonication technique is more effective for the homogenization of microalgae cultures, as it improves the dispersion of solids, the emulsification of liquids, mass transfer, and the rate of reaction.

In another preferred embodiment of the system of the invention, the culture unit (c) comprises at least one open or closed photobioreactor, optionally including an aeration unit, wherein the at least one open or closed photobioreactor includes a temperature control unit. In a further preferred embodiment, the at least one open or closed photobioreactor is a tubular, flat plate or pool-type bioreactor. This optional aeration unit is for controlling bubble agitation and gases distribution, which also improves the exchange of microalgae, between light and dark zones, improving thereby light transfer to the culture. The photobioreactor may be equipped with low speed stirring blades to avoid the deposition of microalgae and fouling of the reactor walls when the culture reaches high concentrations. The photobioreactor may be equipped with pumps to control gradients in pH and nutrient concentration.

In another preferred embodiment of the system of the invention, the temperature in the at least one open or closed photobioreactor is between 10° and 35 °C, preferably between 21 and 31 °C. This temperature is usually controlled by a temperature control unit by means of a coil immersed inside the culture, which allows them to work in a thermostatic manner.

In another preferred embodiment of the system of the invention, the unit (d) and unit (e) comprise a filtration unit, a drying unit and extraction unit. These units allow to obtain the compounds of interest, such as lipids, proteins, and carbohydrates.

In another preferred embodiment of the system of the invention, the control and monitoring unit (f) comprises at least one sensor and actuator to control culture parameters, nutrient concentration and greenhouse gases emissions.

In a second aspect, the present invention relates to a process for transforming animal waste *in situ* using the on-site modular system according to the first aspect of the invention including any of the embodiments disclosed herein alone or combined, comprising the steps of:

(i) treating animal waste for obtaining a homogenized nutrient-rich liquid fraction;
(ii) culturing microalgae in a culture unit using the homogenized nutrient-rich liquid fraction of step (b) as the culture medium;
(iii) collecting and processing the microalgae culture produced in step (c);
(iv) collecting and processing the homogenized nutrient-rich microalgae-derived colloid suspension produced in step (d);
(v) collecting and processing the processed rich-nutrients biologically absorbable nitrogen-rich phosphorus-rich potassium-rich liquid microalgae culture fraction and the processed rich-carbon solid inactivated microalgae culture fraction after step (d).

In a third aspect, the present invention relates to the use of the processed rich-nutrients biologically absorbable nitrogen-rich phosphorus-rich potassium-rich liquid microalgae culture fraction obtained according to the process of the second aspect of the invention including any of the embodiments disclosed herein alone or combined as animal feed.

As a way of example, the process for obtaining this animal feed is detailed as follows:

Step 1. To treat animal waste to obtain a liquid fraction of slurry by solid-liquid separation unit. The slurry separation unit may be interconnected with the in-house slurry pond exit and both, the external slurry solid fraction storage pond and homogenization tanks. The target is to separate at least a 97% of liquid nutrient-rich fraction of original deposited slurry.

Step 2. To homogenize the nutrient-rich liquid fractions of slurry by stirring blades, high pressure, or sonication. The homogenization unit may be interconnected with solid-liquid slurry separation unit and fermentation bioreactors. The target is to reduce significantly original deposited microbial burden and to fractionate and disperse nutrient-rich nitrogen-rich phosphorus-rich potassium-rich microbial structures into a colloid media.

Step 3. To cultivate microalgae in a controlled culture unit using the nutrient-rich nitrogen-rich phosphorus-rich potassium-rich colloid dispersion as the culture medium. The culture unit may include open or closed photobioreactors, including but not limited to tubular, flat plate or pool-type bioreactors, interconnected with homogenization tanks and culture solid/liquid filtration unit. The target is to transform the low-microbial burden nutrient-rich colloid media into a rich-nutrients biologically absorbable nitrogen-rich phosphorus-rich potassium-rich pH-controlled liquid microalgae culture.

From step 3 onwards it is noteworthy to mention that original liquid fraction of slurry cannot be no longer considered as animal depositions waste but a transformed media to microalgae culture. Therefore, EMRA-cultured microalgae may be used as a potential food ingredient, pendent of regulation as new animal feed ingredient.(59, 60)

Step 4a. To collect and process the microbial biomass produced in the culture unit (feed ingredient) by using culture fractions separation unit (filtration or similar). The culture filtration unit may include microfiltration or tangential filtration tubular pumps or similar interconnected with culture unit and both, a solid biomass storage deposit (fresh microalgae) and a liquid culture fraction storage tank. The target is to separate solid culture fractions to be used as an on-site food animal ingredient and to storage supernatant liquid culture fraction.

In a third aspect, the present invention relates to the use of the processed rich-nutrients biologically absorbable nitrogen-rich phosphorus-rich potassium-rich liquid microalgae culture fraction obtained according to the process of the second aspect of the invention including any of the embodiments disclosed herein alone or combined, as phytoremediators of agriculture or natural soil amendments.

As a way of example, the process for obtaining this phytoremediators of agriculture or natural soil amendments is detailed as follows:

Step 1 to 3. As previously described for feed animal.

Step 4b. To homogenize the total microbial culture by homogenization unit (stirring blades, high pressure, or sonication). The same homogenization unit of step 2 may be interconnected with culture unit and the liquid culture fraction storage tank, after an intermediate operational cleaning step to remove slurry-derived detritus. The target is to break down microalgae and to get a significantly lower amount of biologically active microorganisms in the processed culture.

Step 5. To separate liquid/solid homogenized culture to obtain a colloidal culture-derived liquid fraction by filtration unit or similar. The same culture filtration unit of step 4a may include microfiltration or tangential filtration tubular pumps or similar interconnected with liquid culture fraction storage tank and both, a solid biomass storage deposit (fresh cellular debris) and a final product storage tank, after an intermediate operational cleaning step to remove culture-derived detritus. The target is to transform the low-microalgae burden nutrient-rich pH-controlled colloid cultured media into a rich-nutrients biologically absorbable nitrogen-rich phosphorus-rich potassium-rich liquid microalgae-derived culture fraction to be used as phytoremediator.

Step 6. To storage newly formed phytoremediator until its use. The final product storage tank may be interconnected with homogenization unit and a flowmeter-equipped dispenser. The target is to storage and dispenser effectively a rich-nutrients biologically absorbable nitrogen-rich phosphorus-rich potassium-rich liquid microalgae-derived culture fraction to be used as phytoremediator.

The present invention further relates to the use of the processed rich-nutrients biologically absorbable nitrogen-rich phosphorus-rich potassium-rich liquid microalgae culture fraction obtained according to the process of the second aspect of the invention including any of the embodiments disclosed herein alone or combined as recycler of toxic or contaminants in animal waste farm.

The present invention further relates to the use of the processed rich-nutrients biologically absorbable nitrogen-rich phosphorus-rich potassium-rich liquid microalgae culture fraction obtained according to the process of the second aspect of the invention including any of the embodiments disclosed herein alone or combined, as fresh compost of agriculture or natural soil amendments.

The present invention further relates to the system according to the first aspect of the invention including any of the embodiments disclosed herein alone or combined in a farm for reducing greenhouse gas emissions and carbon emissions therein, composed but not restricted to ammonia ($NH_3$), nitrous oxide ($N_2O$), methane ($CH_4$), carbon dioxide ($CO_2$) and hydrogen sulfide ($H_2S$). This reduction is achieved by applying the system of the invention during the process of emptying inner slurry pools in swine pens (sanitary recommended max. storage of 20 days) and coupling the system of the invention to the external common slurry pond. The system coupling with regular inner slurry emptying avoids at least 100 days of open-air residues fermentation (legal open-air max. storage of slurry use to be 120 days).(10)

The system and process of the invention for processing and transforming animal waste has other environmental benefits. High-pressure homogenization and/or sonication and filtering reduce the microbial load along the process, preventing environmental contamination by slurry-derived or cultured microorganisms. In addition, the cultivation of microalgae eliminates open-air slurry-fermentation derived odors (significant decrease of $H_2S$). The system is also efficient in the transformation of waste, turning animal waste into a source of nutrients for microalgae cultivation. These waste-derived nitrogen-rich microalgae can be used as animal feed ingredients, providing a circular sustainable solution for animal waste management, and feeding.

It is noted that any of the embodiments disclosed herein can be taken alone or combined with any other embodiment disclosed herein unless the context specifies otherwise. In other words, for example, a preferred option of a defined feature can be combined with a more or less preferred option of another feature.

The invention will be now illustrated with the following examples, which do not intend to limit its scope.

## EXAMPLES

### Design description of the proposed solution: Proof of concept

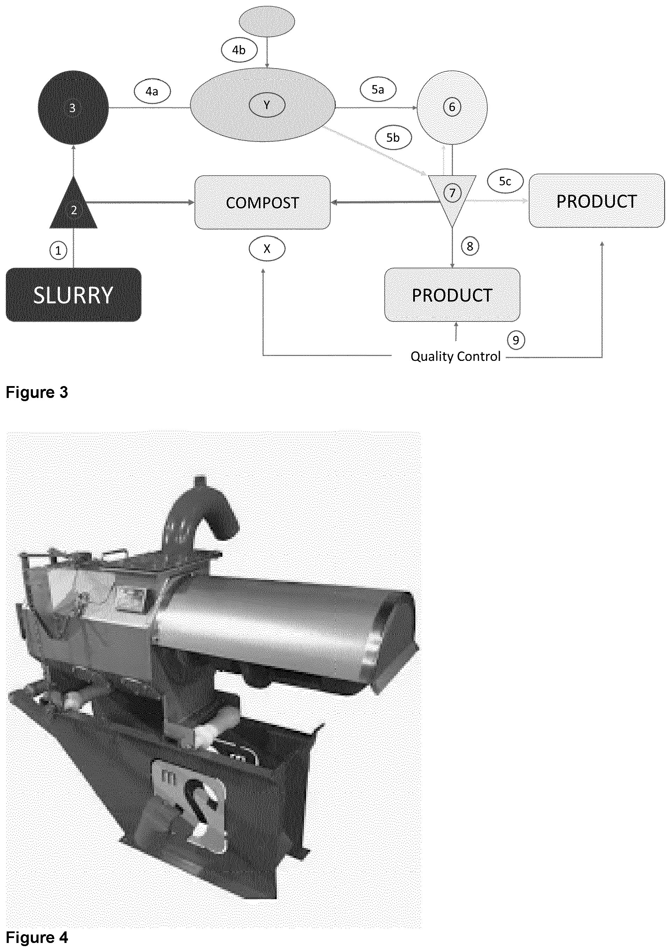

The design of the on-site modular system for the processing and transformation of the animal waste (EMRA) has involved an innovative disrupting coupling various technologies together, resulting in a new efficient and self-sustainable combination for the generation of high-valued slurry-derived by-products. To do so, it must meet several concurrent Quality by Design criteria, listed in Table 2. Briefly, we have designed a high-level sequentially ordered robust extensively tested technological platform for on-farm slurry processing (Figure 3.) The general protocol of EMRA actions is set out on Table 3.

**Table 2. Quality by Design parameters that the Modular Waste Station of Aragon (EMRA) proposed to be viable in the pig industry must comply with.**

| Parameter | Description |
|---|---|
| Efficient | The solution must be efficient in utility processes, energy-sustainable and aimed at net-zero greenhouse gas emissions. |
| Scalable | All designed solutions must be adaptable regardless of the number of animals on the farm. There is no number below which the pig industry ceases to be polluting, but there are numbers that make it sustainable. |
| Easy to use | Farmers need low-maintenance semiautomatic systems that can be operated with basic training. Therefore, closed automatic culture systems are adaptable candidates to meet this criterion. |
| Modular | The farm needs can change over time, so can the waste station. In addition, various EMRA-based operational modules may be required depending on the circumstances, and not always, within the animal production process. |
| Safe | EMRA must have safety mechanisms in place against leaks, accidents, electric shocks, fires, etc. Full control of mechanical, electrical, and biological systems must be guaranteed. |
| Cheap | The installation and maintenance of EMRA, including supplies, must not exceed the costs currently involved in the disposal and processing of slurry in current integrated management systems, and must even minimise them. |
| Sustainable | Ideally, the system should aim for self-management and net-zero emissions; In addition, the generation of new products with intrinsic value of their own can be treated as a new source of revenue. |

The mechanical elements that may make up EMRA are (actions in Table 3):

1. Transfer and liquid handling pumps (all actions except 3, 6 and 8).
2. An industrial high pressure and/or ultrasonic homogenizer (actions 6 and 8).
3. A separator of solid and liquid fractions by filtration or similar (actions 2 and 7).
4. An open or closed photobioreactor, including but not limited to tubular, flat plate or pool-type bioreactors to culture the microalgae that demonstrates better performance under the studied conditions (action Y).
5. A primary conditioning system of the resulting product (liquid culture fraction <LCF> - phytoremediator) (action 8).
6. A composting tank that allows the use of solid slurry fraction (SSF) and solid culture fraction (SCF) in the fertilization calendar related to management slurry plan of the farm (action X).
7. Machinery for handling composted solids (action X).
8. A pond for microalgae harvesting and subsequent inactivation (actions 5b and 5c).
9. A quality control unit to control EMRA-derived byproducts (all actions).

**Table 3. Sequence of actions (#) designed for the processing of slurry by microalgae remediation at the Aragón Modular Waste Station (EMRA) and main achieved objectives.**

| # | Action | Description | Main Objectives |
|---|---|---|---|
| 1 | Slurry extraction | Animal waste is extracted from the in-house slurry storage ponds or authorized waste containment system by means of transfer pumps | To avoid the formation of surface crusts by constantly moving the slurry out of the ponds. |

(continued)

| # | Action | Description | Main Objectives |
|---|--------|-------------|-----------------|
| 2 | Separation of solid and liquid fraction of slurry (SSF and LSF) | Applying physical separation systems, the solid fraction of slurry (SSF) (3-4%) is added to a composting tank (step X) and the liquid fraction of slurry (LSF) in suspension (96-97%) is decanted to the homogenization system. | To facilitate the maintenance of the photobioreactor due to less solid waste (high acidity). Balance SSF infundibulum's for composting (step X). To homogenize LSF by cavitation, high pressure or stirring blades. |
| 3 | Homogenization of the LSF | By means of high-intensity ultrasound, high pressure or stirring blades, EMRA break up as many infundibula (slurry microorganisms) as possible; the resulting suspension of substances is dispersed on a colloid media. | Significant decrease of viable microorganisms in the LSF. To avoid subsequent competition phenomena when growing with the microalgae strains of choice. To improve culture quality. |
| 4a | Injection of LSF into photobioreactors (PBR) | Conventional transfer pumps sequentially inject the LSF into the FBR; A 10% vol/vol ratio of inoculum has been shown to be the better to culture optimization. | To culture microalgae in a low-burden microorganism nutrient-rich colloid media so that it stays in the growth phase. |
| 4b | Injection of FBR with microalgae of choice | (*) In situations of first injection of an FBR, regrowths or after maintenance operations, it will be necessary to inject again the volume of inoculum necessary to grow the microalgae (10%). | Uprooting of algae growth. To resurrect the FBR after maintenance operations. To refresh production due to strain depletion. |
| Y | CULTURE MAINTENANCE IN 20 DAYS | | |
| X | SLURRY SOLID FRACTION (SSF), AND SOLID PARTICLES OF COLLOIDAL CULTURE-DERIVED LIQUID FRACTION (SPC) (1-4% total slurry) ARE PLACED ON COMPOSTING TANK FOR 100 DAYS (PRODUCT) | | |
| 5a | Extraction of X volume of entire FBR culture | Conventional transfer pumps sequentially extract the entire culture into an auxiliary tank or, preferably, directly into the homogenization system. | To harvest X volume from the entire FBR culture, reducing the concentration of microalgae. To keep the microalgae in a continuous growth phase. |

(continued)

| # | Action | Description | Main Objectives |
|---|--------|-------------|-----------------|
| 5b | Separation of solid and liquid fraction of the culture (SCF and LCF) | By applying physical separation systems, the solid culture fraction (SCF) (3-4%) is added to a processing blaze; the liquid culture fraction (LCF) in suspension (96-97%) is decanted to the homogenizer. | To separate SCF for using in animal food or obtaining biomolecules. |
| 5c | Inactivation and Primary Conditioning of Fresh SCF | By applying mechanical systems (desiccation, physical breakage, homogenization) or chemical systems (osmotic shock) the fresh SCF is inactivated and added to the container (primary packaging) where the product is stored. | To process SCF for using in animal food or to obtain biomolecules. To prepare the product for analysis and subsequent distribution. |
| 6 | Homogenization of the entire culture of microalgae growth in PBR | By means of high-intensity ultrasound, high pressure or stirring blades, EMRA break up as many microalgae and viable growth infundibula as possible; The resulting suspension of substances is dispersed homogeneously. | To break down microalgae vegetative form and potential viable growth infundibula. Significant liquid stabilization to obtain colloidal culture-derived liquid fraction. To improve product quality (increase NPK:C ratio available for plants) |
| 7 | Separation of solid particles and liquid fraction of the colloidal culture-derived liquid fraction (SPC and LPC) for composting | Applying physical separation systems, the solid particles of colloidal culture-derived liquid fraction (SPC) (3-4%) is added to a composting tank (step X); and the liquid fraction of the colloidal culture-derived liquid fraction (LPC) (96-97%) is decanted to the container (primary conditioning) where the product will be stored. | To facilitate the primary conditioning of the product (LPC) (phytoremediator). To storage in composting pond the SPC. |
| 8 | LPC Primary Conditioning | Fill the containers (IBD, transport tanks, etc.) with the product (LPC) in suspension. | Prepare the product for analysis and subsequent distribution. |
| 9 | Quality Control | Measurement of total nitrogen (N), phosphorus (P) and potassium (K) concentrations, in addition to the organic carbon concentration (fatty acids, proteins) (C) of the resulting LPC. | To have an analysed semifinished product to which corrections can be made for use as a phytoremediator or nutrient. |

## EXAMPLE 1. DESIGN, DEVELOPMENT AND SCALE UP OF THE MICROALGAE PRODUCTION PROOF-OF-CONCEPT

The present invention concerns a modular biotechnological platform for *in situ* recycling and transforming animal waste, specifically slurry, through the production of microalgae and its derivatives cultured on sequentially physical-processed liquid slurry-derived fractions. This innovation makes it possible to obtain high value-added by-products, such as nutrients for animal feed and a new phytoremediator for agricultural and natural soils amendments.

*Arthrospira platensis paracas* (txid634502) (61) growth in such slurry-derived colloid media had not been used so far in any previous project, nor had similar results been published in indexed journals. However, laboratory conditions (500 mL) had been established for its basic cultivation in water and regular slurry. As part of the project, our R+D team has established them in basic cultivation medium and fresh slurry. Parameters such as the pre-treatment of the slurry without added water contributions or volumetric correction outside of aqueous pig residues separated from solids, the study of the lighting and aeration of the culture or kinetic changes of the same in the face of environmental stressors were modified (temperature, reactor opening/closing cycles, pH-correcting salts or not, etc.) Likewise, the precise engineering elements were designed to model the experimental production facility built in Mediana de Aragón 1:1,000. The study of the natural variation of pH and its correction kinetics was subjected to special tests at smaller scales (150 L), before moving on to the main semi-closed tanks and FBRs (2,000 L) (example 1). The theoretical products obtained (intraplasmic lipids, cytoplasmic residues and complete microalgae) were characterized to establish the starting matter for by-products validation and is one of the main research results (example 2).

## 1.1. Solution formulation and design of full-scale cultivation operations

With the execution of example 1, EMRA carbon capture and slurry removal solution was designed and validated through scaling *Arthrospira platensis paracas* culture, controlled addition of substrates and carbon sources and monitored production of by-products. Carbon capture and slurry removal solution has been designed and based on validated scaling criteria,(62, 63, 64) and the study of the mechanism and materials used in the proof-of-concept.

The demonstrative facility for the implementation of the new greenhouse station coupled to a modified slurry drain system have been designed and graphed to add a substrate and carbon source to the microalgae in a controlled manner. The technical design of the cultivation operations has been carried out in a close-cycle intensive farm of 180 sows and their piglets (Granja de Experimentación Tecnológica S.L. -B02859924-, ES50164.1, Central Region of Zaragoza, Mediana de Aragon, Spain).

## 1.2. Development and implementation of the proposed EMRA proof-of-concept at Mediana de Aragón

Described design elements in Tables 2 and 3 and Figure 3 were used to design and define the implementation phase at the experimental facility. Critical points of the platform were controlled and established following EMRA design descriptions cited on Summary of Invention. The solution we have proposed to the problem of slurry is phytoremediation with microalgae, which generate chlorophyll by capturing organic and environmental carbon, by treating wastewater and producing by-products.

### 1.2.1. Equipment description used to validate liquid slurry-derived colloidal fraction as microalgae culture media from 3L to 1.000L photobioreactor.

Selected designed-as-essential mechanical elements in Table 3 were provided by specialized companies. Installation of the mobile industrial homogenizer (it is a non-fixed, temporary, mobile equipment that was retired after carrying out the homogenization tasks) and its coupling by pipes to the intermediate settling ponds inside the greenhouse at Mediana de Aragón.

The solid/liquid separation unit used was the MS 100 SEPARATOR (Mecàniques Segalés S.L., Gurb, Spain) (Figure 4.) It is a specially designed equipment for pigs, with an optimal functioning with a slurry that does not exceed 5% solids. It had a filter that incorporates a special thread to obtain a higher percentage of dry matter in the solid fraction (35%). Depending on the homogenized material of origin, it may be able to obtain a yield of 3-20 $m^3$/H, with a range of electricity consumption of 4 kW per 6 - 30 $m^3$/H. It is equipped with security pressing regulation unit with automatic stop in case of clogging of the filter and sieve by compression. The inner sieve achieves a separation in the liquid slurry fraction (LSF) of up to 250 microns. A mobile station was installed on the slurry ponds of Mediana de Aragón to separate the 20 $m^3$ used in the experiments carried out in this research. Manufacturer gives as an example of energy consumption a 2000 fattening places farm whose consumption ranges about 2.45 kW/H (from 0.02 to 0.03 €/$m^3$), which represents less than 100 €/year.

The homogenization unit used was the UIP2000hdT-230 (2000 W) industrial ultrasonic processor (Hielscher Ultrasonics GmbH, Teltow, Germany) This equipment works continuously by recirculating industrial liquids and sludge on protected tubular water in an acoustic insulation box. It is capable of processing 65,000 L every 6 hours. For this reason, it becomes the ideal system for the homogenization of liquid slurry fractions and liquid culture fractions in EMRA (Figure 1.) Ultrasonic processor was coupled to the outlet of the separated liquid slurry fraction to continuously sonicate and directly feed the microalgae culture in the FBRs inside the greenhouse. The energetic cost of sonicating 65 $m^3$ of liquid slurry fraction for less than 2 kW/H.

Filtering mechanism were composed on 200 to 0,45 $\mu$m sequential sieves to separate by high pressure-directed microfiltration and decantation (in-house system, GET, Spain). The system coupled recirculating pumps with liquid filters, separating up to 97% of liquid fractions. Problems related to filter obturation and discontinuous flow in this in-house system may suggest that a tangential microfiltration semiautomatic system shall obtain better results. Currently, research team is working on ongoing experiments confirming such hypothesis.

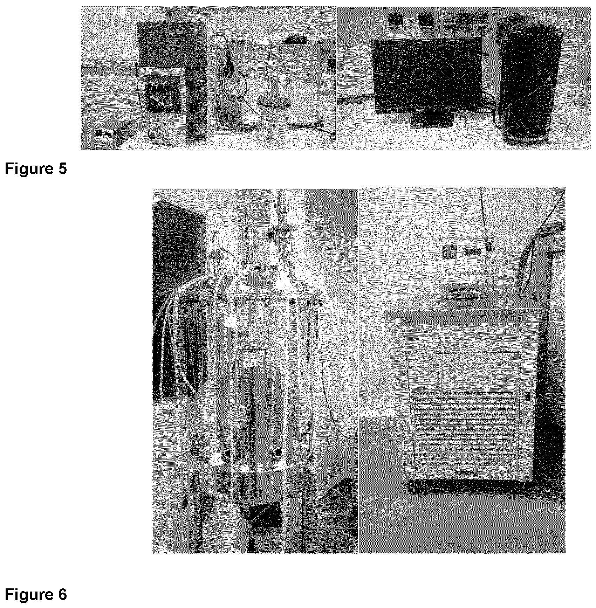

Photobioreactors of 3L (BioBundle 3L Bioreactor Tubular & Ez-Control Process Controller, WP0173, Figure 5) and 100L (Biorreactor 100L tubular & DHW/AFS recirculator WP0175, Figure 6) were used for the generation of GLP master and working seed for the selected strains and for the optimization of the resuscitation, recovery, and initial culture operations of the microorganisms under study. BioBundle consists of a 316L SS irox steal 3L-capacity add-on-pack jacketed reaction assembly and a 3-rotameter system with a selenoid valve for the addition of components. The BioXpert Lite control system is complemented by an ADDA card, I/O extension, which allows the complete digitalization of the fermentation process. Bioreactor 100L tubular consists of a 316L SS iron steal 100L-capacity add-on-pack jacketed reaction assembly with multiple sensors and configuration, coupled to a DHW/AFS recirculatory unit. Both systems are configured to measure and control pH, temperature, dissolved oxygen (DO), level/foam, and agitation. The 3L fermenter and the software used have sensors for pH, temperature, dissolved oxygen, and level/foam.

This equipment has some essential characteristics for the technological validation of cultivation processes such as those presented in this report:

- All materials in contact with the culture are constructed of 316L SS (stainless steel), EPDM, silicone rubber, PTFE, or borosilicate glass.
- All of these materials have been proven to be non-toxic and chemically inert in tests conducted by the supplier.
- The 3-liter BioBundle is fully validable for cGMP application. The Ez-Control was developed and documented in accordance with the GAMP4 industry standard.
- The system is delivered with all appropriate documentation required to operate the system, including operating manuals, general maintenance instructions, parts lists, and selected drawings.

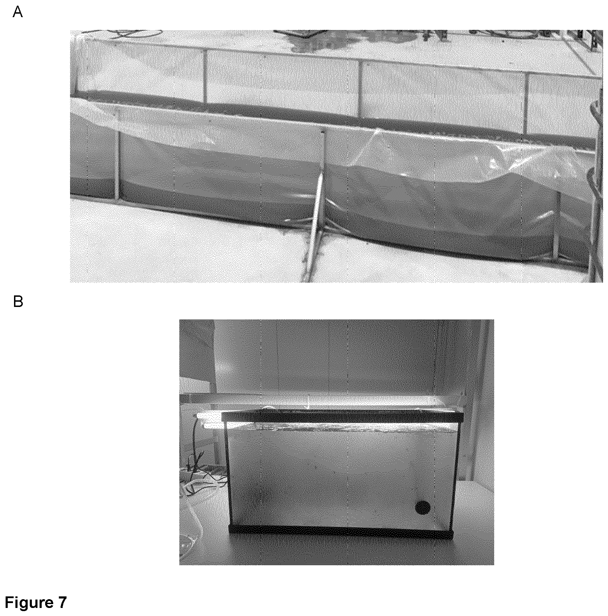

Research teams used open-air rectangular or circular ponds to scaleup the modular platform and validate onsite management of the system. Square ponds consisted of open culture system designed in open polyethylene outdoor rafts. To this end, a rectangular swimming pool was manufactured with an AISI 316 stainless steel structure with dimensions of 1 × 4 × 0.4 meters and was installed in proof-of-concept facility. This structure was clad in a flexible polyethylene-type plastic material and protected with a polyester mesh to reduce environmental pollution. The reactor had an aeration system at the bottom that allowed the algae to move and prevent it from being deposited (Figure 7.)

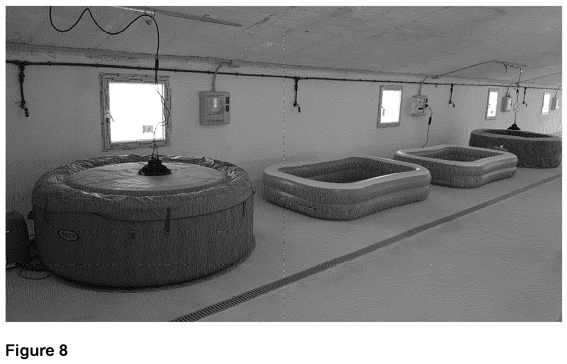

The circular pools consist of a reinforced polyethylene system, with a semi-automatic filter and transfer system, which recirculates the cultured medium continuously. Light control is done with ultraviolet lamps with programmable on/off (not shown in photo). Note that the semi-open FBR system allows for better aeration and temperature transmission. The correction of salts for pH is done manually by adding to the transfer pump that comes with the equipment and preparing the solution in the clean room of 1-5L inoculum. The temperature is maintained between 5-25°C in order to progressively accustom the microorganisms to the climatic conditions of Mediana (Figure 8.)

### 1.2.2. Scaleup criteria for EMRA platform validation

Aerobic culture scaling presents inherent challenges in the control and monitoring of reaction conditions to obtain the desired product.(31, 32, 33) To achieve this goal, the cultivation conditions of the strains chosen and selected for study have been thoroughly studied from the laboratory to the pilot scale (2L). The aim was to properly transfer this process to the industrial scale while minimizing the risks of failure.(31, 33, 37) Several scaling criteria were fixed depending on the types of FBR used in the project (Table 4.)

**Table 4. Scaling criteria applied to the types of FBR used in this project, tubular or rectangular (raft). Note that if two or more FBRs could not apply common scaling criteria, scalability based on this criterion was rejected.**

| Criteria | Tubular | | | | Pond | | Scale up |
|---|---|---|---|---|---|---|---|
| Volume | 3L | 100L | 100 L | 1.200L | 2.000L | 10.000L | **Scale up** |
| Location | Interior | Interior | Exterior | Interior | Exterior | Exterior | |
| Oxygen Transfer Coefficient (kla) | Constant | Constant | Constant | Ambient | Ambient | Ambient | Fixed by Resemblance |
| Power per unit volume (Pg/V) | Constant | Constant | Constant | Constant | Constant | Constant | Fixed by Resemblance |

(continued)

| Criteria | Tubular | | | | Pond | | Scale up |
|---|---|---|---|---|---|---|---|
| Volume | 3L | 100L | 100 L | 1.200L | 2.000L | 10.000L | **Scale up** |
| Location | Interior | Interior | Exterior | Interior | Exterior | Exterior | |
| Tangential stirring speed (p N Di) | Constant | Constant | Not applicable | Constant | Not applicable | Not applicable | Not applicable |
| Reynold Number Constancy (N Di2 r/m) | Constant | Constant | Constant | Constant | Not applicable | Not applicable | Not applicable |
| Aeration Pumping Speed (F/V) | Gas Constant | Gas Constant | Gas Constant | Open pumping | Open pumping | Open pumping | Fixed by Resemblance |
| Geometric Similarity | 1 | 1:100 | 1 | 1:1000 | 1:200 | NA | Fixed by Resemblance |
| Percentage of dissolved oxygen | 21% | 21% | 21% | 21% | 21% | 21% | Fixed by Resemblance |
| Gas Flow Per Unit Volume of Culture Medium (vvm) | Constant | Constant | Constant | Ambient | Ambient | Ambient | Fixed by Resemblance |

**1.2.3. Experimental designs and control parameters used on proof-of-concept trials.**

Experimental design was conducted to optimize culture parameters of microalgae, modelized by *Arthrospira platensis paracas* growing, in EMRA-related 1.000-L-sized conditions. Control parameters and experimental conditions optimized used on proof-of-concept trials are indicated in Table 5. All results and conclusions obtained in this example were consistently checked for robustness (at least x3 operators) and repeatability (at least x3 times) during 2022 and 2023. Only relevant results are included in the resume of conclusions.

**Table 5. Control parameters and experimental conditions optimized used on proof-of-concept trials. Actions numbers and order (#) are taken from Figure 3. Measures are indicated: Liter (L), Hour (H), Kilograms (Kg)**

| # | Action | Control parameters | Experimental conditions |
|---|---|---|---|
| 1 | Slurry extraction | Slurry flows towards solid/liquid separation unit (L/H) | Slurry L / H |
| 2 | Separation of solid and liquid fraction of slurry (SSF and LSF) | Solid fraction of slurry (SSF) (Kg/H) / liquid fraction of slurry (LSF) separation (L/H) | SSF Kg / H LSF L/H Chemical Oxygen Demand (COD) $(mgO_2/L)$ |
| 3 | Homogenization of the LSF | Nutrient-rich low-burden microorganism colloid media (L/H) | Sonication to erase 99% infundibula vegetative forms (20-30 kHz, 80 A, 15 sg-2 min, X cycles) Volume sonicated (L/H) |
| 4a | Injection of LSF into photobioreactors (PBR) | 1-10 L of inoculum (10%). | Light (UV) pH (5-9) Dissolved oxygen (L/H) |

(continued)

| # | Action | Control parameters | Experimental conditions |
|---|--------|--------------------|--------------------------|
| 4b | Injection of FBR with microalgae of choice | 50-100 L of inoculum (10% total FBR volumen). | Light (UV)<br>pH (5-9)<br>Dissolved oxygen (L/H) |
| Y | CULTURE MAINTENANCE IN 20 DAYS | | |
| X | SLURRY SOLID FRACTION (SSF), AND SOLID PARTICLES OF COLLOIDAL CULTURE-DERIVED LIQUID FRACTION (SPC) (1-4% total slurry) ARE PLACED ON COMPOSTING TANK FOR 100 DAYS (PRODUCT) | | |
| 5a | Extraction of X volume of entire FBR culture | Entire culture flows towards the homogenization system (L/H). | Biomass mg / L<br>FBR culture L / H |
| 5b | Separation of solid and liquid fraction of the culture (SCF and LCF) | Solid culture fraction (SCF)<br>(Kg/H) / Liquid culture fraction<br>(LCF) (L/H) is decanted to the homogenizer. | SCF mg / L<br>LCF mL / L<br>Separation process (L / H) |
| 5c | Inactivation and Primary Conditioning of Fresh SCF | Solid culture fraction (SCF) (Kg/H) inactivation. | SCF processing timing (H) |
| 6 | Homogenization of the entire culture of microalgae growth in PBR | Targets:<br>(1) processed rich-nutrients biologically absorbable nitrogen-rich phosphorus-rich potassium-rich liquid microalgae culture fraction, and (2) processed rich-carbon solid inactivated microalgae culture fraction | Sonication to erase 99% infundibula vegetative forms (20-30 kHz, 80 A, 15 sg-2 min, X cycles)<br>Volume sonicated (L/H) |
| 7 | Separation of solid particles and liquid fraction of the colloidal culture-derived liquid fraction (SPC and LPC) for composting | Solid particles of colloidal culture-derived liquid fraction (SPC) (Kg/H) / liquid fraction of the colloidal culture-derived liquid fraction (LPC) (L/H)<br><br>Target (2) processed rich-carbon solid inactivated microalgae culture fraction (product 2) | SPC mg / L<br>LPC mL / L<br>Kg product 2 (*)<br>Separation process ( Kg-L / H) |
| 8 | LPC Primary Conditioning | Target (1) processed rich-nutrients biologically absorbable nitrogen-rich phosphorus-rich potassium-rich liquid microalgae culture fraction. | L product 1 (*) |
| 9 | Quality Control | (*) laboratory methods adapted to the physical product specifications. | Total nitrogen (N)<br>Total phosphorus (P)<br>Total potassium (K)<br>Organic carbon concentration (fatty acids, proteins) (C) COD (mgO$_2$/L) |

### 1.2.3.1. Cultivation of *Arthrospira platensis paracas*

Different conditions for the growth of *A. platensis paracas* primary inoculum were tested in laboratory by using Zarrouk liquid culture medium. Medium characteristics and its chemical composition are detailed in several reviewed papers.(34, 49, 65) In addition, additional culture experiments were conducted in *Salinity*™ liquid medium to optimize microalgal growing.

### 1.2.3.3. Growth Assessment Using Spectrophotometry

Growth assessment of *A. platensis paracas* cultures was carried out by periodically taking 1 mL aliquots from the cultures and performing optical density (OD) readings with a spectrophotometer at wavelengths of 730 nm to measure turbidity and 665 nm to measure chlorophyll.

### 1.2.3.4. Growth Assessment Using Light Microscopy

Growth of *A. platensis paracas* cultures evaluation was performed qualitatively, placing a drop of culture on a slide and observing the sample under an inverted microscope with a 20x magnification objective. Acceptable growth of *A. platensis paracas* provides images of green colonies and intact spiral structures characteristic of this species. Instead, the visualization of discolored colonies was indicative of photoinhibition.

### 1.2.3.5. Determination of culture growth by Dry Weight

For cultures larger than 50 mL, an aliquot of an adequate volume was taken, ensuring homogeneous suspension of cyanobacteria. The sample was then transferred to a 50 mL conical bottom tube and centrifuged at 7000 rpm for 30 min. at room temperature. On the other hand, filter paper was weighed after drying in an oven at 100 °C for 12 hours and placed in a funnel. The supernatant was then filtered and once all the liquid was eluted through the filter, the pellet was transferred.

Subsequently, 2 to 3 washes were carried out with distilled water to remove traces of salts from the culture medium and when it was observed that the filter did not contain liquids, it was transferred to a watch glass and dried in an oven at 100 °C for 12 h. Finally, the filter paper together with the cyanobacteria was left to temper and weighed. The difference in mass between the mass of the dry filter and the mass of the filter containing *A. platensis paracas* resulted in the mass of the latter in the initially aliquoted volume.

### 1.2.3.6. Study of the effect of photoperiod and culture medium on the growth of the chosen strain.

After optimizing the conditions for growing the algae in the most optimizable medium, it was considered to make changes in the lighting conditions to adjust the energy consumption during the subsequent scaling. In addition, the first adaptation test to the slurry present on the farm was carried out and the obtaining of an alternative culture medium (*Salinity*™ artificial seawater) for the generation of primary injected inoculum in tanks larger than 25L was modelled.

### 1.2.3.7. Evaluation of the 5, 50, 100 and 2.000L cultivation units.

Once the cultivation and optimization conditions of *A. platensis paracas* under laboratory conditions and with controlled temperature were known, it was decided to establish the yields in photobioreactors ranged from 5 to 100 L biosecurity level 2 (NBS2) laboratory facilities, in order to evaluate whether the adaptation of the algae and its inoculation in slurry at room temperature, starting from an inoculum grown in Salinity™ artificial seawater, was possible to obtain sustainable yields. After initial upstream and complete microalgae adaptation, primary inoculum were injected into FBR up to 2.000 L to optimize microalgae culture in different experimental conditions.

### 1.2.3.8. Cryopreservation

One (1) mL of culture was transferred to a cryovial along with 1 mL of 10% methanol (2 units of each species were preserved). Immediately, the cryovials were placed in a cold block at - 20 °C to be transferred to a freezer at -80 °C where they were preserved for 1 hour. After this time, they were transferred to a container and/or Dewar with liquid nitrogen (-196 °C) for preservation.

### 1.2.4. Schemes for the basic implementation and location of EMRA at Mediana of Aragon.

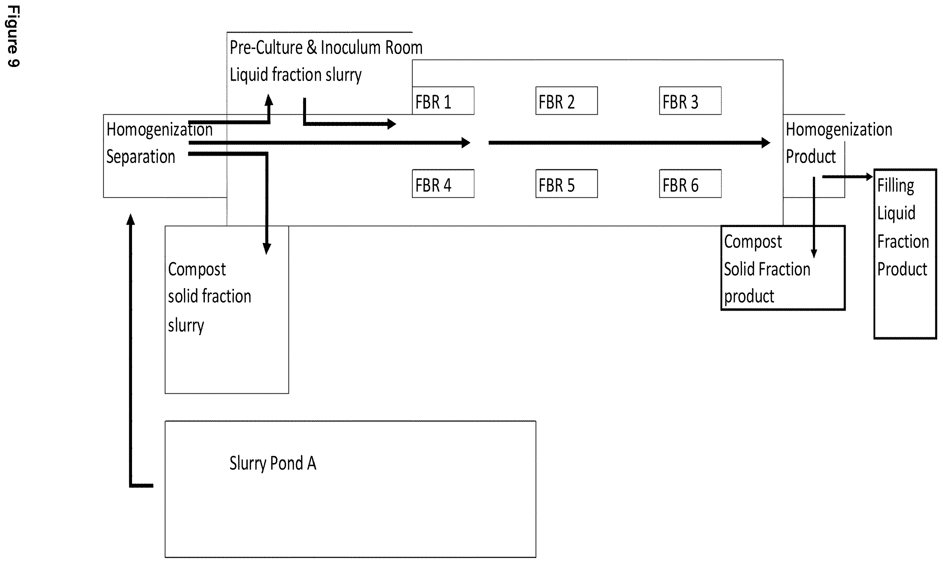

Implementation planning of the laboratory demonstration of the new greenhouse station coupled to a modified slurry drain have been designed and graphed to add substrate and carbon source to the microalgae in a controlled way. Basically, a laboratory system has been designed that can be attached to slurry ponds, the most common way of storing this manure on production farms. The basic outline is presented in Figure 3. The basic outline of experimental facility in Mediana de Aragón is set in Figure 9. Strain preparation and inoculum laboratory room were designed to support the experimental production facility built at Mediana de Aragon 1:1,000. Proof-of-concept was monitored and analyzed from March to November 2021, 2022, and 2023 (this last year, until September).

### 1.2.5. Modular station for the inclusion of indoor tubular photobioreactors and their auxiliary installations.

Photobioreactors (FBR), both rectangular pool type (1,200L outdoors/ 50L indoors) and tubular (open pool-type bioreactors, 2,000L indoors), were installed and controlled at experimental facility. A lighting internal circuit composed by eighteen panels (18) was installed to guarantee appropriate luxes supply to culture FBR and culture ponds.

### 1.2.6. High-contaminated challenging slurry present in Mediana de Aragón experimental swine farm.

The concentration of aluminum, copper, manganese, selenium and zinc in the drinking water from the network that reaches Mediana de Aragón, and by extension the farm, exceeds the parameters allowed by the water quality standard (data no shown).(66)

The concentration of silver, aluminum, arsenic, boron, barium, beryllium, cadmium, cobalt, chromium, copper, iron, lithium, manganese, molybdenum, nickel, lead, rubidium, anthodium, selenium, tin, titanium, vanadium and zinc in GET slurry exceeds the parameters allowed by the Spanish water quality standard (data no shown).(66)

Nominal composition parameters of slurry have been established in our operating conditions.(12, 13) High concentration of various heavy metals related to the stabilization of paints, construction materials and traces of chemical disinfection and coating compounds were detected on the slurry, and that can be related to poor farm management during many years of lack of maintenance. The company GET is young (24 months at the time of writing) and acquired the 50-years-old farm at the end of 2021. Since then, it has made a significant effort to rehabilitate the infrastructure, facilities and equipment of the swine farm it acquired. The condition of the farm affected the employees (respiratory problems) as well as the animals (increase in mortality compared to the industry averages). During this time of existence, the company has implemented a biosecurity, maintenance and management plan approved by local and regional authorities that has managed to reverse all environmental toxic levels in the form of volatile gases, drinking water and water pipes on farm buildings. Detected-on-excess toxics have been considerably reduced to their practical control (data no shown). However, the presence of these toxic concentrations of heavy metals in the slurry storage ponds, practically impossible to remove completely, were carefully considered for the result interpretation of the rest of the project's actions.

Solid slurry fraction used in the research was particularly high (49%), compared to the average slurry content in the areas of Aragón and Catalonia, which is usually around 30% solid fraction.(13) This could be due to the confluence of several circumstances: (i) on the one hand, a greater loss of water by direct evaporation (excess heat for the summer season during the sampling phase); (ii) increased leakage losses (during the autumn renovation works, cracks were found in the main blaze; and (iii) a lower load of animals during the sampling season that allowed the longer permanence of "old" slurry in the ponds, which contributes to natural caking and drying (costrification) of the slurry. In any case, the higher concentration of solids may be related to a higher nominal concentration of substrates and contaminants in the slurry, complicating its use as a phytoremediator.

Nitrogen dioxide is a toxic, irritating gas and precursor to the formation of nitrate particles. These lead to the production of acids in the environment. It mainly affects the respiratory system. The annual limit set by European directive is 40 micrograms $m^{-3}$. Under conditions of high ammonium concentration, it establishes an inconstant equilibrium that reflects an aging of the slurry, which makes this sample the most representative of the conventional storage systems existing in the study area.(13) These parameters concentrations can be reduced by anaerobic and aerobic denitrification processes. The biological removal of ammonia nitrogen is applied to the liquid slurry fraction and is generally carried out by combining two processes catalyzed by different microorganisms:

- Nitrification (N): autotrophic aerobic microorganisms, which obtain carbon from $CO_2$ or bicarbonate contained in slurry, convert $NH^{4+}$ into nitrites ($NO^{2-}$) and nitrates ($NO^{3-}$)
- Denitrification (DN): Another group of heterotrophic microorganisms convert $NO^{2-}$ and

$NO^{3-}$ into $N_2$ gas, using organic matter as a source of carbon and energy.

According to the studies reviewed on this system, system efficiency is highly variable since nitrifying bacteria are very sensitive to temperature changes. A well-operated system, regulated according to temperature and variations in the composition of the slurry, can have an average nitrogen removal of 60% in the form of $N_2$ with respect to the nitrogen of the liquid fraction separated in the initial Solid/Liquid separator. It is common for a fraction in the order of 3-7% of nitrogen to escape in the form of $NH_3$ and $N_2O$, which should be avoided.(17, 50, 62) GET currently produces 9,219 Kg of nitrogen per year, measured in accordance with the environmental standards of Aragón.(67, 68) Through biological nitrogen removal technologies, a fertilizer resource is lost, at the cost of consuming energy, contrary to the principles of sustainability and circular economy.(3, 63) For this reason, it is not considered a best available technology (BAT) for new livestock farms.(13)

Chemical demand for oxygen (COD) is the amount of oxygen needed to chemically oxidize organic matter into $CO_2$ and $H_2O$. The higher the COD, the more polluted the water is.(66, 69) This result in the test slurry indicates that strong aeration is necessary to consume all the organic matter, which in principle would not be indicated in our study conditions, which aim to convert organic matter and environmental $CO_2$ into fatty acids and algal proteins that are likely to be consumed or used in other processes.

Recommended electrical conductivity of greywater used for agricultural applications should be less than 1000 μS/cm. The ideal pH for irrigation water should be between 6.5 and 8.4 pH, so in principle, Mediana slurry meets this condition (data not shown).

Anyway, Mediana farm slurry was a real challenge to process for the innovative on-site modular biotechnology platform for the processing and transformation of the animal waste.

### 1.3. Resume of validation results of the liquid slurry-derived colloid media experimental proof of concept in 200 mL to 2.000 L microalgae aerobic culture model: *Arthrospira platensis*

Production of aerobic culture variables in large volumes have been investigated and fixed in parameters such as temperature, agitation, aeration and oxygenation conditions, homogenization of colloid slurry-derived culture media. Optimal culture times have been established as reaching the highest possible yields for spirulina in the scaling facilities. Ecological platform behavior was checked on conventional photoreactors (Figures 5 and 6) and rectangular flatter cultures surfaces (Figures 7 and 8.) The behavior of *Chlorella vulgaris, Anabaena sp., Nannochloropsis gaditana* and *Scenedesmus* sp. was analyzed under the extreme weather conditions suffered in the proof-of-concept experimental facilities during study period (spring 2022 to autumn 2023).

Except for *Arthrospira platensis paracas* (txid634502),(61) none of the strains initially chosen have managed to grow in the culture media resuspended in Mediana de Aragón network water or in the processed GET slurry-derived fraction. This conclusive result is surprising because of the behavioral antagonism observed in other cases, especially for *Chlorella vulgaris*,(19, 70) *Scenedesmus sp.(71) o Nannochloropsis gaditana,(72)* in which growth consistent with slurry and runoff water in pigs has been observed. Culture media composition has shown a very marked effect on the yields of *Chlorella vulgaris*,(35, 73, 74) This could point to a dramatic effect of the contaminants detected, both in water and in slurry, on the viability of the algae under these conditions. We could also observe an effect of excess solid content in the slurry used for the research, which can modify the cultivation patterns of the microalgae under study.(75, 76, 77) In any case, the growth conditions of any microorganism must be subjected to the reality observed in the field,(50, 57, 78, 79) to avoid technological failures when it comes to transferring the system to the real world. After this empirical verification, we chose *Arthrospira platensis paracas* to carry out the rest of the tests.(61) We have selected and presented in this example *Arthrospira platensis* as a resistant strain to grow and bioremediate the high-contaminated challenging slurry of Mediana de Aragón (continental Mediterranean climate).

### 1.3.1. About the candidate strain for EMRA: *Arthrospira platensis paracas*

Optimal conditions for the cultivation of *Arthrospira platensis* in circular or rectangular FBR up to 100 L were set out after a complete set of experimental trials. Obtained optimal results are summarized in Table 6. Based on the scaling elements reviewed in activity 1.2.2. (Table 4.), we prepared inoculum optimization criteria for higher volumes in *situ;* in Pre-culture & primary inoculum room at Mediana de Aragón (Figure 9.)

Microalgae need to be subjected to some light exposure due to their photosynthetic nature, however, it has been described in the literature that prolonged exposures of light inhibit the growth of the microorganism.(49) These data correlate with the data observed in this project at 16 hours of exposure (lower growth) versus 12 hours of exposure (higher growth). After a trial of optimization experiments, *A. platensis paracas* culture conditions were finally set at a 12/12 photoperiod, intensity of 3000 lux, photoperiod of 12:12 hours (light/dark), 30°C, manual aeration and cultivation in Zarrouk medium. Microalgae growth results were achieved between 2.43 - 3.29 g $L^{-1}$, or in other words, 2.67 $\pm$ 0.42 g $L^{-1}$, which means reaching and exceeding the best yields observed for similar strains in the literature consulted, namely 4.055 mg $L^{-1}$ to 2.3 g $L^{-1}$.(34, 80, 81, 82, 83)

Using the Arthrospira-specific medium with highly contaminated drinking water and liquid slurry-derived fractions from Mediana de Aragón in cultures outside FBR, we obtained a growth rate of *Arthrospira platensis paracas* between 0.06 $\pm$ 0.001 g $L^{-1}$ (low volumes) and 0.05 $\pm$ 0.02 g $L^{-1}$ (high volumes), which made it the ideal candidate to model the proposed solution in an highly-contaminated environment, that has demonstrated manifest hostility in several microalgae culture and cyanobacteria (*Chlorella vulgaris, Scenedesmus* sp., *Anabaena sp.,* and *Nannochloropsis gaditana*). This production is within the previously reported growth ranges for similar strains of this microalgae: 4,055 mg $L^{-1}$ (80) a 1,5 g $L^{-1}$ (81), and even 2.3 g $L^{-1}$ in special lighting conditions.(34) Optimized cultivation conditions in the proof-of-concept produced media and environments that have shown the best performance are listed in Table 6. These yields and conditions, obtained with controlled bioreactor models and continental inland environmental conditions, reliably exceed the data obtained so far by the reviewed literature on *Arthrospira* sp. ensuring that we can scale this strain cheaply, sustainably and for various economically profitable purposes.

**Table 6. Preferential culture conditions and yields in photobioreactors of less than 100 L for *Arthrospira platensis paracas* in Mediana de Aragón (GET).**

| Condition | Results |
|---|---|
| Temperature (°C) | 26-34 |
| pH | 9,5-10 |
| Lighting (hours) | 12/12 LED o natural |
| Growth (g/L) | 0,1-0,9 |
| Maximum vegetative stage (g/L) | 2,67 $\pm$ 0,42 |
| Stationary phase (in days) | 26,7-47,6 |

### 1.3.2. About the conditions of the slurry-derived colloid medium and results of the on-site modular biotechnology platform proof-of-concept

Solid slurry fraction (SSF) obtained at Mediana has been particularly high (49%), compared to the average SSF in the areas of Aragón and Catalonia, which is usually around 30% SSF.(13) This could be due to the confluence of several circumstances: (i) on the one hand, a greater loss of water by direct evaporation (excess heat for the summer season during the sampling phase); (ii) increased leakage losses (during the renovation works of autumn 21, cracks were found in the main basin); and (iii) a lower load of animals during the sampling season, which allowed the longer permanence of "old" slurry in the ponds and under grids (inner slats of buildings), which contributes to natural caking and drying (crostification) of the slurry. In any case, the higher concentration of SSF may be related to a higher nominal concentration of substrates and contaminants in the slurry, complicating its use, direct and unprocessed, as a phytoremediator. Separation systems are also less efficient with aged slurry, especially due to the problems of settling and caking that are observed in the interior ponds of the farms, which could have had to do with the yields and results observed with the microalgae studied that did not show any growth.(13)

Nitrogen dioxide ($NO_2$) is a toxic, irritating gas and precursor to the formation of nitrate particles. Under conditions of high ammonium ($NH^{4+}$) concentration, it establishes an inconstant equilibrium that correlates with slurry aging; the more gas, the older slurry.(13) These parameters concentrations can be reduced by anaerobic and aerobic denitrification processes. Ammonia nitrogen biological removal is applied to the liquid slurry fraction and, under the conditions tested with *Arthrospira,* is carried out by nitrification (N). *Arthrospira platensis paracas* is an autotrophic aerobic microorganism that obtains carbon from $CO_2$ or bicarbonate contained in slurry and converts ammonia ($NH^{4+}$) into nitrites ($NO^{2-}$) and nitrates ($NO^{3-}$). According to the studies reviewed on other strains of *Arthrospira* sp., system efficiency is highly variable, as nitrifying bacteria are very sensitive to temperature changes. It is common for a fraction of the order of 3-7% of nitrogen to escape in the form of ammonia ($NH_3$) and $NO_2$, which should be avoided in the proposed model.(17, 50, 62) On the farm used as a proof-of-concept, 9,219 Kg N year$^{-1}$ is currently produced, what it is in accordance with the environmental standards of Aragón. This nitrogen is usable by *Arthrospira platensis paracas* to convert it into organic nitrogen absorbable by plants.(67, 68) Through highly-extended biological nitrogen removal technologies of slurry in conventional farms, a fertilizer resource is lost at the cost of consuming energy, what it is contrary to the principles of sustainability and circular economy.(3, 63) For this reason, *removing* nitrogen is not considered a best available technology (BAT) for new livestock farms.(13) Our solution *fix* the slurry nitrogen in the form of amino acids and proteins (microalgae growth), consuming carbon from the suspended solid matter of the liquid slurry fraction (colloid media) (feeding) and from the environment during the process (respiration).

One of the most obvious results of the project has been the high impact of slurry contamination in the viability and yields of microalgae culture. Original non-processed slurry COD was greatly increased, which leads to a high degree of contamination.(66, 69) The scale-up conditions for the effective comparison of the different culture agitation test and volumization systems have been established by similarity (Table 4.) with the following parameters: i) oxygen transfer coefficient (kla) as constant (low volumes, indoor) or environment (large volumes, indoor or outdoor); ii) tangential stirring velocity (p N Di) as constant (low volumes, indoor) or not applicable (large volumes, indoor, or outer); and (iii) aeration pumping rate (F/V) as constant by gas (low volumes, indoor) or by ambient pumping (large volumes, indoor or outdoor). These parameters have been set to improve the general oxygenation of the cultures (regardless of their volume), and to help eliminate or buffer part of the contamination detected in original non-processed slurry, a fact that most likely may be behind the relatively low growth that we have observed in original non-processed slurry derived *Arthrospira* cultures. The relevance of oxygenation has been evident, since, on two occasions, various incidences in the culture aeration caused (a) the death of the Spirulina or (b) the entry into the seasonal phase (data no shown). Even so, we have been able to convert LSF-derived organic matter and environmental $CO_2$ into fatty acids and algal proteins that can be consumed as rich-nutrients biologically absorbable nitrogen-rich phosphorus-rich potassium-rich liquid phytoremediator.

Recommended electrical conductivity of greywater used for agricultural applications should be less than 1000 $\mu$S/cm. The ideal pH for irrigation water should be between 6.5 and 8.4 pH, so in principle, original non-processed slurry met this condition. It has been possible to adequately control the pH in all the cultivation conditions tested, we believe that due to the decrease in the concentration of vegetative phases of microorganisms in the liquid slurry-derived colloid media, but the fact is that there have been no deleterious effects when climbing from early stages, as described in other research. The pH conditions have been set out in Table 6.

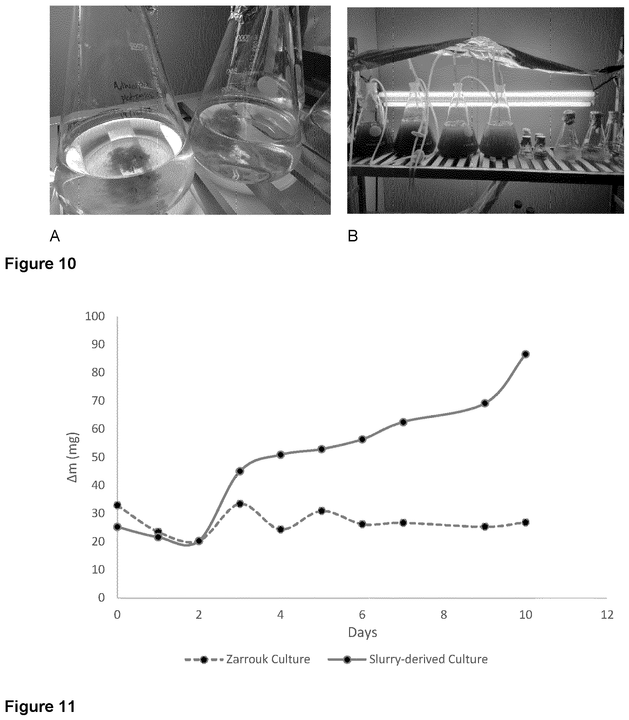

Laboratory *Arthrospira* cultures were optimized comparing Zarrouk media (specific for this microalgae) and EMRA-processed slurry. A remarkable increase in growing was observed in EMRA-processed slurry-derived *Arthrospira* in <5L laboratory cultures with 3000 lux intensity (LED), 12:12 hour photoperiod, 30°C and manual agitation (Figure 11.) These parameters were used as initial experimental conditions to scaleup EMRA process.

### 1.3.3. About scaling up results and the critical parameters to control

The first experimental culture trials of *A. platensis* were carried out in open systems, in which the culture was mechanically agitated with a paddle wheel. This type of system had certain limitations: (i) As it was an open culture system, the temperature was irregular, therefore, productivity could decrease. (ii) It had limited light availability due to a low surface-to-volume ratio, which caused shadowing between the microalgae, reducing the cellular lighting flow. (iii) Biomass harvesting (filtration) was costly and unfavorable, given that very low amounts of biomass were produced. (iv) As it was a non-isolated system, it was more susceptible to contamination. (v) As it was not an automated system, it was more complicated to optimize the resources obtained.(84) Against this system, and taking into account the aforementioned drawbacks, the use of FBRs as closed systems for cultivation was considered as a possible solution to the problems posed in open systems. One of the most important contributions of the FBRs designed in this proof-of-concept was the direct incorporation of culture lighting systems with an adequate surface-to-volume ratio.(85) An *Arthrospira platensis paracas* growth pattern significant improvement has been established in the laboratory FBRs, whose growth pattern in the laboratory has not been possible to transfer completely to the slurry-derived colloid media. One of the limiting factors for achieving high levels of biomass production from *A. platensis* is the availability of suitable FBRs, hence we have tested various models and systems (described on 1.2.1.)(86)

Our results show that, in specific cultures free of heavy metals of <5L and using a photoperiod of 12:12 hours (light/dark), a stabilization of the growth of *A. platensis paracas* was observed over time, achieving microalgae growth results between 2.43-3.29 g L$^{-1}$, or 2.67 $\pm$ 0.42 g L$^{-1}$. These results reach and exceed the best yields observed in the reviewed literature, namely 4.055 mg L$^{-1}$ to 2.3 g L$^{-1}$.(34, 80, 81, 82, 83)

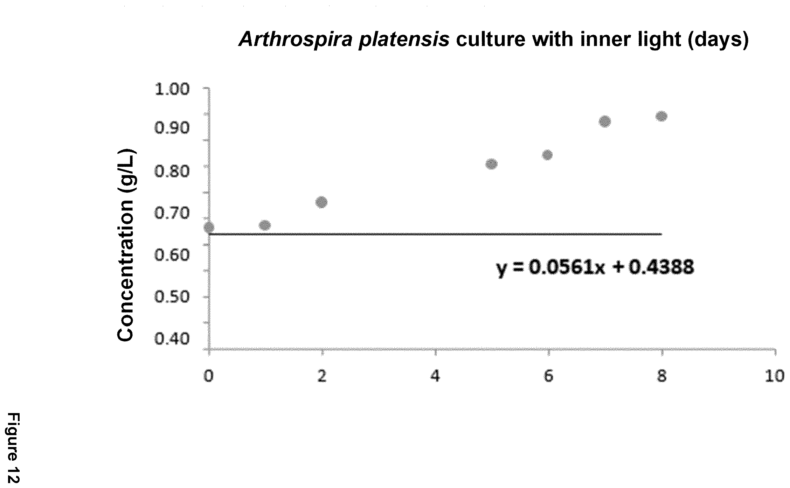

In 5-25 L trial cultures performed on indoor tubular FBRs with EMRA nutrient-rich low-burden microorganism colloid media, an *Arthrospira* growth of 0.1 $\pm$ 0.06 g L$^{-1}$ day$^{-1}$ should be considered, maintaining an effective range of 10.53 $\pm$ 0.14 pH (Table 7) and in outdoor environmental conditions of 30.43 $\pm$ 1.34 °C for 25L outdoors (26.7 days to theoretical maximum); and a growth of 0.0561 g·L$^{-1}$·day$^{-1}$ maintaining an effective pH range of 9.5 (Figure 12) and under controlled indoor conditions of 29-34 °C irradiated with LED lights in 25L indoors (47.6 days to theoretical maximum). These unexpected growths were observed in conditions of very marked ambient heat and changes in relative humidity in the area, with higher-than-expected irradiations for the time of year, which could exceed 45°C at specific times of the day. In <100L FBR, growths of 0.8-0.9 g L$^{-1}$ were obtained with constant natural irradiation, forced aeration at 0.4L min$^{-1}$ and 26 $\pm$ 0.5 °C controlled temperature. Repeating the trial in autumn, culture yields varied between 0.05 and 0.07 g L$^{-1}$ day$^{-1}$, much lower than those achieved in summer.

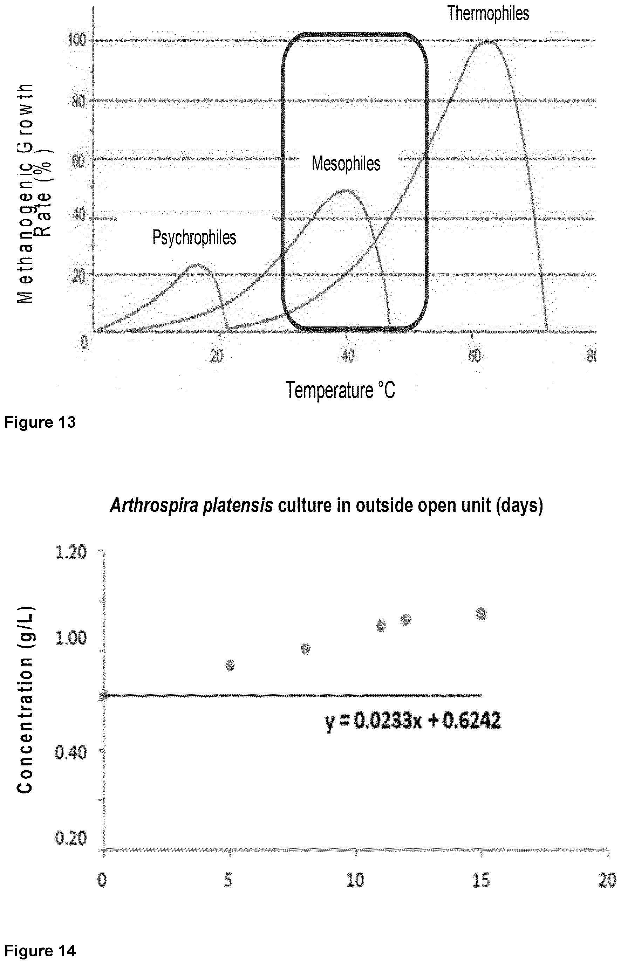

Anaerobic FBR culture temperature can be operated in three different ranges: psychrophilic (ambient temperature, below 20°C), mesophilic (temperature between 30 and 40°C) or thermophilic (temperature greater than 40°C).(58) By increasing temperature, the growth rate of microorganisms increases, as shown in Figure 13. As a result of the increased growth of microorganisms, the production speed of by-products (biogas, biomass, biochars) is higher and retention times in reaction tanks decrease.

**Table 7. Culture yields, (g L$^{-1}$) observed daily growth, and initial (i) and final (f) pH conditions in outdoor photobioreactors seeded in summer. Note that the FBRs collected ambient temperatures and had full natural light capture. Data collected at the outdoor tubular FBRs. Data are shown in mean $\pm$ standard deviation ($\sigma$).**

| TRIAL | FBR (n) | g L$^{-1}$ | g L$^{-1}$ day$^{-1}$ | pH*i* | pH*f* | T*media* (°C) |
|---|---|---|---|---|---|---|
| A | 3 | 0,3 | 0,03 | 10,2 | 10,4 | 31,6 |
| B | 4 | 0,4 | 0,15 | 10,0 | 10,6 | 30,4 |
| C | 5 | 0,2 | 0,09 | 9,8 | 10,5 | 30,0 |
| D | 4 | 0,4 | 0,11 | 10,0 | 10,6 | 29,7 |

(continued)

| TRIAL | FBR (n) | g L$^{-1}$ | g L$^{-1}$ day$^{-1}$ | pH$i$ | pH$f$ | T$media$ (°C) |
|---|---|---|---|---|---|---|
| N ± σ | 16 | 0,33 ± 0,07 | 0,10 ± 0,06 | 10,0 ± 0,14 | 10,53 ± 0,14 | 30,43 ± 1,34 |

*Arthrospira* usually growths in places with quite extreme conditions with a very high alkalinity (pH=11) making it impossible for other organisms, both animals and plants, to live. Therefore, *Arthrospira* reaches its maximum productivity surviving in alkaline pH media. By surviving in such extreme conditions, it is considered a very easy species to grow since it tolerates pH variations remarkably.(77, 87)

These results on photoperiod and temperature associated with lighting correlate with those previously obtained for *Arthrospira,* in which culture growth kinetics showed a wide range of temperature tolerance from 20 °C to 40 °C. The maximum growth rate, i.e. cell production with maximum accumulation of chlorophyll and phycobiliproteins, was found at a temperature of 35 °C and a light intensity of 2000 lux.(88, 89) Regarding the growth temperature, and as mentioned above, the strain of *A. platensis paracas* used in these trials develops in quite extreme environments, as original non-processed slurry or high-contaminated drinking water. This characteristic is not only reflected in the pH but also in the temperature, having a higher growth range between 24 and 35 °C, although the optimum is obtained at a temperature of 35 °C.(88) In our conditions, the best yields have been obtained at around 30 °C, which implies a huge energy expenditure in cold times of the year.

Photoinhibition can lead to problems in the top layer of outdoor grow pools, the temperature distribution of which will depend on agitation. Vomshak and Guy's experiments (90) in outdoor cultures of strains sensitive to photoinhibition have shown that shadowing the culture with nets increased the daily photosynthetic rate by up to 35%. Other studies concluded that the strong solar radiation incident on the double-curved photobioreactor culture system affects the morphology and productivity of *A. platensis.*(91) It is important to consider that light intensity per cell varies considerably depending on the growth density. The amount of light available to one cell will depend on the shadowing of other cells. This means that light can become a limiting factor in very dense culture. This variation in light intensity during growth makes it convenient, in indoor FBR, to adjust the number of lamps as the culture develops. Agitation of the culture also contributes to improving this aspect.(87) Proper agitation of the culture medium is essential for the growth and development of the microalgae. When the requirements are met and the environmental conditions are satisfactory, agitation is the most important requirement for obtaining high yields of microalgal biomass.(77) Perhaps the absence of agitation in conditions of high density of fecal solid matter (49% in our test conditions, with Mediana slurry), has caused the shading and lack of lighting, adjusted to a relative lack of aeration outdoors, to decrease the growth range to the maximum 0.1-0.9 g L$^{-1}$ day$^{-1}$ observed in our preliminary test conditions.

However, these factors alone do not explain the lack of performance observed from laboratory to semi-industrial scales. The surprising results of environmental contamination present both in the drinking (tap) water and in the original non-processed slurry of Mediana de Aragón make us think about its probable influence on the observed growth. This effect was evident in the lack of growth and inhibition of the vegetative phase of *Chlorella vulgaris, Anabaena sp., Nannochloropsis gaditana and Scenedesmus* sp. Microalgae have been shown to effectively remove nitrogen (90-98%), phosphorus (66-98%), zinc (16-99.7%), chromium (52-96%) and mercury (77-97%) from polluted aquatic systems. It is possible to find in the literature strains of algae capable of reducing metals such as Fe, Zn and Cd by 85%, 95% and 99%, respectively, or even 100% eliminations of Al and Cu.(92) In our test conditions we have selected *Arthrospira platensis paracas* as a resistant strain to grow (sharp pH, temperature and lighting variations) and bioremediate highly contaminated slurry.

Culturing *Arthrospira platensis paracas* outdoors in an open culture system consisting of open outdoor polyethylene rafts was contemplated in a first approach to large-volume scaling. Following the methodology applied in the previous tests (colloid media, preparation, methods of analysis), a specific-media culture growth of 0.0233 g·L$^{-1}$·day$^{-1}$ of *Arthrospira* in the outer pond (Figure 14) with thermal inversions of 33 (night)-46 (day) °C was obtained. Comparing this result with that obtained in the closed reactor where the inoculum was prepared (25 °C), which yielded a result of 0.0335 g·L$^{-1}$·day$^{-1}$ (data not shown), we observe that we have lost growth capacity. Obviously, having a thermostatic system allows a significant increase in the degree of growth of the algae, being in the order of 43% higher. However, this growing reduction achieved in outdoor rafts could not be explained solely by the variation in temperature, and further experiments are still pending to find out the reasons for this decrease with respect to the central inoculum.

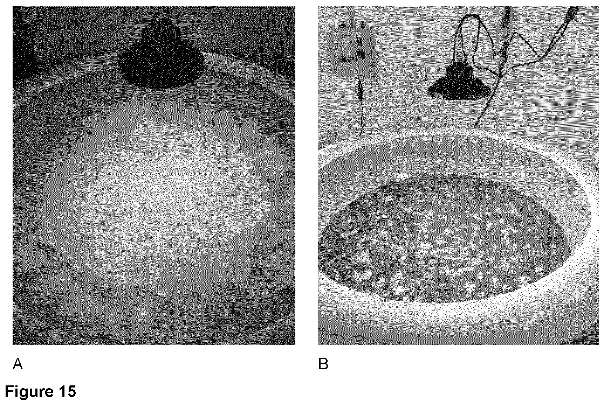

Growth data of *A. platensis* with slurry-derived colloid fraction in 1000L FBRs (Figure 15) confirmed a remarkable capacity to decrease the total amount of nitrogen (4.98 times less than non-cultivated slurry) and ammonia (4.25 times less), total phosphorus (6.93 times less), COD (7.77 times less) and potassium (1.58 times less) after the amendment of the residues by EMRA process (Table 8). Such conversions are related to several critical parameters, optimized in serial trials in the proof-of-concept facility (data no shown). Therefore, media oxygenation shall be permanently guarantee by agitation (stirring) and aeration (bubbling) of the open pond. Media oxygenation is even more critical than temperature or pH control. Such oxygenation triggers a greater bioremediation of the slurry if compared to the decrease in the control parameters in the laboratory cultures without such continuous oxygenation. As a general conclusion, EMRA-processed slurry derived *A. platensis* culture in 1000L FBRs (open ponds) with light intensity 3000-4000 lux, 27 °C, photoperiod 12 on / 12 off and constant oxygenation with bubbling has obtained a better performance in the conversion of nitrogen, phosphorus and potassium into protein, phospholipids, and critical biomolecules for subsequent industrial use in our proof-of-concept facility.

**Table 8. Physicochemical parameters comparison after 11 days of culture of *A. platensis* between original liquid slurry medium (dilution 1:1 v/v) and EMRA-processed slurry. The difference (slurry - amendment) is calculated to check the decrease factor (+) or increase (-) of the parameter originally measured. The fraction (slurry/amendment) is calculated to check the proportion of such decrease (+) or increase (-) in the original slurry after amendment.**

|  | Untreated slurry Dilution 1:1 V/V | EMRA-processed Dilution 1:1 V/V | Slurry - Amendment | Slurry/ Amendment |
|---|---|---|---|---|
| Conductivity 25°C ($\mu$s/cm) | 5.464 | 8.538 | -3.074 | -0,64 |
| N-Ammonia (N) (mg/L) | 287 | 67,6 | 219 | 4,25 |
| Total Nitrogen (N) (mg/L) | 329 | 66 | 263 | 4,98 |
| Total Phosphorus (P) (mg/L) | 19,6 | 2,83 | 17 | 6,93 |
| COD ($O_2$) (mg/L) | 2.068 | 266 | 1.802 | 7,77 |
| Potassium (K) (mg/L) | 464 | 293 | 171 | 1,58 |

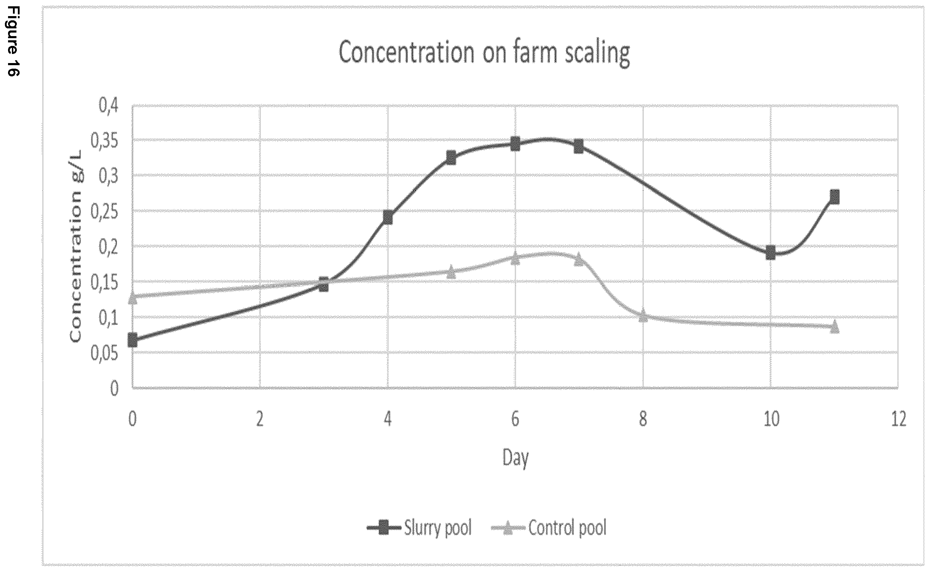

According to the optimized experimental trials results (data no shown) with Spirulina-specific high-balanced media, cultures should be considered successful when they reached nominal concentrations of $2.67 \pm 0.42$ g $L^{-1}$. EMRA-processed slurry derived *A. platensis* culture yields in open 1,000 L FBR (circular ponds) were 0.35 g $L^{-1}$ (Table 9 and Figure 16). It should be noted that the reference values were obtained in the low-volume scaling steps in Zaragoza, after the adaptation of the growth of the microalgae and the definition of its optimal culture conditions. In short, pH control is essential and pH variations must be avoided to a greater extent if optimal growth is to be achieved. Regarding light, it is necessary to avoid shadowing at very high densities and photoinhibition when direct light is administered, and to establish the optimal growing temperature. Optimizing pH and light control and with the optimal nutrient-rich low-burden microorganism colloid media culture in a longer experiment (up to 40 days), a higher viability and growing of *A. platensis* is achieved (validation on going). It was estimated that maximum production values per liter for *Arthrospira platensis paracas* should take about 26.7-47.6 days on average to reach following the tested conditions. We confirmed an *Arthrospira platensis paracas* yield of 0.35 g $L^{-1}$ from the 11-day after an injection of 10%-volume Zarrouk-like cultured *Arthrospira* inoculum in the best-available-technology FBR (open pond) in a hostile environment, which consisted on; (a) an original highly contaminated drinking water, and (b) abnormally high temperatures for more than 58 days suffered in summer-autumn in Spain during proof-of-concept serial trials. Several longer-period experimental trials are being studying for large-scale culture optimization by applying EMRA process.

**Table 9. Comparison of *A. platensis* growth between synthetic sea salt (Zarrouk-similar media) (control) and EMRA-processed slurry (derived from a dilution 1:1 v/v) in 1000L FBR (open ponds). The difference (slurry - control) is calculated to check the decrease factor (+) or increase (-) of the parameter originally measured. The fraction (slurry/control) is calculated to check the proportion of this decrease (+) or increase (-) in the original growth control in drinking water of Mediana de Aragón.**

| Control | | EMRA-processed slurry | | Slurry-Control | Slurry / control |
|---|---|---|---|---|---|
| Day | Concentration (g/L) | Day | Concentration (g/L) | | |
| 0 | 0,13 | 0 | 0,068 | 0,06 | 1,91 |
| - | - | 3 | 0,148 | | |
| - | - | 4 | 0,241 | | |
| 5 | 0,165 | 5 | 0,325 | -0,16 | -0,51 |
| 6 | 0,185 | 6 | 0,3455 | -0,16 | -0,54 |
| 7 | 0,1825 | 7 | 0,3425 | -0,16 | -0,53 |
| 8 | 0,1035 | - | - | | |

(continued)

| Control | | EMRA-processed slurry | | Slurry-Control | Slurry / control |
|---|---|---|---|---|---|
| Day | Concentration (g/L) | Day | Concentration (g/L) | | |
| - | - | 10 | 0,1915 | | |
| 11 | 0,0875 | 11 | 0,27 | -0,18 | -0,32 |

Within this framework, the applications of the EMRA system based on the cultivation of *Arthrospira platensis paracas* for the treatment of pig slurry reduce the environmental impact caused by this effluent; (a) uses the biomass obtained to generate a safe by-product for animal feed under ideal conditions (processed rich-carbon solid inactivated microalgae culture fraction); (b) produces high value-added NPK:C fertilizers in the same area and without odors (34.8% more production with the same water) (processed rich-nutrients biologically absorbable nitrogen-rich phosphorus-rich potassium-rich liquid microalgae culture fraction); (c) promotes a strong absorption of environmental $CO_2$; and (d) produces a consistent production of fatty acids, proteins and polysaccharides, sustainable biomolecules of an industrializable and high-value nature, among others.(84)

## EXAMPLE 2. EMRA-PROCESSED RICH-NUTRIENTS BIOLOGICALLY ABSORBABLE NITROGEN-RICH PHOSPHORUS-RICH POTASSIUM-RICH LIQUID MICROALGAE-DERIVED CULTURE FRACTION USED AS BIOFERTILIZER

The main objective of this task was to carry out a basic safety test and usability and efficacy tests as an agricultural biofertilizer of microalgal crop by-products and to study the characterization of the biofertilizers obtained.

## 2.1. Experimental design

An experimental trial was carried out to evaluate the bioremedial capacity of EMRA-processed rich-nutrients biologically absorbable nitrogen-rich phosphorus-rich potassium-rich liquid *A. platensis* -derived culture fraction obtained in 1000L FBR (open ponds) (example 1). In the present study, 5 L of culture was processed by as described in summary of the invention. Three experimental groups of cotton-sown *Cicer arietinum* seeds were tested in this trial: (a) seeds irrigated with drinking water from Mediana de Aragón (Zaragoza) (control); (b) seeds irrigated with synthetic sea salt-cultured *A. platensis* media; (c) and, finally, seeds irrigated with the EMRA-derived slurry-based amendment generated in the invention. After 18 days of cultivation, sprouted seeds, non-germinated seeds and developed seedlings were collected, their chlorophyll content was estimated, and a nutritional analysis was performed.

## 2.2. Materials and methods

### 2.2.1. Materials

- Drinking water from Mediana de Aragón (Zaragoza).
- Cotton.
- Slurry amendment obtained thanks to the bioremediation carried out by *A. platensis* at IFE-22-046 Validation of the technology and study of semi-industrial scale-up.
- Bottles for 1L centrifuge.
- Seeds lot no. 303411.
- Germinators.
- Nitrile gloves.
- Salinity™ Medium (Sal Aquavitro Salinity, SALAQUA16, Acuario Plantado SL or similar).
- Plastic wrap.
- Pincers.
- Disposable serological pipettes.
- Pipetus.
- Sprayer.
- 50 mL tubes.
- Pool control solution.

### 2.2.2. Equipment

- Balance.
- Thermal stability chamber.
- Centrifuge.
- TL-D 90 Graphica 36W/965 d lamps.
- Branson SLPe 150 Sonicator.

### 2.3. Methods

#### 2.3.1. Preparation of Crop Irrigation Solutions

Once the bioremediation stage was completed, it was necessary to process the culture together with the biomass of *A. platensis*. For this process, 5 L were taken from the control pools and diluted EMRA-treated slurry and sonicated at an amplitude of 50% for 30 minutes with a Branson SLPe 150 sonicator. Subsequently, the liquid fraction was separated from the cellular debris and other solids by centrifugation at 10,000 r.p.m. for 30 minutes at 20 °C to obtain a processed rich-nutrients biologically absorbable nitrogen-rich phosphorus-rich potassium-rich liquid microalgae culture fraction (from now onwards, also called "amendment"). Finally, obtained supernatants were stored at 4 °C until use.

#### 2.3.2. *Cicer arietinum* crop

##### 2.3.2.1. Sowing

*Cicer arietinum* seeds were counted and weighed for distribution in germinators according to the following conditions, with 2 replicates for conditions 1, 2 and 3 (Figure 15.)

- "White" condition: This group was made up of seeds from the same batch as those used in the rest of the conditions, but without any type of treatment. This condition was called "White" because it referred to a control sample that did not contain the substance or analyte of interest and that was used as a reference to establish a baseline for measuring the parameters to be determined in the other conditions.

- Condition 1 (Negative control): Seeds were irrigated every day with drinking water from Mediana de Aragón (more information in section 1.2.6.). This condition served as a negative control to compare the results of this treatment with those obtained in the seeds that received the amendment, and to rule out that the observed effects are simply due to the addition of water. It provides a reference of the natural variability of the system and ensures that any observed effects are due to the variable being measured, in this case, the application of the amendment.

- Condition 2 (Control pool): Irrigation was carried out with the solution from the control pool, which contained the *A. platensis* specific-media culture liquid fraction, previously obtained during the 1000L scaling up process (example 1). Once the solution was obtained, it was processed as described in section 2.3.1. This culture achieved a microalgae growth results of $2.67 \pm 0.42$ g L$^{-1}$ in validation experiments. AS previously discussed, these results reached and exceeded the best yields observed in the reviewed literature, namely 4.055 mg L$^{-1}$ to 2.3 g L$^{-1}$.(34, 80, 81, 82, 83) This control pool group served as a reference to determine whether the changes observed in the experimental group (slurry pool) are really the result of the variable being investigated or of other uncontrolled factors.

- Condition 3 (EMRA-derived slurry-based amendment): Seed irrigation was made with the amendment (EMRA-derived slurry-based processed rich-nutrients biologically absorbable nitrogen-rich phosphorus-rich potassium-rich liquid microalgae culture fraction) generated during the 1000L scaling up process (example 1). Subsequently, this amendment was processed as indicated in section 2.3.1.

The sowing, germination and plant growing was carried out in a thermal stability chamber at a temperature of 25 °C, 75% relative humidity, a light intensity of 10000 lux and a photoperiod of 12/12 hours (light/dark).

##### 2.3.2.2. Germination

Experimental groups and replica were followed daily and watered each day by adding 2 mL of solution according to condition per well. Detecting that this amount was not enough to keep the cottons completely moist, irrigation was combined with a spray of dechlorinated water to ensure moisture during the germination phase. Spraying with non-chlorinated water was carried out first thing in the morning and watering six hours later, around noon, repeating these times whenever possible.

### 2.3.2.3. Collecting and Preparing Samples for Analysis

For collection, each replica was divided into 3 groups: (a) non-germinated seeds, (b) germinated seeds, and (c) developed seedlings. Each of the new groups was then weighed, along with the "target" and placed in 50 mL tubes for further analysis.

### 2.3.3. Analysis of *Cicer arietinum*

### 2.3.3.1. Analysis of chlorophyll content in plant material

A sample of plant material ($\approx$0.5 g) was weighed on an analytical balance and transferred to a ceramic mortar. Ten milliliters (10 mL) of 90/10 v/v acetone/water mixture were added as a solvent extractor for the pigments. It was crushed and the resulting residue was transferred to a 50 mL conical bottom tube with the help of another 20 mL of 90/10 v/v acetone/water mixture. The extracts thus obtained were left to rest in the dark at 4 °C for 24 h. After this period, the extracts were allowed to reach room temperature and centrifuged at 7000 xg for 15 minutes. The liquid supernatant was filtered through a filter and collected in a 25 mL volumetric flask and flushed to volume. Finally, the optical density of the liquid extract was measured at 665, 645 and 630 nm, verifying that there was no turbidity or suspended particles. The solvent itself was used as a target.

### 2.3.3.2. Nutritional analysis

A standard nutritional analysis was carried out of each of the replicates of each condition, in which the ungerminated and germinated seeds and developed seedlings were grouped. In this analysis, moisture, proteins, carbohydrates, fats, ashes, sugars, saturated fatty acids, salts, and nitrogen were measured.

### 2.3. Results

### 2.3.1. *Cicer arietinum* crop

### 2.3.1.1. Sowing

Six (6) germinators were available, 2 for each condition and 1 per replicate, to carry out the sowing of *Cicer arietinum* seeds. Each replica consisted of 50 wells and a total of 5 seeds were sown in each of them.

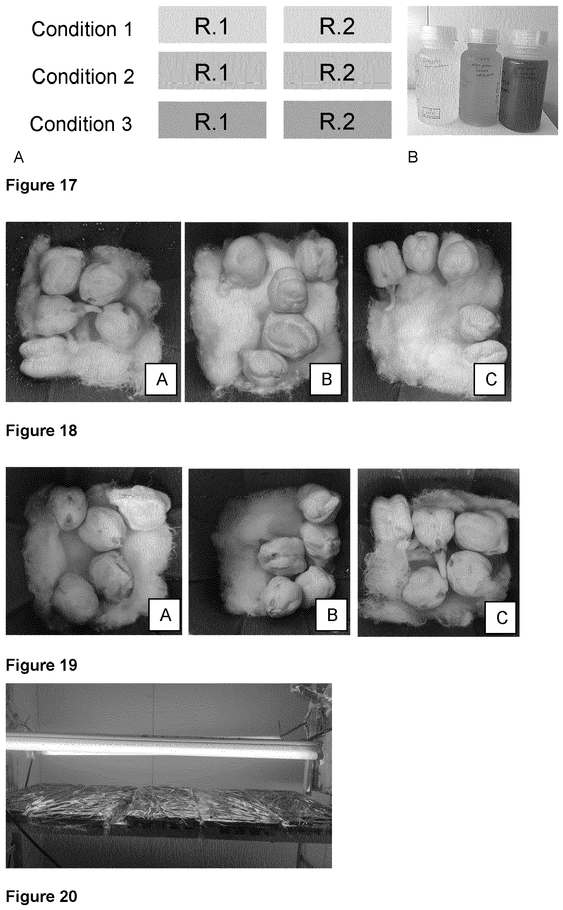

A specific identification was established for each of the conditions to differentiate the seedbeds, with condition 1 being A, condition 2 = B and condition 3 = C (Figure 17A.) Irrigation solutions used for each condition are shown in Figure 17B.

First, a layer of transparent paper was placed at the bottom of the seedbed to prevent water lost during irrigation, also favoring that the water that came out of the lower part could be available again by capillarity. Cotton was then placed in each of the wells to serve as a water reservoir for the crop. Before sowing, 250 seeds were counted, weighed, and then 5 seeds were sown per well, for a total of 50 per replicate. The following results were obtained by condition and replication:

Condition Weight 1: Replica 1 (164.92 g), Replica 2 (159.99 g)
Condition Weight 2: Replica 1 (164.25 g), Replica 2 (161.92 g)
Condition Weight 3: Replica 1 (162.41 g), Replica 2 (160.50 g)

The conditions used for cultivation were (Table 10).

**Table 10. Germination conditions for *Cicer arietinum*.**

| Conditions | Parameter |
|---|---|
| Temperature | 25 °C |

(continued)

| Conditions | Parameter |
|---|---|
| Humidity | 75% |
| Light Intensity | 10.000 lux |
| Photoperiod | 12/12 h |

### 2.3.1.2 Germination

On the first day of cultivation (D1) it was observed that the cottons were not completely wet after irrigation, so it was decided to combine irrigation with a spray of dechlorinated water, to preserve a certain degree of moisture during germination, an important factor in this phase. Spraying with non-chlorinated water was carried out first thing in the morning and watering six hours later, around noon, repeating these times whenever possible.

Already on the second day (D2) of the trial, changes in germination began to be seen, with condition 1 having the highest number of germinated seeds, followed by condition 3 and finally condition 2 (Figure 18.)

On the fourth day (D4), it was observed that the cottons were not very wet, therefore, it was decided to spray first thing in the morning and two hours after irrigation, to maintain humidity during the night. On the other hand, changes in the evolution of the seeds were again perceived. Seeds in conditions 1 and 3 began to germinate, showing a change to a greener coloration, indicating the appearance of another new germination stage: the seedling (Figure 19.)

Next, it was decided to cover the trays with cling paper (Figure 20), which let UV light to pass, to concentrate the moisture inside each of the seedbeds, making small holes to prevent overcondensation.

On the fifth day (D5), an incipient growth of fungi was observed, typical of the new humidity conditions, specifically in two wells of each of the replicates of condition 2.

The next day, D6, it was observed that the fungi were expanding along replica 2 of condition 2. On the other hand, small seedlings began to develop, and germination continued to occur exclusively in condition 1 and condition 3. From D5 onwards, germination process, fungi development and crop evolution were observed in all conditions to the end of study.

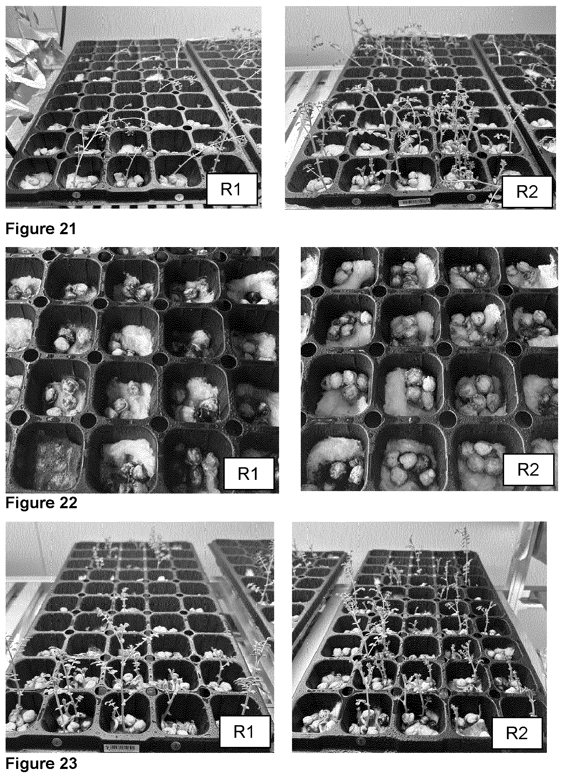

On day 15 (D15), the last day of photographic record, vegetative seedlings were continuously increasing in size in condition 1 (Figure 21) and 3 (Figure 23). In condition 3 continued in the seed stage with more presence of fungi (Figure 22.)

### 2.3.1.3. Collecting and preparing samples for analysis

Finally, on the eighteenth day (D18) of the trial, the seeds were collected for nutritional analysis. Each replica was divided into 3 groups: germinated seeds, non-germinated seeds, and developed seedlings. They were weighed (Table 11) and placed in 50 mL tubes.

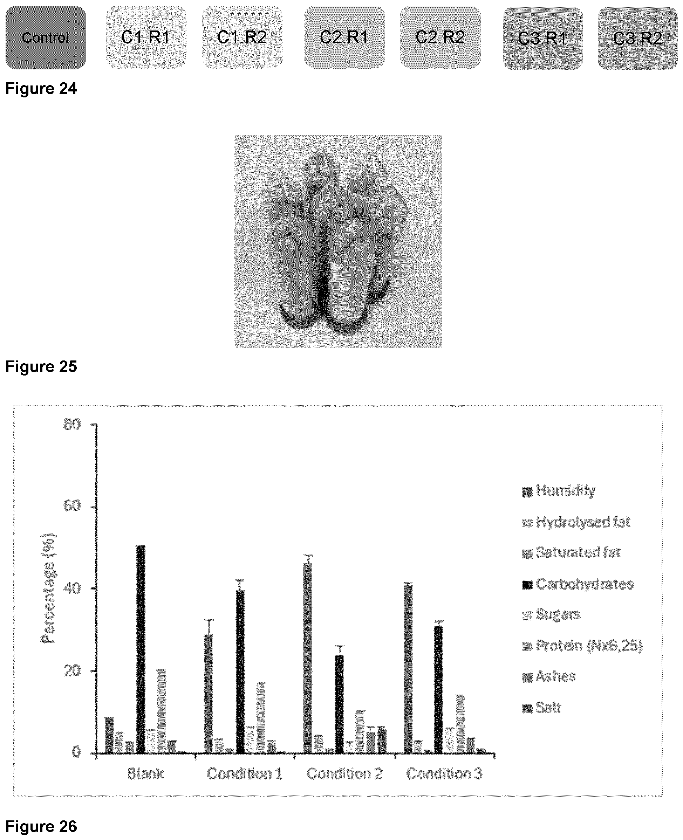

During the collection of the samples, it was observed that the seedlings that were in wells with fungi had a considerably lower root development than the rest. At the end of the test, 7 groups were generated (Figure 24), the "white" group with seeds from the same uncultivated batch (252,6 g, Figure 25), and the other 3 test conditions with their corresponding replicates.

Weights in grams by condition, replication, and group (ungerminated, germinated, seedling, and leaf mass) are presented below (Table 11) In the case of condition 2 (control), seedlings irrigated with liquid fraction of culture derived from *A. platensis* in synthetic sea salt, the samples were not divided into groups since all the seeds were not germinated, so the weights were not considered for comparison as no germination had been obtained. Presumably it is due to the high concentration of salts in the medium, which makes it more difficult for the seed to germinate. In any case, the test was validated as expected with this negative control.

A 34.8% [1-(36/55.2)] increase in weight of *Cicer arietinum* seeds was observed after cropping with EMRA-derived slurry-based processed rich-nutrients biologically absorbable nitrogen-rich phosphorus-rich potassium-rich liquid microalgae culture fraction.

Table 11. *Cicer arietinum* seeds weight under different conditions after 18 days (D18) of cultivation. Condition 1 (negative), seedlings irrigated with drinking water from Mediana de Aragón. Condition 2 (control), seedlings irrigated with liquid fraction of specific-media *A. platensis* culture (negative data not shown). Condition 3 (slurry), seedlings irrigated with EMRA-derived slurry-based processed rich-nutrients biologically absorbable nitrogen-rich phosphorus-rich potassium-rich liquid microalgae culture fraction.

| Condition | Replica | No germination (g) | Germinated (g) | Seedlings (g) | Leaf mass (g) | Pre-cultivation (g) | Post-cultivation (g) | Difference (g) |
|---|---|---|---|---|---|---|---|---|
| 1 | 1 | 93,8 (41,9%) | 64,5 (30,8%) | 49,1 (23,5%) | 1,5 (0,7%) | 164,9 | 208,9 | 44 |
|  | 2 | 77,4 (41,1%) | 50,3 (26,7%) | 58,5 (31,1%) | 1,9 (1,0%) | 160 | 188,1 | 28,1 |
|  | Media | 85,6 (43,1%) | 57,4 (28,9%) | 53,8 (27,1%) | 1,7 (0,9%) | 162,5 | 198,5 | 36 |
| 3 | 1 | 97,5 (42,9%) | 62,8 (27,6%) | 65,7 (28,9%) | 1,4 (0,6%) | 162,4 | 227,4 | 65 |
|  | 2 | 89,69 (43,5%) | 56,2 (27,3%) | 58,3 (28,3%) | 1,8 (0,9%) | 160,5 | 206 | 45,5 |
|  | Media | 93,6 (43,2%) | 59,5 (27,5%) | 62,0 (28,6%) | 1,6 (0,7%) | 161,5 | 216,7 | 55,2 |

### 2.3.2. Cultivation yield of *Cicer arietinum*

#### 2.3.2.1. Nutritional analysis

Once the physicochemical analysis of the samples was carried out, the following results were obtained as a percentage per 100 g of sample (Table 12). As shown in Table 12 and in Figure 26, the seeds of the "white" are the ones that contain the most saturated fats (2.5%), total carbohydrates (50.7%) and proteins (20.3%). Comparing the 3 experimental conditions, condition 1 (drinking water) is the one with the most total carbohydrates (39.6%), sugars (6.42%), and proteins (16.5%). As for the rest of the physicochemical values in greater quantity, they are found in condition 2 (specific media), with more moisture (46.2%), saturated fats (0.94%), ash (5.2%) and salt (5.7%). Condition 3 (EMRA) is the one that presents the intermediate values between 1 and 2. Finally, the saturated fat content was similar in the three test conditions.
It is not possible to observe significant differences in the content of the nutrients analyzed in the growth stage of the seedlings in each condition. It is important to note that both the seedlings of condition 1 and 3 presented a very similar protein content that increased with respect to the white one, considering the moisture content. The decrease in the amount of carbohydrates and fats in condition 3 may be indicative of plant development, since the rapid consumption of cotyledon nutrients may be accelerated by the presence of nutrients, mainly amino acids, in the environment.(93) In condition 2, where none of the seeds germinated, higher salt and ash values were observed compared to the white and conditions 1 and 3, which prevented the germination of the seeds.(94) In addition, taking into account humidity, the values of carbohydrates, proteins and lipids were lower.

Table 12. Nutritional values of *Cicer arietinum* from different culture media. Condition 1 (negative), seedlings irrigated with network water from Mediana de Aragón. Condition 2 (control), seedlings irrigated with liquid fraction of specific-media *A. platensis* culture (negative data not shown). Condition 3 (slurry), seedlings irrigated with EMRA-derived slurry-based processed rich-nutrients biologically absorbable nitrogen-rich phosphorus-rich potassium-rich liquid microalgae culture fraction.

| Condition | Réplica | Humidity (%) | Fats with hydrolysis (%) | Saturated fats (%) | Total carbohydrates (%) | Azúcares (%) | Protein (Nx6,25) (%) | Ashes (%) | Salt (%) |
|---|---|---|---|---|---|---|---|---|---|
| White | 1 | 8,6 | 5,1 | 2,5 | 50,7 | 5,6 | 20,3 | 2,8 | 0,2 |
| | 1 | 25,9 | 3,3 | 0,8 | 42,1 | 6,5 | 17,1 | 2,8 | 0,2 |
| 1 | 2 | 32,3 | 2,5 | 0,9 | 37,2 | 6,4 | 15,9 | 2,4 | 0,4 |
| | Media | 29,1 | 2,9 | 0,8 | 39,7 | 6,4 | 16,5 | 2,6 | 0,3 |
| | 1 | 48,3 | 4,1 | 0,9 | 22 | 1,6 | 9,9 | 4,2 | 6,3 |
| 2 | 2 | 44,1 | 4,1 | 1 | 26,1 | 2,5 | 10,3 | 6,3 | 5,1 |
| | Media | 46,2 | 4,1 | 0,9 | 24,1 | 2,1 | 10,1 | 5,2 | 5,7 |
| | 1 | 40,2 | 2,5 | 0,7 | 32,1 | 6 | 14,1 | 3,4 | 0,5 |
| 3 | 2 | 41,6 | 2,8 | 0,7 | 30 | 5,9 | 13,9 | 3,5 | 1,1 |
| | Media | 40,9 | 2,7 | 0,7 | 31,1 | 5,9 | 14 | 3,5 | 0,8 |

Taken together our results, authors suggest that the bioremediation of *Cicer arietinum* with EMRA-derived slurry-based amendment increases seed production (34.8% more by weight under the same conditions) while favoring the development of the seedling with lower moisture and water requirements, accelerating its growth while keeping the nutritional contents intact.

### 2.3.2.2. Analysis of chlorophyll content in plant material

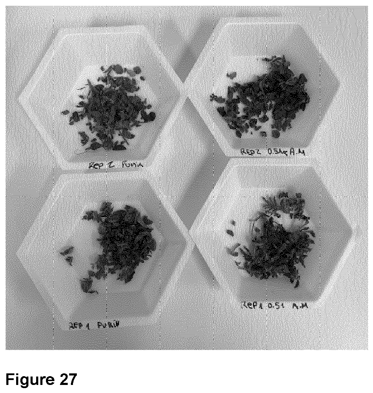

The first part for the calculation of the chlorophyll content was the calculation of the leaf biomass by condition and replication. In this case, this calculation was only carried out under conditions 1 and 3 since condition 2 did not germinate. The leaves of each of the seedlings were removed with tweezers and scissors and preserved in 50 mL tubes. Subsequently, the number of leaves intended for chlorophyll analysis (0.5 g) was taken, and the leftovers were added to their corresponding sample for nutritional analysis (Figure 27.)

Following the procedure described in section 2.3.3.1. for the determination of chlorophyll in plant material, 4 samples corresponding to plant material obtained from *Cicer arietinum* seeds germinated under conditions 1 (Replication 1 and 2) and 3 (Replication 1 and 2) were weighed (Table 13).

Table 13. Weights of plant material obtained from *Cicer arietinum seeds* germinated under conditions 1 and 3. Condition 1 (negative), seedlings irrigated with network water from Mediana de Aragón. Condition 3 (slurry), seedlings irrigated with EMRA-derived slurry-based processed rich-nutrients biologically absorbable nitrogen-rich phosphorus-rich potassium-rich liquid microalgae culture fraction.

| | Mass Condition 1 (g) | Mass Condition 3 (g) |
|---|---|---|
| Replica 1 | 0,4645 | 0,4512 |
| Replica 2 | 0,4258 | 0,4382 |

Chlorophyll content was extracted according to the procedure described in section 2.3.3.1. Finally, absorbances of an aliquot of the processed extracts were measured at different wavelengths 630, 645 and 665 nm (Table 14).

**Table 14. Optical density of chlorophyll extracts obtained under conditions 1 and 3 of replicates 1 and 2. Condition 1 (negative), seedlings irrigated with network water from Mediana de Aragón. Condition 3 (slurry), seedlings irrigated with EMRA-derived slurry-based processed rich-nutrients biologically absorbable nitrogen-rich phosphorus-rich potassium-rich liquid microalgae culture fraction.**

|  | Absorbance 630 nm | Absorbance 645 nm | Absorbance 665 nm |
|---|---|---|---|
| R1.C1.REP1 | 0,570 | 1,090 | 2,349 |
| R1.C1.REP2 | 0,572 | 1,088 | 2,352 |
| Average value | **0.571** | **1,089** | **2,350** |
| R2.C1.REP1 | 0,659 | 1,252 | 2,575 |
| R2.C1.REP2 | 0,663 | 1,255 | 2,582 |
| Average value | **0,661** | **1,253** | **2,580** |
| R1.C3.REP1 | 0,738 | 1,351 | 2,686 |
| R1.C3.REP2 | 0,741 | 1,360 | 2,690 |
| Average value | **0,740** | **1,350** | **2,688** |
| R2.C3.REP1 | 0,527 | 0,977 | 2,215 |
| R2.C3.REP2 | 0,532 | 0,972 | 2,212 |
| Average value | **0,530** | **0,975** | **2,214** |

NOTE: A: Replica; C: condition; REP: repetition.

At this point, the following formulas were applied for the calculation of chlorophyll content A, B and C proposed by Parsons and Strickland (1965) (Formulas 1-3, Table 15):

$$C_a \ (mg/L) = 11,6 \ Abs_{665} - 1,31 \ Abs_{645} - 0,14 \ Abs_{630}$$

**Formula 1.** Chlorophyll A concentration.

$$C_b \ (mg/L) = 20,7 \ Abs_{645} - 4,34 \ Abs_{665} - 4,42 \ Abs_{630}$$

**Formula 2.** Chlorophyll B concentration.

$$C_c \ (mg/L) = 55,0 \ Abs_{630} - 4,64 \ Abs_{665} - 16,3 \ Abs_{645}$$

**Formula 3.** Chlorophyll C concentration.

**Table 15. Calculation of chlorophyll A, B and C concentration according to Parsons and Strickland formulas.**

| Average | $C_a$ (mg/L) | $C_b$ (mg/L) | $C_c$ (mg/L) |
|---|---|---|---|
| C1.R1. | 25,76 | 9,82 | 2,75 |
| C1.R2. | 28,18 | 11,83 | 3,96 |
| C3.R1. | 29,30 | 13,12 | 6,10 |
| C3.R2. | 24,33 | 8,22 | 2,97 |

NOTE: A: Replica; C: Condition.

Finally, the percentage of each type of chlorophyll in each extract was calculated according to the following formulas:

$$\%Chlorophyll \ A = [[(11,6 \ Abs_{665} - 1,31 \ Abs_{645} - 0,14 \ Abs_{630}) \times 0,025 \ L]/m] \times 100$$

**Formula 4.** Percentage of chlorophyll A.

$$\%\text{Chlorophyll B} = [[(20{,}7\ Abs_{645} - 4{,}34\ Abs_{665} - 4{,}42\ Abs_{630}) \times 0{,}025\ L]/m] \times 100$$

**Formula 5.** Percentage of chlorophyll B.

$$\%\text{Chlorophyll C} = [[(55{,}0\ Abs_{630} - 4{,}64\ Abs_{665} - 16{,}3\ Abs_{645}) \times 0{,}025\ L]/m] \times 100$$

**Formula 6.** Percentage of chlorophyll C.

Where m: the mass of plant material obtained from *Cicer arietinum* seeds germinated under conditions 1 and 3 of replicates 1 and 2. The results are presented in Table 16.

**Table 16. Calculation of Chlorophyll A, B and C Percentage according to Formulas 4-6.**

| Average | % Chlorophyll A | % Chlorophyll B | % Chlorophyll C |
|---------|-----------------|-----------------|-----------------|
| C.1.R1. | 0,14 | 0,053 | 0,015 |
| C.1.R2. | 0,16 | 0,069 | 0,023 |
| C.3.R1. | 0,16 | 0,073 | 0,034 |
| C.3.R2. | 0,14 | 0,047 | 0,017 |
| NOTA: R: replica; C: condition. | | | |

No significant differences were observed in the content of chlorophyll A, B or C in the growth stage of the seedlings measured for both conditions and replicates.

### 2.4. Resume of validation results of bioremediation of *Cicer arietinum* seeds with EMRA-derived slurry-based amendment.

Swine wastewater (slurry) steady increase has become a serious environmental concern. High levels of nutrients and toxic pollutants in slurry have a significant impact on the ecosystem and public health.(3) Regulators set up a complex and complete administrative machinery to try to control or maintain at acceptable levels these problems associated with industrial activity.(4) Therefore, it is necessary to work on strategies for the use or elimination of these risks (odors, phreatic pollutants) to promote a more efficient, profitable, sustainable, respectful and healthy production for (a) the animals, (b) the populations surrounding the farms, and (c) the environment. The economic value of fattening swine slurry has been quantified between 5 and 7 euros/$m^3$ in its equivalent in prices of the NPK mineral fertilizers it contains.(6) While the reference of the cost of management and transport of these same slurry is estimated at 2.92 euros/$m^3$ on average (data from GET, Central Region of Zaragoza, Median of Aragon, Spain). It is estimated that it is possible to replace mineral fertilizer with swine slurry under conditions of minimum tillage, totally in barley and partially in corn, being also economically profitable in rainfed areas with a continental Mediterranean climate.(2)

Microalgae, with a short shelf life and a high growth rate and $CO_2$ utilization efficiency, have proven to be an optimal ecological and renewable alternative for producing biomass using nutrients from wastewater.(81) Microalgae can grow in real-world conditions, outdoors, in wastewater from swine farms as the sole source of nutrients, having been treated by anaerobic digestion, different A/O processes, microbial fuel cells and the cultivation of various microalgae.(3, 14, 16) Examples of this management are the outdoor reactor culture of *Scenedesmus* sp.(71), for the production of biofuels, and *Chloroflexus aurantiacus,* a very robust extremophile organism.(95) Green microorganisms find the nitrogen and phosphorus necessary for their development by using slurry as a growing medium.

*Arthrospira* has growth regulators and antioxidants capable of increasing the tolerance of plants by helping them synthesize certain components. *Arthrospira* composition is very varied; it is one of the best vegetable sources of protein of high biological value and good digestibility; it contains between 65-70% protein, it is rich in chlorophyll, a pigment with great detoxifying power, in vitamins of group B (especially B12), C, D and E, it contains minerals such as potassium, selenium, copper, manganese, magnesium, zinc and phosphorus, digestive enzymes and 7% of their content are essential fatty acids.(48) However, it can also act as a sequestrant of cyanotoxins, heavy metals, pesticides or polycyclic aromatic hydrocarbons (PAHs), growing without excessive problems in natural conditions in polluted environments.(48) The special composition of these molecules causes them to aggregate into depot organelles or adhere to phospholipid membranes. Therefore, in the design devised by the researchers, ultrasonication technology was introduced into the process, to minimize the risk of high concentrations of contaminants being diverted to the liquid fraction of the culture (see Figure 1.)

Briefly, ultrasonication generates alternating high- and low-pressure waves in the exposed liquid. During the low-pressure cycle, ultrasonic waves create tiny vacuum bubbles in the liquid that collapse violently during a high-pressure cycle. This phenomenon is called cavitation. The implosion of the cavitation bubble causes strong hydrodynamic shear forces. These Shear forces can break down fibrous cellulosic material into fine particles and break down the walls of the cell structure. This releases more intracellular material, such as starch or sugar into the liquid, but does not break down depot or highly concentrated organelles.(96, 97) On top of that, the cell wall material is breaking down into tiny debris, allowing us to flocculate or separate.

Microalgae have a better C, N and P removal rate (91%, 91% and 73% respectively) than bacteria (74%, 80% and 55% respectively) in the treatment of slurry as runoff water.(98) This experiment explored the feasibility of this type of low-temperature treatments by comparing 10 elimination results between two membrane anaerobic photobioreactors at 10°C and 22°C operated at a residence time of less than one day. Finally, an effluent with concentrations within the discharge limit and removal efficiencies was reached for the reactor at 22°C C, N and P of 91%, 81% and 82%, respectively, and for the reactor at 10°C C, N and P of 87%, 77% and 98%, respectively.

EMRA-derived slurry-based amendment carried out in our test conditions increased the crop productivity of *Cicer arietinum* by 34.8% in the seed weight ratio and during the germination process, compared to a standardized control of the traditionally irrigated plant. These results suggest that bioremediation with EMRA-derived slurry-based amendment would favor the development of seedlings with lower moisture and water requirements, accelerating their growth while keeping the nutritional content without differences with the controls. In addition, the chlorophyll content of the bioremediated group remains unchanged with respect to the expected growth pattern (control group), so the seedling's capacity for respiration, growth and development remains intact, but from an accelerated and improved starting point. These results surpass those obtained in similar experiments with chickpeas, in which foliar spraying with the Spirufert biofertilizer at two points in the vegetative phase increased the yield of the chickpea crop; although there was no influence on the levels of N, P, K in the grains. In addition, the treatment modified the levels of phosphorus and magnesium in the soil.(99) The main difference of this work is that it requires the addition of two different biofertilizers [Azofert®, biofertilizer based on the *Rhizobium leguminosarum* strain, at a cell concentration of 2.8 x 1010 CFU) and Spirufert, aqueous suspension of Spirulina jelly (64%) and vinasse (36%)] to achieve an additive or synergistic effect that increased chickpea yield between 21-28% in a soil classified as lixic ferralic nitisol (rhodic alcylic eutric).(99) Nitisol is considered a fertile soil despite its low content of assimilable phosphorus and its low saturation in bases. They are deep, stable soils with favorable physical properties. We have achieved a 10% improvement in crop yield and weight (34.8%) in the absence of fertile soil by using EMRA-derived slurry-based amendment, which makes us very optimistic about its application in damaged or contaminated soils. In other growth models, extracts of *Cicer arietinum L.* and *Spirulina platensis* exerted positive effects on maize plants (*Zea mays*). (100) However, *Cicer arietinum L.* extracts appeared to be more efficient than *Spirulina platensis* (another strain of this microalgae) in improving maize nutrition and growth parameters in some respects, probably by virtue of its higher content of phytochemicals [hormones (indole-3-acetic acid, isopentenyladenosine, gibberellin), phenols, amino acids, reducing sugars] that can act as signaling molecules.(100) Perhaps this incremental effect of hormones and transmitters related to EMRA-derived slurry-based amendment is behind the higher yield in Cicer seeds observed in this work. Future work should confirm our hypothesis with validable data from fertile eutrophic soils.

## EXAMPLE 3. ABOUT EMRA ECO-PERFORMANCE AND CARBON CAPTURE

The use of macro and microalgae as raw material has been a revolution in recent years, due to their high growth rate and high yield per cultivation area,(81) although we have already seen in our results that the EMRA-derived slurry-based liquid fraction of culture derived from *Arthrospira platensis paracas* can exceed by 10% the yields obtained in similar or sister species.(99) In other words, using the same amount of water, the seed can take advantage of the carbon and nutrients derived from the EMRA amendment to produce 34.8% more agricultural product (same nutrients, same chlorophyl), thanks to the spirulina bioremediation. We fixed carbon and nitrogen from the slurry, and what was a residue can triple agricultural production with the same amount of water, in the model studied. In addition, thanks to their high photosynthetic rate and low biological demands, *Arthrospira* can transform solar energy into carbon storage products, and therefore mitigate $CO_2$ and greenhouse gas emissions (GGE).(40) At present, the technology required for its use entails a deficit cost/benefit ratio, limiting its mass application on an industrial scale. In this sense, there is still room to delve into the research of cutting-edge technological solutions based on phototropic algae, which will allow the elimination of current economic barriers.

A large part of the gaps to be evaluated have been fully or partially answered in this project: (a) adaptability of the chosen microalgae strains, both proprietary and those chosen for comparison, to the continental Mediterranean climate (dry, extreme in its cold-heat cycles); (b) the influence of the characterization of slurry adapted to European intensive production diets on the productivity of the adapted strains; and (c) the demonstration of an effective generation of reusable by-products for nutritional or phytoremediation applications.

**[0174]** * Note: Calculated and interpreted based on the difference and comparison with strains grown in mild or tropical climates, leaders in microalgae cultivation today. Stable biological systems, such as those found in Japan, allow microalgae to direct all their energies to growth and multiplication in a semi-continuous or near-continuous manner.

However, in more extreme climates, microalgae invest a lot of energy in surviving, allowing for more seasonal reproductive cycles (see reviewed citations).

EMRA-based inner cyclic *Arthrospira* production shall lead to a necessary overall reduction in the carbon footprint (the current revised calculations do not take into account consumption and generation by energy supplied outside the system.) of the farm, by applying right away the invention during the process of emptying inner slurry pools in swine pens (sanitary recommended max. storage of 20 days) and coupling the invention to the external common slurry pond. The systems coupling with regular inner slurry emptying avoids at least 100 days of open-air residues fermentation (legal open-air max. storage of slurry use to be 120 days).(10) Removing 100 days of external ponds GGE may avoid up to 83% of total GGE farm.

Constant incremental industrialization and the urgent need to avoid the use of non-renewable fossil fuels due to climate change has led to the finding of alternative viable renewable resources to meet the growing demand for energy with less carbon dioxide generation.(54) The modern global energy strategy is based on looking for green and cost-effective alternatives to fossil fuels. In the meantime, microalgae cultivation has the potential to reduce significantly slurry-derived animal farm greenhouse gas emissions, composed but not restricted to ammonia ($NH_3$), nitrous oxide ($N_2O$), methane ($CH_4$), carbon dioxide ($CO_2$) and hydrogen sulfide ($H_2S$). Microalgae have been identified as a promising and sustainable alternative for wastewater treatment (urban, agricultural, industrial) along with the production of valuable by-products. Research into these technologies is fully aligned with the strategy outlined in Spain's National Plan for Adaptation to Climate Change,(101) in which the sustainability of the food system and adaptation to climate change in rural areas are promoted, promoting, among others, the circular economy and the establishment of strategies that generate less climate impact and greater resilience.

The conversion of carbon and phosphorus from the slurry to microalgal fatty acids (phospholipids) allows its fixation and extraction from the fecal material. In addition, through the conventional respiratory effect of spirulina we can convert atmospheric carbon into linear chains of hydrocarbons (fats) more efficiently than with simple eutrophic carbonization of soil. The selected spirulina feeds on the nutrients in the slurry that they transform into biomass through photosynthesis, initiating a carbon capture cycle in which, in addition to nitrogen and phosphorus, they need 2 kg of $CO_2$ for each kg of biomass produced. Our proof-of-concept experimental data show that, at maximum theoretical yield of *Arthrospira platensis paracas,* spirulina production could range from 181.51 g day$^{-1}$ to 14,685.73 g day$^{-1}$, which brings us to 363.02 - 29,371.46 g day$^{-1}$ of environmental carbon removed, added to autotrophic consumption. Both processes combined achieved a better result of removing up to 9.1% of the inorganic carbon from the slurry, converting it into usable organic fatty acids such as palmitic, detected in EMRA-derived slurry-based amendment (data non shown). This performance is above what has been described so far by the literature consulted, which makes us very optimistic when it comes to deploying EMRA as an environmental solution to improve the sustainability of swine farms, not so much as a carbon sequester per se, but as an avoider of greenhouse gas emissions into the atmosphere from slurry (methane, nitrates, etc.)

## REFERENCES

1. Loyon L. Overview of Animal Manure Management for Beef, Pig, and Poultry Farms in France. Frontiers in Sustainable Food Systems. 2018;2.

2. Quilez D. Fertilización con purin en doble cultivo anual, en mínimo laboreo, y riego por aspersión. Informaciones Técnicas N° 223: Dirección General de Desarrollo Rural. Centro de Transferencia Agroalimentaria. Gobierno de Aragón.; 2010.

3. Cheng H-H, Narindri B, Chu H, Whang L-M. Recent advancement on biological technologies and strategies for resource recovery from swine wastewater. Bioresource Technology. 2020;303:122861.

4. Decreto 53/2019, por el que se regula la gestión de estiércoles y los procedimientos de acreditación y control, (2019).

5. Mestre Martinez MV. Ganaderia intensiva: el reto ambiental del sector porcino en España. The Conversation. 2021:2.

6. Ederra F. MODELO DE GESTION SOSTENIBLE DE PURIN PORCINO SOSTENIBLE DE PURIN PORCINO EN TAUSTE. In: Huesca EPS, editor. XXVII JORNADAS DE CIENCIA Y TECNOLOGIA; 12 de Mayo de 2016; Huesca2016.

7. Gómez Garrido M. Efectos ambientales de la valorización agronómica de purines de ganado porcino: dinámica del nitrógeno en el sistema suelo-agua-planta. 2014.

8. Salmerón M, Isla R, Cavero J, Delgado I. Viabilidad de las aplicaciones de purin en alfalfa de regadio en el Valle del Ebro. Agriculture: Revista agropecuaria y ganadera 2011(942):458-62.

9. Marin CE, Marin RG, editors. Tratamiento de purines de ganado porcino en España para minimizar la contaminación de suelos y su impacto ambiental. Congreso Internacional sobre Desertificación; 2009.

10. Ministerio de Agriculture PyAM. Jornada sobre Sistemas de gestión de deyecciones ganaderas. In: MAPA, editor. Jornada sobre Sistemas de gestión de deyecciones ganaderas; 26th Nov 2015: MAGRAMA; 2015.

11. Xavier Flotats, Henning L. Foged, August Bonmati, Karl M. Schelde, Jordi Palatsi, Albert Magri, et al. Inventory of manure processing activities in Europe. In: Comission E, editor. Protecting waters against pollution caused by nitrates from agricultural sources: European Comission; 2013.

12. Besel S. Biomasa: digestores anaerobios. Editorial Idea, Madrid, España. 2007.

13. Flotats Ripoll X. La gestión integral de purines2019 12/01/2023. Available from: https://www.3tres3.com/articulos/la-gestion-integral-de-purines 41865/.

14. Cheng DL, Ngo HH, Guo WS, Chang SW, Nguyen DD, Kumar SM. Microalgae biomass from swine wastewater and its conversion to bioenergy. Bioresource Technology. 2019;275:109-22.

15. López-Pacheco IY, Silva-Núñez A, Garcia-Perez JS, Carrillo-Nieves D, Salinas-Salazar C, Castillo-Zacarias C, et al. Phyco-remediation of swine wastewater as a sustainable model based on circular economy. Journal of Environmental Management. 2021;278:111534.

16. Nagarajan D, Kusmayadi A, Yen H-W, Dong C-D, Lee D-J, Chang J-S. Current advances in biological swine wastewater treatment using microalgae-based processes. Bioresource Technology. 2019;289:121718.

17. Varma VS, Parajuli R, Scott E, Canter T, Lim TT, Popp J, et al. Dairy and swine manure management - Challenges and perspectives for sustainable treatment technology. Science of The Total Environment. 2021;778:146319.

18. Zheng M, Dai J, Ji X, Li D, He Y, Wang M, et al. An integrated semi-continuous culture to treat original swine wastewater and fix carbon dioxide by an indigenous Chlorella vulgaris MBFJNU-1 in an outdoor photobioreactor. Bioresource Technology. 2021;340:125703.

19. Zheng M, Ji X, He Y, Li Z, Wang M, Chen B, et al. Simultaneous fixation of carbon dioxide and purification of undiluted swine slurry by culturing Chlorella vulgaris MBFJNU-1. Algal Research. 2020;47:101866.

20. Li X, Wu S, Yang C, Zeng G. Microalgal and duckweed based constructed wetlands for swine wastewater treatment: A review. Bioresource Technology. 2020;318:123858.

21. DiPietre D. La politica, una causa mas de la variabilidad de los precios y los costes. www.3tres3.com: 333 Corporate 1998, S.L.; 2013 [Available from: http://www.3tres3.com/situacion-mercado-porcino/la-politica-una-causa-mas-de-la-variabilidad-de-precios-y-costes 32848/

22. Kuhn T, Kokemohr L, Holm-Müller K. A life cycle assessment of liquid pig manure transport in line with EU regulations: A case study from Germany. Journal of Environmental Management. 2018;217:456-67.

23. Ruiz FJA. Integrated and Cost-Effective Solution to reduce the volume of Pig Slurry, minimize Pollutant Emissions and Process Energy Consumption. CORDIS: European Commission 30 September 2013. Contract No.: 277624.

24. Gesing F. The material politics of slurry: Mobilisations and transformations along the waste-fertiliser continuum. Political Geography. 2023;101:102832.

25. Galeano Ó. Discover all the benefits of the agronomic use of slurry. In: ROTECNA, editor. 2022.

26. Nolan T, Troy SM, Gilkinson S, Frost P, Xie S, Zhan X, et al. Economic analyses of pig manure treatment options in Ireland. Bioresource Technology. 2012;105:15-23.

27. Vallés Pérez M, Espada Domingo M, Magallón Botaya E. Coyuntura del sector porcino aragonés en 2022. Empleo generado, Normativa Medioambiental y Análisis DAFO [Internet]. 2022; Informaciones Técnicas(285):[20 p.]. Available from: https://www.aragon.es/documents/20127/77519864/IT 285 22.pdf/140504cf-323c-1435-295b-ee51efa8cc97?t=1668601063000.

28. Ninyerola M, Pons X, Roure JM. Atlas climático digital de la Peninsula Ibérica. 1 ed: Universitat Autònoma de Barcelona Departament de Biologia Animal, Biologia Vegetal i Ecologia (Unitat de Botànica); 2005 septiembre 2005.

29. Garcia J, Ortiz A, Álvarez E, Belohlav V, García-Galán MJ, Diez-Montero R, et al. Nutrient removal from agricultural run-off in demonstrative full scale tubular photobioreactors for microalgae growth. Ecological engineering. 2018;120:513-21.

30. Rushton J. The economics of animal health and production. London: Royal Veterinary College; 2008.

31. Lee JM. Biochemical Engineering. eBook Version 2.3 ed. http://imlee.net: Department of Chemical Engineering; 2009.

32. Dutta R. Fundamentals of biochemical engineering: Springer; 2008.

33. Kholssi R, Ramos PV, Marks EA, Montero O, Rad C. Biotechnological uses of microalgae: a review on the state of the art and challenges for the circular economy. Biocatalysis Agricultural Biotechnology. 2021 ;36: 1 02114.

34. Sánchez-Bayo A, Morales V, Rodriguez R, Vicente G, Bautista LF. Cultivation of microalgae and cyanobacteria: Effect of operating conditions on growth and biomass composition. Molecules. 2020;25(12):2834.

35. Sánchez-Borroto Y, Tobío-Pérez I, Romero-López TdJ, Díaz-Domínguez Y, Melo-Espinosa E, Piloto-Rodriguez R. Evaluación de las condiciones experimentales básicas para la producción de biomasa a partir de la microalga Chlorella vulgaris. Afinidad. 2019;76(585).

36. Gonzalez-Camejo J, Aparicio S, Jiménez-Benítez A, Pachés M, Ruano M, Borrás L, et al. Improving membrane photobioreactor performance by reducing light path: operating conditions and key performance indicators. Water Research. 2020-172-115518.

37. Ortiz Ruiz A. Microalgae-based wastewater treatment systems at demonstrative scale: gravity harvesting and thickening of biomass, and advanced design of bioreactors. 2021.

38. Iguácel Soteras F, Yagüe Carrasco MR, Orús Pueyo F, Quilez Sáez de Viteri D. Fertilización con purin en doble cultivo anual, en mínimo laboreo, y riego por aspersión. 2010.

39. Jiménez Veuthey M. Depuración de purines en fotobiorreactores abiertos. Universidad de Almeria. 2015.

40. Bolado Rodriguez S, Lorenzo Hernando A. Estudio de hidrólisis enzimática en biomasa microalgal procedente del tratamiento de aguas residuales con purines UNIVERSIDAD DE VALLADOLID; 2016.

41. Cárdenas Calvachi GL, Sánchez Ortiz IA. Nitrógeno en aguas residuales: origenes, efectos y mecanismos de remoción para preservar el ambiente y la salud pública. Universidad y Salud. 2013;15(1):72-88.

42. Likiliki C. Étude des procédés biologiques d'élimination d'azote appliqués à la phase liquide du lisier de pores après séparation en bâtiment: Universit6 Rennes 1; 2021.

43. Ortuño Cases C. Aplicación de ultrasonidos de potencia para la mejora de procesos de inactivación con fluidos supercriticos: Universitat Politècnica de València; 2014.

44. n.d. Sonicación para Lisis: Disrupción y extracción celular25th Nov 2023. Available from: https://www.hielscher.com/es/ultrasonic-lysis-cell-disruption-extraction.htm.

45. Taha A, Ahmed E, Ismaiel A, Ashokkumar M, Xu X, Pan S, et al. Ultrasonic emulsification: An overview on the preparation of different emulsifiers-stabilized emulsions. Trends in Food Science & Technology. 2020;105:363-77.

46. Sireesha T, Gowda NN, Kambhampati V. Ultrasonication in seafood processing and preservation: a comprehensive review. Applied Food Research. 2022;2(2):100208.

47. Sutariya S, Sunkesula V, Kumar R, Shah K. Emerging applications of ultrasonication and cavitation in dairy industry: a review. Cogent Food & Agriculture. 2018;4(1):1549187.

48. Grosshagauer S, Kraemer K, Somoza V. The True Value of Spirulina. J Agric Food Chem. 2020;68(14):4109-15.

49. Camacho-Morales S. Producción de spirulina: aislamiento, optimización de cultivo y secado. Fase: aislamiento y preservación Arthrospira (spirulina) nativa de Jalisco. 2016.

50. Lopez-Pacheco IY, Silva-Nunez A, Garcia-Perez JS, Carrillo-Nieves D, Salinas-Salazar C, Castillo-Zacarias C, et al. Phyco-remediation of swine wastewater as a sustainable model based on circular economy. J Environ Manage. 2021;278(Pt 2):111534.

51. Harvey M. HIGH-PRESSURE SLURRY ABLATION FOR IMPROVING SEPARATION OF COPPER/MOLYBDENUM AND FLOTATION OF GOLD TAILINGS. Spring 5-2023: Montana Tech; 2023.

52. Perlmutter B. Combination mechanical slurry conditioning and filtration2012 02/12/2023. Available from: https://www.digitalrefining.com/article/1000370/combination-mechanical-slurry-conditioning-and-filtration.

53. DiPietre D. La maximización del beneficio, el objetivo último de la producción porcina http://www.3tres3.com: 333 Corporate 1998, S.L.; 2013 [Available from: http://www.3tres3.com/situacion-mercado-porcino/el-objetivo-de-la-produccion-porcina-la-maximizacion-del-beneficio 33048/

54. Hussain F, Shah SZ, Ahmad H, Abubshait SA, Abubshait HA, Laref A, et al. Microalgae an ecofriendly and sustainable wastewater treatment option: Biomass application in biofuel and bio-fertilizer production. A review. Renewable and Sustainable Energy Reviews. 2021; 137: 11 0603.

55. Tanvir RU, Zhang J, Canter T, Chen D, Lu J, Hu Z. Harnessing solar energy using phototrophic microorganisms: A sustainable pathway to bioenergy, biomaterials, and environmental solutions. Renewable and Sustainable Energy Reviews. 2021;146:111181.

56. Chiu S-Y, Kao C-Y, Chen T-Y, Chang Y-B, Kuo C-M, Lin C-S. Cultivation of microalgal Chlorella for biomass and lipid production using wastewater as nutrient resource. Bioresource Technology. 2015;184:179-89.

57. Kholssi R, Ramos PV, Marks EAN, Montero O, Rad C. 2Biotechnological uses of microalgae: A review on the state of the art and challenges for the circular economy. Biocatalysis and Agricultural Biotechnology. 2021 ;36: 1 02114.

58. Blanco L. Análisis y caracterización de purines para la obtención de estruvita y biogás. Valencia: Universitat Politécnica de Valéncia. 2015.

59. Commission E. Regulation (EC) No 767/2009 of the European Parliament and of the Council of 13 July 2009 on the placing on the market and use of feed, amending European Parliament and Council Regulation (EC) No 1831/2003 and repealing Council Directive 79/373/EEC, Commission Directive 80/511/EEC, Council Directives 82/471/EEC, 83/228/EEC, 93/74/EEC, 93/113/EC and 96/25/EC and Commission Decision 2004/217/EC. Off J Eur Union. 2009;229:1-28.

60. Commission E. Commission Regulation (EU) No 68/2013 of 16 January 2013 on the Catalogue of feed materials. Off J Eur Union. 2013;29:1-64.

61. Lefort F, Calmin G, Crovadore J, Falquet J, Hurni JP, Osteras M, et al. Whole-Genome Shotgun Sequence of Arthrospira platensis Strain Paraca, a Cultivated and Edible Cyanobacterium. Genome Announc. 2014;2(4).

62. Cheng HH, Narindri B, Chu H, Whang LM. Recent advancement on biological technologies and strategies for resource recovery from swine wastewater. Bioresour Technol. 2020;303:122861.

63. Cheng DL, Ngo HH, Guo WS, Chang SW, Nguyen DD, Kumar SM. Microalgae biomass from swine wastewater and its conversion to bioenergy. Bioresour Technol. 2019;275:109-22.

64. Nagarajan D, Kusmayadi A, Yen HW, Dong CD, Lee DJ, Chang JS. Current advances in biological swine wastewater treatment using microalgae-based processes. Bioresour Technol. 2019;289:121718.

65. Habib A, Parvin M, Huntington T, Hasan M. A review on culture, production and use of spirulina as food for humans and eeds for domestic animals and fish. FAO; 2008.

66. Real Decreto 60/2011, de 21 de enero, sobre las normas de calidad ambiental en el ámbito de la politica de aguas., BOE-A-2011-1139 (2011).

67. Hedo EB. Real Decreto 306/2020, de 11 de febrero, por el que se establecen normas básicas de ordenación de las granjas porcinas intensivas, y se modifica la normativa básica de ordenación de las explotaciones de ganado porcino extensive. Actualidad Juridica Ambiental. 2020(99):108-11.

68. ORDEN DRS/330/2019, de 26 de marzo, por la que se actualizan varios anexos de las Directrices sectoriales sobre actividades e instalaciones ganaderas, cuya revisión se aprobó por el Decreto 94/2009, de 26 de mayo, del Gobierno de Aragón., (2019).

69. Real Decreto 47/2022, de 18 de enero, sobre protección de las aguas contra la contaminación difusa producida por los nitrates procedentes de fuentes agrarias. BOE. 2022.

70. Ortiz-Villota MT, Romero-Morales MA, Meza-Rodriguez LDJRdi, desarrollo e innovación. La biorremediación con microalgas (Spirulina máxima, Spirulina platensis y Chlorella vulgaris) como alternativa para tratar la eutrofización de la laguna de Ubaque, Colombia. 2018;9(1):163-76.

71. Morales-Amaral MdM, Gómez-Serrano C, Acién FG, Fernández-Sevilla JM, Molina-Grima E. Outdoor production of Scenedesmus sp. in thin-layer and raceway reactors using centrate from anaerobic digestion as the sole nutrient source. Algal Research. 2015;12:99-108.

72. Resina Rueda MJ. Análisis de la influencia de la temperature en la respuesta fotosintética de cultivos de Nanochloropsis gaditana bajo regimenes de luz continua. 2020.

73. Lugo De Ossa CA. Evaluación de algunos factores abióticos que favorecen la producción de biomasa y de ácidos grasos totales en la microalga nativa Chlorella sp en cultivo heterotrófico. 2022.

74. González-Delgado ÁD, Barajas-Solano AF, Ardila-Álvarez AMJCyTA. Biomass and protein production of Chlorella vulgaris Beyerinck (Chlorellales: Chlorellaceae) via the design of selective culture media. 2017;18(3):451-61.

75. Chiu SY, Kao CY, Chen TY, Chang YB, Kuo CM, Lin CS. Cultivation of microalgal Chlorella for biomass and lipid production using wastewater as nutrient resource. Bioresour Technol. 2015;184:179-89.

76. Wollmann F, Dietze S, Ackermann JU, Bley T, Walther T, Steingroewer J, et al. Microalgae wastewater treatment: Biological and technological approaches. Eng Life Sci. 2019;19(12):860-71.

77. Richmond A, Becker E. Technological aspects of mass cultivation-a general outline. CRC handbook of microalgal mass culture: CRC Press; 2017. p. 245-64.

78. Goswami RK, Agrawal K, Shah MP, Verma P. Bioremediation of heavy metals from wastewater: a current perspective on microalgae-based future. Lett Appl Microbiol. 2021;75(4):701-17.

79. Hussain F, Shah SZ, Ahmad H, Abubshait SA, Abubshait HA, Laref A, et al. Microalgae an ecofriendly and sustainable wastewater treatment option: Biomass application in biofuel and bio-fertilizer production. A review. Renewable Sustainable Energy Reviews 2021-137-110603.

80. Ferreira LS, Rodrigues MS, Converti A, Sato S, Carvalho JC. Kinetic and growth parameters of Arthrospira (Spirulina) platensis cultivated in tubular photobioreactor under different cell circulation systems. Biotechnol Bioeng. 2012;109(2):444-50.

81. Kubar AA, Ali A, Kumar S, Huo S, Ullah MW, Alabbosh KFS, et al. Dynamic Foam Characteristics during Cultivation of Arthrospira platensis. Bioengineering (Basel). 2022;9(6).

82. Figueroa-Torres GM, Bermejo Padilla E, Pittman JK, Constantinos T. Microalgae strain catalogue. EAPA_338; 2016.

83. Licett B, Guevara M, Lemus N, Freites L, Romero L, Lodeiros C, et al. Crecimiento y composición Bioquimica de Arthrospira platensis cultivadas a diferentes salinidades y fuentes de Nitrógeno. Instituto de investigaciones agricolas (INIA). 2014.

84. Huarachi R, Yapo U, Dueñas A, Soto J, González R. Producción de Spirulina platensis (Cyanophyta) en fotobioreactor tubular cónico bajo condiciones de laboratorio. The Biologist. 2013;11(2).

85. Torzillo G, Carlozzi P, Pushparaj B, Montaini E, Materassi R. A two-plane tubular photobioreactor for outdoor culture of Spirulina. Biotechnol Bioeng. 1993;42(7):891-8.

86. Fuentes Escobar KL, Olivera Bonilla JA. Evaluación de diferentes procesos de tratamiento de agua para la obtención de subproductos utilizando microalgas. 2017.

87. Abalde J, Cid A, Fidalgo Paredes P, Torres E, Herrero C. Microalgas: cultivo y aplicaciones. Universidad de A Coruña. 1995.

88. Kumar M, Kulshreshtha J, Singh GP. Growth and biopigment accumulation of cyanobacterium Spirulina platensis at different light intensities and temperature. Braz J Microbiol. 2011;42(3):1128-35.

89. Kumar DP, Murthy SD. Photoinhibition induced alterations in energy transfer process in phycobilisomes of PS II in the cyanobacterium, Spirulina platensis. J Biochem Mol Biol. 2007;40(5):644-8.

90. Vonshak A, Guy R. Photoinhibition as a limiting factor in outdoor cultivation of Spirulina platensis. Algal biotechnology/edited by T Stadler. 1988:6.

91. Huarachi-Olivera R, Yapo-Pari U, Dueñas-Gonza A, Condori-Huamanga J, Pacheco-Salazar D, Soto-Flores J. Cultivation of Arthrospira platensis (Spirulina) in curved doubly tubular photobioreactor to environmental conditions in the South of the Peru. Revista Colombiana de Biotecnologia. 2015;17(1):142-9.

92. Hernández-Pérez A, Labbé JI. Microalgas, cultivo y beneficios. Revista de biologia marina y oceanografia 2014;49(2):157-73.

93. Noroozlo YA, Souri MK, Delshad M. Stimulation Effects of Foliar Applied Glycine and Glutamine Amino Acids on Lettuce Growth. Open Agriculture. 2019;4(1):164-72.

94. Rosales A, Bedmar E, Martinez R, Nebot E, Aranda P, Porres J, et al. Influencia de la salinidad sobre el crecimiento de cinco variedades de Cicer Arietinum inoculadas con Mesorhizobium ciceri y efecto de la coinoculación con H. maura.

95. Thakur CJ, Saini S, Notra A, Chauhan B, Arya S, Gupta R, et al. Deciphering the functional role of hypothetical proteins from Chloroflexus aurantiacs J-10-f1 using bioinformatics approach. Mol Biol Res Commun. 2020;9(3):129-39.

96. Zhang H, Zhao X, Chen X, Xu X. Thoroughly review the recent progresses in improving O/W interfacial properties of proteins through various strategies. Front Nutr. 2022;9:1043809.

97. Wu D, Tu M, Wang Z, Wu C, Yu C, Battino M, et al. Biological and conventional food processing modifications on food proteins: Structure, functionality, and bioactivity. Biotechnol Adv. 2020;40:107491.

98. Hulsen T, Hsieh K, Lu Y, Tait S, Batstone DJ. Simultaneous treatment and single cell protein production from agri-industrial wastewaters using purple phototrophic bacteria or microalgae - A comparison. Bioresour Technol. 2018;254:214-23.

99. López-Padrón I, Martínez-González L, Pérez-Domínguez G, Cedeño-Rodriguez L, Reyes-Guerrero Y, Cárdenas-Travieso RM, et al. Efectos de productos bioactivos en plantas de Cicer arietinum L. Cultivos Tropicales. 2020;42(e06).

100. Ertani A, Nardi S, Francioso O, Sánchez-Cortés S, Foggia MD, Schiavon M. Effects of two protein hydrolysates obtained from Chickpea (Cicer arietinum L.) and Spirulina platensis on zea mays (L.) plants. Frontiers Media; 2019.

101. MITECO. Plan Nacional de Adaptación al Cambio Climático 2021- 2030. In: Demográfico MpITEyeR, editor. M-29241-2020 ed. Madrid: MITECO; 2021. p. 246.

## Claims

1. An on-site modular system for processing and transforming animal waste, comprising:

   **(a)** a treatment unit for treating animal waste comprising a solid/liquid separation unit in order to obtain a slurry-derived liquid animal waste fraction;
   **(b)** a treatment unit for homogenizing liquid animal waste fraction of unit (a) comprising a high-pressure homogenization and/or sonication unit in order to obtain a homogenized nutrient-rich liquid fraction;
   **(c)** a culture unit for culturing microalgae by using the homogenized nutrient-rich liquid fraction obtained in the unit (b) as a culture medium;
   **(d)** a unit for collecting and processing the microalgae culture produced in unit (c) comprising a high-pressure homogenization and/or sonication unit in order to obtain a homogenized nutrient-rich microalgae-derived colloid suspension;
   **(e)** a unit for separating solid/liquid homogenized nutrient-rich microalgae-derived colloid suspension produced in unit (d) comprising a solid/liquid separation unit and/or a microfiltration unit in order to obtain two byproducts: (1) processed rich-nutrients biologically absorbable nitrogen-rich phosphorus-rich potassium-rich liquid microalgae culture fraction, and (2) processed rich-carbon solid inactivated microalgae culture fraction;
   **(f)** a control and monitoring unit for monitoring cultivation conditions and processing efficiency.

2. The system according to claim 1, wherein the microalgae are selected from *Arthrospira platensis, Chlorella vulgaris* or a combination thereof.

3. The system according to any of claims 1 or 2, wherein the solid/liquid separation unit in unit (a) and unit (e) comprises at least one of the followings: a particle size and gravity separation unit, a pressure separation unit, and centrifugal separation unit.

4. The system according to any of claims 1 to 3, wherein the culture unit (c) comprises at least one open or closed photobioreactor, optionally including an aeration unit, wherein the at least one open or closed photobioreactor includes a temperature control unit.

5. The system according to claim 4, wherein the at least one open or closed photobioreactor is a tubular, flat plate or pool-type bioreactor.

6. The system according to claim 4 or 5, wherein the temperature in the at least one open or closed photobioreactor is between 10° and 35 °C.

7. The system according to any of the preceding claims, wherein the unit (d) and unit (e) comprises a filtration unit, a drying unit and extraction unit.

8. The system according to any of the preceding claims, wherein the control and monitoring unit (f) comprises at least one sensor and actuator to control culture parameters, nutrient concentration and greenhouse gases emissions.

9. A process for transforming animal waste *in situ* using the on-site modular system according to any of claims 1 to 8, comprising the steps of:

   (i) treating animal waste for obtaining a homogenized nutrient-rich liquid fraction;
   (ii) culturing microalgae in a culture unit using the homogenized nutrient-rich liquid fraction of of claim 1(b) as the culture medium;
   (iii) collecting and processing the microalgae culture produced in claim 1(c);
   (iv) collecting and processing the homogenized nutrient-rich microalgae-derived colloid suspension produced in claim 1(d);
   (v) collecting and processing the processed rich-nutrients biologically absorbable nitrogen-rich phosphorus-rich potassium-rich liquid microalgae culture fraction and the processed rich-carbon solid inactivated microalgae culture fraction after claim 1(d).

10. Use of the processed rich-nutrients biologically absorbable nitrogen-rich phosphorus-rich potassium-rich liquid microalgae culture fraction obtained according to the process of claim 9 as phytoremediators of agriculture or natural soil amendments.

11. Use of the processed rich-carbon solid inactivated microalgae culture fraction obtained according to the process of claim 9 as recycler of toxic or contaminants in animal waste farm.

12. Use of the processed rich-carbon solid inactivated microalgae culture fraction obtained according to the process of claim 9 as animal feed.

13. Use of the processed rich-carbon solid inactivated microalgae culture fraction obtained according to the process of claim 9 as fresh compost of agriculture or natural soil amendments.

14. Use of the system according to any of claims 1 to 8 in a farm for reducing greenhouse gas emissions and carbon emissions therein.

Figure 1

$Q_2$ [t/day] = ?
$L$
$C_{N2}$ [kg N/t] = 2,9

$Q_3$ [t/day] = 2,6
$S$
$C_{N3}$ [kg N/t] = ?

$Q_1$ [t/day] = 21
$C_{N1}$ [kg N/t] = 3,5

Figure 2

Figure 3

Figure 4

Figure 5

Figure 6

A

B

**Figure 7**

Figure 8

Figure 9

A                                            B

**Figure 10**

**Figure 11**

Figure 12

Figure 13

***Arthrospira platensis* culture in outside open unit (days)**

$$y = 0.0233x + 0.6242$$

Figure 14

A

B

**Figure 15**

Figure 16

Concentration on farm scaling

Figure 17

Figure 18

Figure 19

Figure 20

**Figure 21**

**Figure 22**

**Figure 23**

**Figure 24**

**Figure 25**

**Figure 26**

Figure 27

Europäisches
Patentamt

European
Patent Office

Office européen
des brevets

# EUROPEAN SEARCH REPORT

**Application Number**

EP 24 38 2074

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/207069 A1 (KRIVOV ANJU D [US]) 8 July 2021 (2021-07-08) * paragraphs [0002] - [0004], [0035] - [0038], [0043], [0048], [0057] - [5863]; figure 1 * | 1-14 | INV.<br>C02F9/00<br>C05F11/00<br>C12M1/00<br>C12N1/12 |
| A | US 2014/124438 A1 (ANKER YAAKOV [IL] ET AL) 8 May 2014 (2014-05-08) * the whole document * | 1-14 | ADD.<br>C02F1/34<br>C02F1/36<br>C02F1/38 |
| A | US 2016/102007 A1 (JU LU-KWANG [US] ET AL) 14 April 2016 (2016-04-14) * the whole document * | 1-14 | C02F1/44<br>C02F3/32<br>C02F11/127<br>C02F103/20 |
| A | WO 2015/095941 A1 (REIS LUCAS MARDER DE OLIVEIRA [BR]; ZESCHAU MURILO CANOVA [BR] ET AL.) 2 July 2015 (2015-07-02) * the whole document * | 1-14 | |

**TECHNICAL FIELDS SEARCHED (IPC)**

C02F
C12M
C05G
C12R
C05F
C12N

The present search report has been drawn up for all claims

| Place of search | Date of completion of the search | Examiner |
|---|---|---|
| The Hague | 31 May 2024 | Liebig, Thomas |

EPO FORM 1503 03.82 (P04C01)

## ANNEX TO THE EUROPEAN SEARCH REPORT
## ON EUROPEAN PATENT APPLICATION NO.

EP 24 38 2074

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2024

| Patent document cited in search report | Publication date | Patent family member(s) | | Publication date |
|---|---|---|---|---|
| US 2021207069 A1 | 08-07-2021 | US | 2021207069 A1 | 08-07-2021 |
| | | US | 2023203414 A1 | 29-06-2023 |
| US 2014124438 A1 | 08-05-2014 | EP | 2704999 A2 | 12-03-2014 |
| | | US | 2014124438 A1 | 08-05-2014 |
| | | WO | 2012153174 A2 | 15-11-2012 |
| US 2016102007 A1 | 14-04-2016 | CA | 2943899 A1 | 04-12-2014 |
| | | EP | 3003995 A1 | 13-04-2016 |
| | | US | 2016102007 A1 | 14-04-2016 |
| | | WO | 2014194174 A1 | 04-12-2014 |
| WO 2015095941 A1 | 02-07-2015 | BR | 102013033383 A2 | 24-02-2015 |
| | | WO | 2015095941 A1 | 02-07-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LOYON L**. Overview of Animal Manure Management for Beef, Pig, and Poultry Farms in France. *Frontiers in Sustainable Food Systems*, 2018, vol. 2
- Fertilización con purin en doble cultivo anual, en mínimo laboreo, y riego por aspersión. **QUILEZ D**. Informaciones Técnicas N° 223: Dirección General de Desarrollo Rural. Centro de Transferencia Agroalimentaria. Gobierno de Aragón, 2010
- **CHENG H-H** ; **NARINDRI B** ; **CHU H** ; **WHANG L-M**. Recent advancement on biological technologies and strategies for resource recovery from swine wastewater. *Bioresource Technology.*, 2020, vol. 303, 122861
- *Decreto 53/2019, por el que se regula la gestión de estiércoles y los procedimientos de acreditación y control*, 2019
- **MESTRE MARTINEZ MV**. Ganaderia intensiva: el reto ambiental del sector porcino en España. *The Conversation.*, 2021, vol. 2
- MODELO DE GESTION SOSTENIBLE DE PURIN PORCINO SOSTENIBLE DE PURIN PORCINO EN TAUSTE. **EDERRA F.** JORNADAS DE CIENCIA Y TECNOLOGIA. 12 May 2016, vol. XXVII
- **GÓMEZ GARRIDO M**. *Efectos ambientales de la valorización agronómica de purines de ganado porcino: dinámica del nitrógeno en el sistema suelo-agua-planta*, 2014
- **SALMERÓN M** ; **ISLA R** ; **CAVERO J** ; **DELGADO I**. Viabilidad de las aplicaciones de purin en alfalfa de regadio en el Valle del Ebro. *Agriculture: Revista agropecuaria y ganadera*, 2011 (942), 458-62
- Tratamiento de purines de ganado porcino en España para minimizar la contaminación de suelos y su impacto ambiental. Congreso Internacional sobre Desertificación. 2009
- Ministerio de Agriculture PyAM. Jornada sobre Sistemas de gestión de deyecciones ganaderas. Jornada sobre Sistemas de gestión de deyecciones ganaderas. 26 November 2015
- Inventory of manure processing activities in Europe. **XAVIER FLOTATS** ; **HENNING L. FOGED** ; **AUGUST BONMATI** ; **KARL M. SCHELDE** ; **JORDI PALATSI** ; **ALBERT MAGRI et al.** Protecting waters against pollution caused by nitrates from agricultural sources: European Comission. 2013
- **BESEL S**. Biomasa: digestores anaerobios.. Editorial Idea, 2007
- **FLOTATS RIPOLL X**. *La gestión integral de purines2019*, 12 January 2023, https://www.3tres3.com/articulos/la-gestion-integral-de-purines 41865/
- **CHENG DL** ; **NGO HH** ; **GUO WS** ; **CHANG SW** ; **NGUYEN DD** ; **KUMAR SM.** Microalgae biomass from swine wastewater and its conversion to bioenergy. *Bioresource Technology*, 2019, vol. 275, 109-22
- **LÓPEZ-PACHECO IY** ; **SILVA-NÚÑEZ A** ; **GARCIA-PEREZ JS** ; **CARRILLO-NIEVES D** ; **SALINAS-SALAZAR C** ; **CASTILLO-ZACARIAS C et al.** Phyco-remediation of swine wastewater as a sustainable model based on circular economy. *Journal of Environmental Management*, 2021, vol. 278, 111534
- **NAGARAJAN D** ; **KUSMAYADI A** ; **YEN H-W** ; **DONG C-D** ; **LEE D-J** ; **CHANG J-S**. Current advances in biological swine wastewater treatment using microalgae-based processes. *Bioresource Technology*, 2019, vol. 289, 121718
- **VARMA VS** ; **PARAJULI R** ; **SCOTT E** ; **CANTER T** ; **LIM TT** ; **POPP J et al.** Dairy and swine manure management - Challenges and perspectives for sustainable treatment technology. *Science of The Total Environment.*, 2021, vol. 778, 146319
- **ZHENG M** ; **DAI J** ; **JI X** ; **LI D** ; **HE Y** ; **WANG M et al.** An integrated semi-continuous culture to treat original swine wastewater and fix carbon dioxide by an indigenous Chlorella vulgaris MBFJNU-1 in an outdoor photobioreactor. *Bioresource Technology.*, 2021, vol. 340, 125703
- **ZHENG M** ; **JI X** ; **HE Y** ; **LI Z** ; **WANG M** ; **CHEN B et al.** Simultaneous fixation of carbon dioxide and purification of undiluted swine slurry by culturing Chlorella vulgaris MBFJNU-1.. *Algal Research*, 2020, vol. 47, 101866
- **LI X** ; **WU S** ; **YANG C** ; **ZENG G**. Microalgal and duckweed based constructed wetlands for swine wastewater treatment: A review. *Bioresource Technology*, 2020, vol. 318, 123858
- **DIPIETRE D**. *La politica, una causa mas de la variabilidad de los precios y los costes.*, 2013, http://www.3tres3.com/situacion-mercado-porcino/-la-politica-una-causa-mas-de-la-variabilidad-de-precios-y-costes 32848/

- **KUHN T** ; **KOKEMOHR L** ; **HOLM-MÜLLER K**. A life cycle assessment of liquid pig manure transport in line with EU regulations: A case study from Germany. *Journal of Environmental Management.*, 2018, vol. 217, 456-67
- **RUIZ FJA**. Integrated and Cost-Effective Solution to reduce the volume of Pig Slurry, minimize Pollutant Emissions and Process Energy Consumption. *CORDIS: European Commission*, 30 September 2013
- **GESING F.** The material politics of slurry: Mobilisations and transformations along the waste-fertiliser continuum.. *Political Geography*, 2023, vol. 101, 102832
- **GALEANO Ó**. Discover all the benefits of the agronomic use of slurry. 2022
- **NOLAN T** ; **TROY SM** ; **GILKINSON S** ; **FROST P** ; **XIE S** ; **ZHAN X et al.** Economic analyses of pig manure treatment options in Ireland.. *Bioresource Technology.*, 2012, vol. 105, 15-23
- **VALLÉS PÉREZ M** ; **ESPADA DOMINGO M** ; **MAGALLÓN BOTAYA E**. Coyuntura del sector porcino aragonés en 2022. Empleo generado, Normativa Medioambiental y Análisis DAFO [Internet]. 2022. *Informaciones Técnicas*, vol. 285, 20, https://www.aragon.es/documents/20127/77519864/IT 285 22.pdf/140504cf-323c-1435-295b-ee51efa8cc97?t=1668601063000
- **NINYEROLA M** ; **PONS X** ; **ROURE JM**. Atlas climático digital de la Peninsula Ibérica. Universitat Autònoma de Barcelona Departament de Biologia Animal, Biologia Vegetal i Ecologia (Unitat de Botànica), September 2005
- **GARCIA J** ; **ORTIZ A** ; **ÁLVAREZ E** ; **BELOHLAV V** ; **GARCÍA-GALÁN MJ** ; **DIEZ-MONTERO R et al.** Nutrient removal from agricultural run-off in demonstrative full scale tubular photobioreactors for microalgae growth. *Ecological engineering.*, 2018, vol. 120, 513-21
- **RUSHTON J**. The economics of animal health and production. Royal Veterinary College, 2008
- Biochemical Engineering. **LEE JM.** Department of Chemical Engineering. 2009
- **DUTTA R**. Fundamentals of biochemical engineering. Springer, 2008
- **KHOLSSI R** ; **RAMOS PV** ; **MARKS EA** ; **MONTERO O** ; **RAD C**. Biotechnological uses of microalgae: a review on the state of the art and challenges for the circular economy. *Biocatalysis Agricultural Biotechnology*, 2021, vol. 36, 1-02114
- **SÁNCHEZ-BAYO A** ; **MORALES V** ; **RODRIGUEZ R** ; **VICENTE G** ; **BAUTISTA LF**. Cultivation of microalgae and cyanobacteria: Effect of operating conditions on growth and biomass composition. *Molecules*, 2020, vol. 25 (12), 2834
- **SÁNCHEZ-BORROTO Y** ; **TOBÍO-PÉREZ I** ; **ROMERO-LÓPEZ TDJ** ; **DÍAZ-DOMÍNGUEZ Y** ; **MELO-ESPINOSA E** ; **PILOTO-RODRIGUEZ R**. Evaluación de las condiciones experimentales básicas para la producción de biomasa a partir de la microalga Chlorella vulgaris. *Afinidad*, 2019, vol. 76 (585)
- **GONZALEZ-CAMEJO J** ; **APARICIO S** ; **JIMÉNEZ-BENÍTEZ A** ; **PACHÉS M** ; **RUANO M** ; **BORRÁS L et al.** Improving membrane photobioreactor performance by reducing light path: operating conditions and key performance indicators. *Water Research*, 2020, vol. 172, 115518
- **ORTIZ RUIZ A**. *Microalgae-based wastewater treatment systems at demonstrative scale: gravity harvesting and thickening of biomass, and advanced design of bioreactors.*, 2021
- **IGUÁCEL SOTERAS F** ; **YAGÜE CARRASCO MR** ; **ORÚS PUEYO F** ; **QUILEZ SÁEZ DE VITERI D**. *Fertilización con purin en doble cultivo anual, en mínimo laboreo, y riego por aspersión*, 2010
- **JIMÉNEZ VEUTHEY M**. Depuración de purines en fotobiorreactores abiertos. Universidad de Almeria., 2015
- **BOLADO RODRIGUEZ S** ; **LORENZO HERNANDO A**. Estudio de hidrólisis enzimática en biomasa microalgal procedente del tratamiento de aguas residuales con purines. UNIVERSIDAD DE VALLADOLID, 2016
- **CÁRDENAS CALVACHI GL** ; **SÁNCHEZ ORTIZ IA**. Nitrógeno en aguas residuales: origenes, efectos y mecanismos de remoción para preservar el ambiente y la salud pública. *Universidad y Salud*, 2013, vol. 15 (1), 72-88
- **LIKILIKI C**. *Étude des procédés biologiques d'élimination d'azote appliqués à la phase liquide du lisier de pores après séparation en bâtiment: Universit6 Rennes*, 2021, vol. 1
- **ORTUÑO CASES C**. Aplicación de ultrasonidos de potencia para la mejora de procesos de inactivación con fluidos supercriticos. Universitat Politècnica de València, 2014
- *n.d. Sonicación para Lisis: Disrupción y extracción celular*, 25 November 2023, https://www.hielscher.com/es/ultrasonic-lysis-cell-disruption-extraction.htm
- **TAHA A** ; **AHMED E** ; **ISMAIEL A** ; **ASHOKKUMAR M** ; **XU X** ; **PAN S et al.** Ultrasonic emulsification: An overview on the preparation of different emulsifiers-stabilized emulsions. *Trends in Food Science & Technology*, 2020, vol. 105, 363-77
- **SIREESHA T** ; **GOWDA NN** ; **KAMBHAMPATI V**. Ultrasonication in seafood processing and preservation: a comprehensive review. *Applied Food Research*, 2022, vol. 2 (2), 100208

- **SUTARIYA S** ; **SUNKESULA V** ; **KUMAR R** ; **SHAH K**. Emerging applications of ultrasonication and cavitation in dairy industry: a review. *Cogent Food & Agriculture*, 2018, vol. 4 (1), 1549187
- **GROSSHAGAUER S** ; **KRAEMER K** ; **SOMOZA V.** The True Value of Spirulina. *J Agric Food Chem.*, 2020, vol. 68 (14), 4109-15
- **CAMACHO-MORALES S**. Producción de spirulina: aislamiento, optimización de cultivo y secado. *Fase: aislamiento y preservación Arthrospira (spirulina) nativa de Jalisco*, 2016
- **LOPEZ-PACHECO IY** ; **SILVA-NUNEZ A** ; **GARCIA-PEREZ JS** ; **CARRILLO-NIEVES D** ; **SALINAS-SALAZAR C** ; **CASTILLO-ZACARIAS C et al.** Phyco-remediation of swine wastewater as a sustainable model based on circular economy. *J Environ Manage.*, 2021, vol. 278, 111534
- **HARVEY M**. *HIGH-PRESSURE SLURRY ABLATION FOR IMPROVING SEPARATION OF COPPER/MOLYBDENUM AND FLOTATION OF GOLD TAILINGS*, May 2003
- **PERLMUTTER B**. *Combination mechanical slurry conditioning and filtration2012*, 02 December 2023, https://www.digitalrefining.com/article/1000370/-combination-mechanical-slurry-conditioning-and-filtration
- **DIPIETRE D**. *La maximización del beneficio, el objetivo último de la producción porcina http://www.3tres3.com: 333 Corporate 1998*, 2013, http://www.3tres3.com/situacion-mercado-porcino/el-objetivo-de-la-produccion-porcina-la-maximizacion-del-beneficio 33048/
- **HUSSAIN F** ; **SHAH SZ** ; **AHMAD H** ; **ABUBSHAIT SA** ; **ABUBSHAIT HA** ; **LAREF A et al.** Microalgae an ecofriendly and sustainable wastewater treatment option: Biomass application in biofuel and biofertilizer production. *A review. Renewable and Sustainable Energy Reviews*, 2021, vol. 137 (11), 0603
- **TANVIR RU** ; **ZHANG J** ; **CANTER T** ; **CHEN D** ; **LU J** ; **HU Z**. Harnessing solar energy using phototrophic microorganisms: A sustainable pathway to bioenergy, biomaterials, and environmental solutions. *Renewable and Sustainable Energy Reviews*, 2021, vol. 146, 111181
- **CHIU S-Y** ; **KAO C-Y** ; **CHEN T-Y** ; **CHANG Y-B** ; **KUO C-M** ; **LIN C-S**. Cultivation of microalgal Chlorella for biomass and lipid production using wastewater as nutrient resource. *Bioresource Technology*, 2015, vol. 184, 179-89
- **KHOLSSI R** ; **RAMOS PV** ; **MARKS EAN** ; **MONTERO O** ; **RAD C**. 2Biotechnological uses of microalgae: A review on the state of the art and challenges for the circular economy. *Biocatalysis and Agricultural Biotechnology*, 2021, vol. 36 (1), 02114
- **BLANCO L**. Análisis y caracterización de purines para la obtención de estruvita y biogás. Universitat Politécnica de Valéncia, 2015
- Commission E. Regulation (EC) No 767/2009 of the European Parliament and of the Council of 13 July 2009 on the placing on the market and use of feed, amending European Parliament and Council Regulation (EC) No 1831/2003 and repealing Council Directive 79/373/EEC, Commission Directive 80/511/EEC, Council Directives 82/471/EEC, 83/228/EEC, 93/74/EEC, 93/113/EC and 96/25/EC and Commission Decision 2004/217/EC.. *Off J Eur Union.*, 2009, vol. 229, 1-28
- Commission E. Commission Regulation (EU) No 68/2013 of 16 January 2013 on the Catalogue of feed materials. *Off J Eur Union*, 2013, vol. 29, 1-64
- **LEFORT F** ; **CALMIN G** ; **CROVADORE J** ; **FALQUET J** ; **HURNI JP** ; **OSTERAS M et al.** Whole-Genome Shotgun Sequence of Arthrospira platensis Strain Paraca, a Cultivated and Edible Cyanobacterium. *Genome Announc.*, 2014, vol. 2 (4)
- **CHENG HH** ; **NARINDRI B** ; **CHU H** ; **WHANG LM**. Recent advancement on biological technologies and strategies for resource recovery from swine wastewater. *Bioresour Technol.*, 2020, vol. 303, 122861
- **CHENG DL** ; **NGO HH** ; **GUO WS** ; **CHANG SW** ; **NGUYEN DD** ; **KUMAR SM**. Microalgae biomass from swine wastewater and its conversion to bioenergy. *Bioresour Technol*, 2019, vol. 275, 109-22
- **NAGARAJAN D** ; **KUSMAYADI A** ; **YEN HW** ; **DONG CD** ; **LEE DJ** ; **CHANG JS**. Current advances in biological swine wastewater treatment using microalgae-based processes. *Bioresour Technol.*, 2019, vol. 289, 121718
- **HABIB A** ; **PARVIN M** ; **HUNTINGTON T** ; **HASAN M.** A review on culture, production and use of spirulina as food for humans and eeds for domestic animals and fish. *FAO*, 2008
- Real Decreto 60/2011, de 21 de enero, sobre las normas de calidad ambiental en el ámbito de la politica de aguas.. *BOE-A-2011-1139*, 2011
- **HEDO EB**. Real Decreto 306/2020, de 11 de febrero, por el que se establecen normas básicas de ordenación de las granjas porcinas intensivas, y se modifica la normativa básica de ordenación de las explotaciones de ganado porcino extensive. *Actualidad Juridica Ambiental*, 2020, vol. 99, 108-11
- ORDEN DRS/330/2019, de 26 de marzo, por la que se actualizan varios anexos de las Directrices sectoriales sobre actividades e instalaciones ganaderas, cuya revisión se aprobó por el Decreto 94/2009, de 26 de mayo. *del Gobierno de Aragón.*, 2019

- Real Decreto 47/2022, de 18 de enero, sobre protección de las aguas contra la contaminación difusa producida por los nitratos procedentes de fuentes agrarias. *BOE*, 2022
- **ORTIZ-VILLOTA MT** ; **ROMERO-MORALES MA** ; **MEZA-RODRIGUEZ LDJRDI**. desarrollo e innovación. *La biorremediación con microalgas (Spirulina máxima, Spirulina platensis y Chlorella vulgaris) como alternativa para tratar la eutrofización de la laguna de Ubaque, Colombia.*, 2018, vol. 9 (1), 163-76
- **MORALES-AMARAL MDM** ; **GÓMEZ-SERRANO C** ; **ACIÉN FG** ; **FERNÁNDEZ-SEVILLA JM** ; **MOLINA-GRIMA E**. Outdoor production of Scenedesmus sp. in thin-layer and raceway reactors using centrate from anaerobic digestion as the sole nutrient source. *Algal Research*, 2015, vol. 12, 99-108
- **RESINA RUEDA MJ**. *Análisis de la influencia de la temperature en la respuesta fotosintética de cultivos de Nanochloropsis gaditana bajo regimenes de luz continua*, 2020
- **LUGO DE OSSA CA**. *Evaluación de algunos factores abióticos que favorecen la producción de biomasa y de ácidos grasos totales en la microalga nativa Chlorella sp en cultivo heterotrófico*, 2022
- **GONZÁLEZ-DELGADO ÁD** ; **BARAJAS-SOLANO AF** ; **ARDILA-ÁLVAREZ AMJCYTA**. *Biomass and protein production of Chlorella vulgaris Beyerinck (Chlorellales: Chlorellaceae) via the design of selective culture media.*, 2017, vol. 18 (3), 451-61
- **CHIU SY** ; **KAO CY** ; **CHEN TY** ; **CHANG YB** ; **KUO CM** ; **LIN CS**. Cultivation of microalgal Chlorella for biomass and lipid production using wastewater as nutrient resource. *Bioresour Technol*, 2015, vol. 184, 179-89
- **WOLLMANN F** ; **DIETZE S** ; **ACKERMANN JU** ; **BLEY T** ; **WALTHER T** ; **STEINGROEWER J et al.** Microalgae wastewater treatment: Biological and technological approaches. *Eng Life Sci.*, 2019, vol. 19 (12), 860-71
- Technological aspects of mass cultivation-a general outline. **RICHMOND A** ; **BECKER E**. CRC handbook of microalgal mass culture. CRC Press, 2017, 245-64
- **GOSWAMI RK** ; **AGRAWAL K** ; **SHAH MP** ; **VERMA P**. Bioremediation of heavy metals from wastewater: a current perspective on microalgae-based future. *Lett Appl Microbiol.*, 2021, vol. 75 (4), 701-17
- **HUSSAIN F** ; **SHAH SZ** ; **AHMAD H** ; **ABUBSHAIT SA** ; **ABUBSHAIT HA** ; **LAREF A et al.** Microalgae an ecofriendly and sustainable wastewater treatment option: Biomass application in biofuel and bio-fertilizer production. A review. *Renewable Sustainable Energy Reviews*, 2021, vol. 137, 110603
- **FERREIRA LS** ; **RODRIGUES MS** ; **CONVERTI A** ; **SATO S** ; **CARVALHO JC**. Kinetic and growth parameters of Arthrospira (Spirulina) platensis cultivated in tubular photobioreactor under different cell circulation systems. *Biotechnol Bioeng*, 2012, vol. 109 (2), 444-50
- **KUBAR AA** ; **ALI A** ; **KUMAR S** ; **HUO S** ; **ULLAH MW** ; **ALABBOSH KFS et al.** Dynamic Foam Characteristics during Cultivation of Arthrospira platensis. *Bioengineering (Basel)*, 2022, vol. 9 (6)
- **FIGUEROA-TORRES GM** ; **BERMEJO PADILLA E** ; **PITTMAN JK** ; **CONSTANTINOS T**. Microalgae strain catalogue. *EAPA_338*, 2016
- **LICETT B** ; **GUEVARA M** ; **LEMUS N** ; **FREITES L** ; **ROMERO L** ; **LODEIROS C et al.** Crecimiento y composición Bioquimica de Arthrospira platensis cultivadas a diferentes salinidades y fuentes de Nitrógeno. *Instituto de investigaciones agricolas (INIA).*, 2014
- **HUARACHI R** ; **YAPO U** ; **DUEÑAS A** ; **SOTO J** ; **GONZÁLEZ R**. Producción de Spirulina platensis (Cyanophyta) en fotobioreactor tubular cónico bajo condiciones de laboratorio. *The Biologist.*, 2013, vol. 11 (2)
- **TORZILLO G** ; **CARLOZZI P** ; **PUSHPARAJ B** ; **MONTAINI E** ; **MATERASSI R**. A two-plane tubular photobioreactor for outdoor culture of Spirulina. *Biotechnol Bioeng*, 1993, vol. 42 (7), 891-8
- **FUENTES ESCOBAR KL** ; **OLIVERA BONILLA JA**. *Evaluación de diferentes procesos de tratamiento de agua para la obtención de subproductos utilizando microalgas*, 2017
- **ABALDE J** ; **CID A** ; **FIDALGO PAREDES P** ; **TORRES E** ; **HERRERO C**. Microalgas: cultivo y aplicaciones. Universidad de A Coruña, 1995
- **KUMAR M** ; **KULSHRESHTHA J** ; **SINGH GP**. Growth and biopigment accumulation of cyanobacterium Spirulina platensis at different light intensities and temperature. *Braz J Microbiol.*, 2011, vol. 42 (3), 1128-35
- **KUMAR DP** ; **MURTHY SD**. Photoinhibition induced alterations in energy transfer process in phycobilisomes of PS II in the cyanobacterium, Spirulina platensis. *J Biochem Mol Biol.*, 2007, vol. 40 (5), 644-8
- Photoinhibition as a limiting factor in outdoor cultivation of Spirulina platensis. **VONSHAK A** ; **GUY R**. Algal biotechnology. 1988, vol. 6
- **HUARACHI-OLIVERA R** ; **YAPO-PARI U** ; **DUEÑAS-GONZA A** ; **CONDORI-HUAMANGA J** ; **PACHECO-SALAZAR D** ; **SOTO-FLORES J**. Cultivation of Arthrospira platensis (Spirulina) in curved doubly tubular photobioreactor to environmental conditions in the South of the Peru. *Revista Colombiana de Biotecnologia*, 2015, vol. 17 (1), 142-9

- **HERNÁNDEZ-PÉREZ A** ; **LABBÉ JI**. Microalgas, cultivo y beneficios. *Revista de biologia marina y oceanografia*, 2014, vol. 49 (2), 157-73
- **NOROOZLO YA** ; **SOURI MK** ; **DELSHAD M**. Stimulation Effects of Foliar Applied Glycine and Glutamine Amino Acids on Lettuce Growth.. *Open Agriculture*, 2019, vol. 4 (1), 164-72
- **ROSALES A** ; **BEDMAR E** ; **MARTINEZ R** ; **NEBOT E** ; **ARANDA P** ; **PORRES J et al.** *Influencia de la salinidad sobre el crecimiento de cinco variedades de Cicer Arietinum inoculadas con Mesorhizobium ciceri y efecto de la coinoculación con H. maura*
- **THAKUR CJ** ; **SAINI S** ; **NOTRA A** ; **CHAUHAN B** ; **ARYA S** ; **GUPTA R et al.** Deciphering the functional role of hypothetical proteins from Chloroflexus aurantiacs J-10-f1 using bioinformatics approach. *Mol Biol Res Commun.*, 2020, vol. 9 (3), 129-39
- **ZHANG H** ; **ZHAO X** ; **CHEN X** ; **XU X**. Thoroughly review the recent progresses in improving O/W interfacial properties of proteins through various strategies.. *Front Nutr.*, 2022, vol. 9, 1043809
- **WU D** ; **TU M** ; **WANG Z** ; **WU C** ; **YU C** ; **BATTINO M et al.** Biological and conventional food processing modifications on food proteins: Structure, functionality, and bioactivity. *Biotechnol Adv*, 2020, vol. 40, 107491
- **HULSEN T** ; **HSIEH K** ; **LU Y** ; **TAIT S** ; **BATSTONE DJ**. Simultaneous treatment and single cell protein production from agri-industrial wastewaters using purple phototrophic bacteria or microalgae - A comparison. *Bioresour Technol*, 2018, vol. 254, 214-23
- **LÓPEZ-PADRÓN I** ; **MARTÍNEZ-GONZÁLEZ L** ; **PÉREZ-DOMÍNGUEZ G** ; **CEDEÑO-RODRIGUEZ L** ; **REYES-GUERRERO Y** ; **CÁRDENAS-TRAVIESO RM et al.** Efectos de productos bioactivos en plantas de Cicer arietinum L. *Cultivos Tropicales.*, 2020, vol. 42 (e06)
- **ERTANI A** ; **NARDI S** ; **FRANCIOSO O** ; **SÁNCHEZ-CORTÉS S** ; **FOGGIA MD** ; **SCHIAVON M**. Effects of two protein hydrolysates obtained from Chickpea (Cicer arietinum L.) and Spirulina platensis on zea mays (L.) plants. *Frontiers Media*, 2019
- MITECO. Plan Nacional de Adaptación al Cambio Climático 2021- 2030. 2021, 246